# EUROPEAN PATENT APPLICATION

(11) **EP 1 995 243 A1**
(43) Date of publication of application: **26.11.2008**
(21) Application number: 07738519.3
(22) Date of filing: 14.03.2007
(51) Int. Cl.: C07D 249/08, A61K 31/4196, A61K 31/4245, A61K 31/427, A61K 31/4439, A61K 31/444, A61K 31/4709, A61K 31/497, A61P 3/04, A61P 3/06, A61P 3/10, A61P 9/12, A61P 19/10, A61P 25/28, A61P 27/06, A61P 43/00, C07D 401/06, C07D 401/12, C07D 401/14, C07D 403/06

(54) **TRIAZOLE DERIVATIVE OR SALT THEREOF**

(30) Priority: 16.03.2006 JP 2006072146
(71) Applicant: Astellas Pharma Inc., Tokyo 103-8411 (JP)
(72) Inventor: YOSHIMURA, Seiji, Tokyo 103-8411 (JP); SHIRAKI, Ryota, Tokyo 103-8411 (JP); KAWANO, Tomoaki, Tokyo 103-8411 (JP); SASUGA, Daisuke, Tokyo 103-8411 (JP); HOSAKA, Mitsuru, Tokyo 103-8411 (JP); FUKUDOME, Hiroki, Tokyo 103-8411 (JP); KUROSAWA, Kazuo, Tokyo 103-8411 (JP); ISHII, Hirofumi, Tokyo 103-8411 (JP); KOIKE, Takanori, Tokyo 103-8411 (JP)
(74) Representative: HOFFMANN EITLE
(86) International application number: PCT/JP2007/055048
(87) International publication number: WO 2007/105753

(57) **Abstract**

[Problem] To provide a compound which may be used in treating diseases in which 11β-hydroxysteroid dehydrogenase type 1 (11β-HSD1) is concerned, especially diabetes and insulin resistance.

[Means for Solution] It was found that a triazole derivative or a pharmaceutically acceptable salt thereof, in which the 3-position of triazole ring is substituted with a trisubstituted methyl group and the 5-position is substituted with a lower alkyl, cycloalkyl or the like, has a strong 11β-HSD1-inhibitory activity. In addition, since the triazole derivative of the present invention shows excellent blood glucose-lowering action, it may be used in the treatment of diabetes and insulin resistance.

## Description

### TECHNICAL FIELD

The present invention relates to a pharmaceutical, particularly a novel triazole derivative or a pharmaceutically acceptable salt thereof, which is useful as an agent for treating or preventing diseases in which 11β-hydroxysteroid dehydrogenase type 1 is concerned, such as diabetes, insulin resistance, and the like.

### BACKGROUND OF THE INVENTION

Glucocorticoid is a hormone which causes the metabolic disorder, such as hyperglycemia, insulin resistance, obesity, hyperlipidemia, hypertension and the like, and is not only produced from adrenal glands but also converted from the inactive form into the active form at the tissue level and acts via its receptor.

11β-Hydroxysteroid dehydrogenase (11β-HSD) is an enzyme which catalyzes this conversion, and the presence of two subtypes is known. 11β-Hydroxysteroid dehydrogenase type 1 (11β-HSD1) is an enzyme which converts the inactive form into the active form and its expression is high in the liver, and 11β-hydroxysteroid dehydrogenase type 2 (11β-HSD2) is an enzyme which converts the active form into the inactive form and its expression is high in the kidney. As the relation of 11β-HSD1 with metabolic diseases, increased activity of 11β-HSD1 in the fat tissue of obese people is known (Non-patent Reference 1), and it has been reported that the 11β-HSD1 activity shows high correlation with BMI as an index of the degree of obesity, with HOMA-IR as an index of insulin resistance, and with fasting blood glucose level (Non-patent Reference 2). In addition, it has been reported that a transgenic mouse in which 11β-HSD1 was fat tissue-selectively over-expressed shows insulin resistance, visceral fat type obesity, hyperlipidemia and hypertension, together with increase of glucocorticoid in the fat tissue (Non-patent References 3 and 4) and that a 11β-HSD1 knockout mouse shows improvement of glucose tolerance, lowering of blood triglyceride and increase of HDL-cholesterol (Non-patent Reference 5).

Accordingly, it is expected that a 11β-HSD1-selective inhibitor will suppress glucocorticoid action in tissues by inhibiting conversion into the active form glucocorticoid, and, as a result, correct the metabolic disorders such as hyperglycemia, insulin resistance, obesity, hyperlipidemia, hypertension and the like caused by glucocorticoid.

In addition, since it has been reported that a non-selective 11β-HSD inhibitor carbenoxolone improves the lowering of insulin secretion in mouse pancreatic β-cell caused by the addition of inactive glucocorticoid (Non-patent Reference 6), there is a possibility that an 11β-HSD1 inhibitor not only improves insulin resistance but also corrects hyperglycemia by accelerating insulin secretion.

As other diseases in which 11β-HSD1 is concerned, osteoporosis (Non-patent Reference 7), glaucoma (Non-patent Reference 8) and lowering of cognitive function (Non-patent Reference 9) are known, so that the improving effect therefor by an 11β-HSD1 inhibitor is expected.

As triazole derivatives having 11β-HSD1 -inhibitory activity, the following Patent References 1 to 9 are known.
In Patent Reference 1, a triazole derivative represented by the formula (A) is reported. However, this is different from the compound of the present invention in terms of the absence of the moieties which correspond to the A and B of the compound of the present invention. 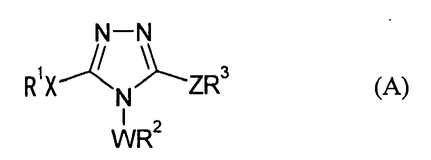 (In the formula, R¹ represents adamantyl which may be substituted, X represents CH₂ or a single bond, and Z represents S or a single bond. See said official gazette for other symbols.)

In Patent Reference 2, a triazole derivative represented by the formula (B) is reported. However, this is different from the compound of the present invention in terms of the absence of the structures which correspond to the A and B of the compound of the present invention. 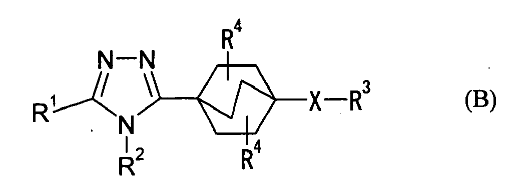 (In the formula, R¹ represents a group selected from arylcarbonyl, -(CH₂)ₙ-aryl and -(CH₂)ₙ-heteroaryl. See said official gazette for other symbols.)

In Patent References 3 and 4, a triazole derivative represented by the formula (C) is reported. However, this is different from the compound of the present invention in terms that the phenyl ring which may be substituted is attached to the triazole ring via one atom of carbon atom. 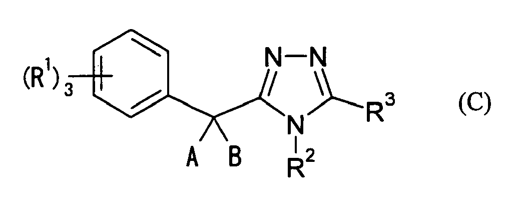 (In the formula, when R² and R³ are separated, R³ represents a group selected from C₁₋₁₄ alkyl, C₂₋₁₀ alkenyl, SC₁₋₆ alkyl, C₆₋₁₀ aryl, heterocycle and heteroaryl, which may be respectively substituted. When A and B are separated, A represents halo, or C₁₋₆ alkyl, OC₁₋₆ alkyl or phenyl, which may be respectively substituted, and B represents H, halo, or C₁₋₆ alkyl, OC₁₋₆ alkyl, SC₁₋₆ alkyl, C₂₋₆ alkenyl, phenyl or naphthyl, which may be respectively substituted. See said official gazette for other symbols.)

In Patent Reference 5, a triazole derivative represented by the formula (D) is reported. However, this is different in terms the 3-position or 5-position of the triazole ring is bonded with oxygen atom or sulfur atom. 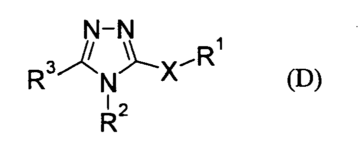 (In the formula, X represents O or S. See said official gazette for other symbols.)

In Patent Reference 6, a triazole derivative represented by the formula (E) is reported. However, this is different from the compound of the present invention in terms that the triazole ring is ring-condensed. 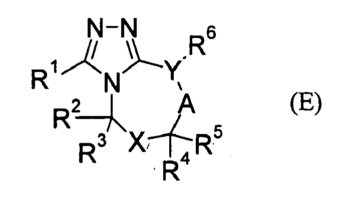 (See said official gazette for symbols in the formula.)

In Patent Reference 7, a triazole derivative represented by the formula (F) is reported. However, when the Ar¹ which corresponds to the R¹ of the compound of the present invention is heteroaryl, only a compound wherein Z-Ar² is phenyl is disclosed as Example. 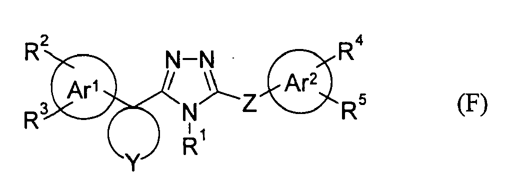 (See said official gazette for symbols in the formula.)

In Patent Reference 8, a compound represented by the formula (G) which includes a broad range of compounds is reported. However, in the case of compounds having substituents which correspond to the A and B of the compound of the present invention, only the compound wherein the moiety which corresponds to the R¹ of the compound of the present invention is aryl is disclosed as Example. 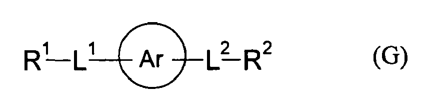 (In the formula, R¹ represents a hydrogen atom or a cyclic group which may be substituted, and R² a cyclic group which may be substituted, Ar a 5- or 6-membered aromatic hetero ring which may be substituted, and L¹ and L² may be the same or different and represent (1) a linking arm, (2) a divalent hydrocarbon group which may be substituted or the like.)

In the Patent Reference 9 which was filed by the present applicant and published after the priority date the instant application, a triazole derivative represented by the formula (H) is reported. However, an illustrative compound wherein the R³ of the formula (H) is the group described in the R³ of the compound of the present invention is not disclosed. 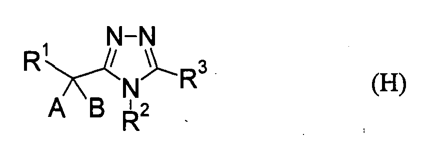 (See said official gazette for symbols in the formula.)

In addition, Patent Reference 10 reports that a triazole derivative represented by the formula (J) is useful as psycholeptic and analgesic, and N-[2-(4-chlorophenyl)ethyl]-N-methyl-1-(5-methyl-4-phenyl-4H-1,2,4-triazol-3-yl)cyclohex-2-ene-1-amine is described therein as Example. However, there are no descriptions on the 11β-HSD1-inhibitory activity and effectiveness for the treatment of diabetes. 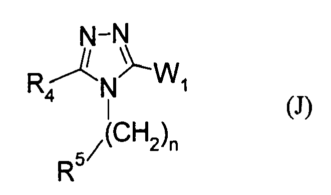 (See said official gazette for symbols in the formula.)

Patent Reference 11 reports that a triazole derivative represented by the formula (K) is useful as analgesic, psycholeptic and ataractic, and (5-chloro-2-{3-[1-(dimethylamino)cyclopropyl]-5-methyl-4H-1,2,4-triazol-4-yl}phenyl)(2-chlorophenyl)methanone is described therein as Example. However, there is no description on the 11β-HSD1-inhibitory activity and effectiveness for the treatment of diabetes. 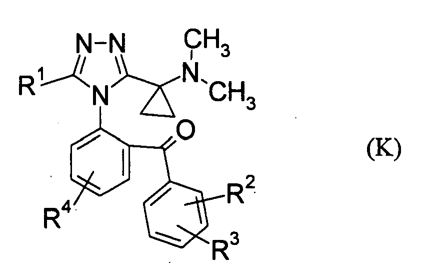 (See said official gazette for symbols in the formula.)

Non-patent Reference 1: Rask E. et al., "The Journal of Clinical Endocrinology & Metabolism", (USA), 2001, vol. 86, p. 1418 - 1421
Non-patent Reference 2: Lindsay R.S. et al., "The Journal of Clinical Endocrinology & Metabolism", 2003, vol. 88, p. 2738 - 2744
Non-patent Reference 3: Masuzaki H. et al., "Science", (USA), 2001, vol. 294, p. 2166 - 2170
Non-patent Reference 4: Masuzaki H. et al., "The Journal of Clinical Investigation", (USA), 2003, vol. 112, p. 83 - 90
Non-patent Reference 5: Morton N.M. et al., "The Journal of Biological Chemistry", (USA), 2001, vol. 276, p. 41293 - 41300
Non-patent Reference 6: Davani B. et al., "The Journal of Biological Chemistry"(USA), 2000, vol. 275, p. 34841 - 34844
Non-patent Reference 7: Cooper M.S. et al., "Bone", (USA), 2000, vol. 27, p. 375 - 381
Non-patent Reference 8: Rauz S. et al., "Investigative Ophthalmology & Visual Science", (USA), 2001, vol. 42, p. 2037 - 2042
Non-patent Reference 9: Sandeep T.C. et al., "Proceedings of the National Academy of Sciences", (USA), 2004, vol. 101, p. 6734 - 6739
Patent Reference 1: International Publication No. 03/65983
Patent Reference 2: US Patent Application Publication No. 2004/133011
Patent Reference 3: International Publication No. 03/104207
Patent Reference 4: International Publication No. 03/104208
Patent Reference 5: International Publication No. 04/089367
Patent Reference 6: International Publication No. 04/089380
Patent Reference 7: International Publication No. 05/044192
Patent Reference 8: JP-A-2005-170939
Patent Reference 9: International Publication No. 06/030805
Patent Reference 10: US Patent No. 4577020
Patent Reference 11: US Patent No. 3907821

### DISCLOSURE OF THE INVENTION

### PROBLEMS THAT THE INVENTION IS TO SOLVE

Since the 11β-HSD1 inhibitors described in the above-mentioned references cannot be satisfied in terms of any one of the efficacy, selectivity, safety and economy, great concern has been directed toward the provision of excellent and selective 11β-HSD1 inhibitor.

### MEANS FOR SOLVING THE PROBLEMS

Under such a situation, the present inventors have conducted extensive studies on the compounds which have an 11β-HSD1-inhibitory activity by which improvement of diabetes and insulin resistance can be expected, and found that the novel triazole derivatives or salts thereof according to the present invention have an excellent and selective inhibitory activity for 11β-HSD1, thus accomplishing the present invention. In addition, these compounds are useful because they are superior to the known 11β-HSD1 inhibitors in terms of any one of the efficacy, selectivity, safety and economy, such as *in vivo* drug effects (blood glucose-lowering action and/or triglyceride-lowering action and the like), pharmacokinetics such as oral absorbability, metabolic stability, or the like, or selectivity from inhibition of cytochrome p450 (CYP) which has a possibility of causing drug interaction, and the like.

That is, the present invention relates to a triazole derivative represented by the following formula (I) or a pharmaceutically acceptable salt thereof, which is useful as an 11β-HSD1 inhibitor. 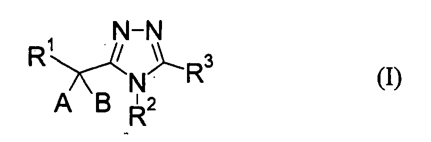 [Symbols in the formula represent the following meanings;
R¹: a heterocyclic group or -N(R⁰)-R⁴,
wherein the heterocyclic group of R¹ may be substituted,
R⁰: -H or lower alkyl,
R⁴: C₁₋₇ alkyl, halogeno-lower alkyl, lower alkyl substituted with cycloalkyl, cycloalkyl, aryl, lower alkylene-aryl, lower alkylene-aromatic heterocyclic group, -S(O)₂-lower alkyl, -S(O)₂-aryl or -S(O)₂-aromatic heterocyclic group,
wherein the cycloalkyl, aryl and aromatic heterocyclic groupof R⁴ may be substituted respectively,
A and B: the same or different from each other and each is lower alkyl, or A and B in combination, and together with the carbon atom to which these are bonded, may form a cycloalkyl ring which may be substituted,
R²: lower alkyl, halogeno-lower alkyl, cycloalkyl, aryl, lower alkylene-CO₂R⁰, lower alkylene-cycloalkyl, lower alkylene-aryl or lower alkylene-aromatic heterocyclic group,
wherein the cycloalkyl, aryl and aromatic heterocyclic group of R² may be substituted respectively,
R³: -H, halogen, lower alkyl, halogeno-lower alkyl, -OR⁰, -CO₂R⁰, cycloalkyl, lower alkylene-cycloalkyl or a saturated heterocyclic group,
wherein the cycloalkyl and a saturated heterocyclic group of R³ may be substituted respectively,
with the proviso that
N-[2-(4-chlorophenyl)ethyl]-N-methyl-1-(5-methyl-4-phenyl-4H-1,2,4-triazol-3-yl)cyclohex-2-ene-1-amine, and
(5-chloro-2-{3-[1-(dimethylamino)cyclopropyl]-5-methyl-4H-1,2,4-triazol-4-yl}phenyl)(2-chlorophenyl)methanone are excluded. The same shall apply hereinafter.]

Further, the present application also relates to a pharmaceutical composition which comprises a triazole derivative represented by the general formula (I) or a pharmaceutically acceptable salt thereof and a pharmaceutically acceptable carrier, particularly a pharmaceutical composition which is an 11β-hydroxysteroid dehydrogenase type 1 inhibitor, an insulin resistance-improving agent or an agent for preventing or treating diabetes.
In addition, the present application also relates to the use of a compound represented by the formula (I) or a pharmaceutically acceptable salt thereof, for the manufacture of an 11β-hydroxysteroid dehydrogenase type 1 inhibitor, an insulin resistance-improving agent or an agent for preventing or treating diabetes, and a method for preventing or treating diabetes, which comprises administering an effective amount of a compound represented by the formula (I) or a pharmaceutically acceptable salt thereof to a patient.
That is, (1) a pharmaceutical composition which comprises a compound described in the formula (I) or a pharmaceutically acceptable salt thereof and a pharmaceutically acceptable carrier.
(2) The pharmaceutical composition described in (1), which is an 11β-hydroxysteroid dehydrogenase type 1 inhibitor;
(3) The pharmaceutical composition described in (1), which is an insulin resistance-improving agent.
(4) The pharmaceutical composition described in (1), which is an agent for preventing or treating diabetes.
(5) Use of the compound described in the formula (I) or a pharmaceutically acceptable salt thereof, for the manufacture of an 11β-hydroxysteroid dehydrogenase type 1 inhibitor, an insulin resistance-improving agent or an agent for preventing or treating diabetes.
(6) A method for preventing or treating diabetes, which comprises administering an effective amount of the compound described in the formula (I) or a salt thereof to a patient.

### EFFECT OF THE INVENTION

The excellent 11β-HSD1-selective inhibitory activity of the compound of the present invention was confirmed by the test methods shown in the following,

### (1) Measuring test of human 11β-HSD1 and 11β-HSD2-inhibitory activities

The procedure for measuring 11β-HSD1-inhibitory activity is as follows. In this connection, the enzyme reaction and measurement were carried out using a 384-well plate. The enzyme was prepared in accordance with a reference (Walker E.A. et al., Journal of Biological Chemistry, 2001, vol. 276,p. 21343 - 21350). The reaction was carried out by adding the compound to be tested having varied concentration to a reaction liquid consisting of 10 mM phosphate buffer (pH 6.6), 20 nM cortisone, 40 µM reduced nicotinamide adenine dinucleotide phosphate (NADPH) and human recombinant 11β-HSD1, and then incubating at room temperature for 1 hour (10 µl/well). The compound to be tested was prepared by dissolving in dimethyl sulfoxide (DMSO) to a DMSO concentration of 1% in the reaction liquid. After the enzyme reaction, the enzyme inhibitory activity was measured by detecting cortisol using a homogeneous time-resolved fluorescence method (HTRF). Each of the XL-665-labeled cortisol containing 400 µM carbenoxolone and cryptate-labeled cortisol antibody (CIS bio international) was added in 5 µl/well portions and incubated at room temperature for 2 hours, and then the fluorescence intensity was measured using a fluorophotometer (trade name: Discovery, Perkin Elmer), and the enzyme inhibitory activity was calculated from the fluorescence intensity ratio at two wavelengths (665 nm/620 nm).
Measurement of the 11β-HSD2 activity was carried out by the same method of the 11β-HSD1 activity measurement, except for the enzyme reaction conditions. The enzyme reaction was carried out by adding the compound to be tested having varied concentration to a reaction liquid consisting of 40 mM Tris-HCl buffer (pH 8.0), 200 nM cortisol, 200 µM nicotinamide adenine dinucleotide (NAD) and human recombinant 11β-HSD2, and then incubating at 37°C for 2 hours (10 µl/well).
The measured result was calculated by averaging the values of 3 wells of the same condition. The ratio when DMSO was added instead of the compound to be tested was regarded as 0% and the ratio when 11β-HSD1 or 11β-HSD2 was not added was regarded as 100%, thereby calculating 50% inhibition concentration of the compound to be tested as IC₅₀ of the compound inhibitory activity.

The IC₅₀ values of typical compounds of the present invention are shown in the following Table 1. In this connection, Ex represents Example number.

**[Table 1]**

| Ex | Human 11β-HSD1 (IC₅₀/µM) | Human 11β-HSD2 (IC₅₀/µM) |
|---|---|---|
| 3 | 0.0079 | > 3 |
| 9 | 0.062 | > 30 |
| 57 | 0.070 | > 30 |
| 77 | 0.012 | > 10 |
| 85 | 0.034 | > 10 |
| 154 | 0.038 | > 30 |
| 167 | 0.070 | > 30 |
| 169 | 0.024 | > 10 |
| 172 | 0.019 | > 10 |
| 173 | 0.012 | > 10 |
| 198 | 0.025 | > 10 |
| 213 | 0.041 | > 10 |
| 280 | 0.0047 | > 3 |
| 281 | 0.0052 | > 3 |
| 283 | 0.0024 | > 3 |

From the above results, it was confirmed that the compounds of the present invention strongly inhibit 11β-HSD1 and that 11β-HSD1-inhibitory activity of the compounds of the present invention is selective against 11β-HSD2.

### (2) Ob/ob mouse blood glucose-lowering test

Using 6% 2-hydroxypropyl-β-cyclodextrin as the solvent, a compound liquid was prepared. Blood glucose values were measured under non-fasting using ob/ob male mice of 9 weeks of age (blood glucose value, 300 mg/dl or more), and then arrangement into groups was carried out at random in such a manner that their blood glucose values became uniform. The compound to be tested was repeatedly orally administered (10 mg/kg, bid) twice a day for 9 days, and blood glucose values after 12 hours of the final administration were measured (n = 6). The blood glucose value was measured by carrying out colorimetric determination of the amount of glucose (mg/dl) in heparin blood plasma obtained by collecting blood in a heparin-coated glass capillary and subsequently centrifuging it.
As a result, Example compound 3 showed a blood glucose -lowering action of 27%, and Example compound 154 that of 2 1 %, Example compound 167 that of 24%, Example compound 172 that of 27% and Example compound 280 that of 35%, so that it was confirmed that the compounds of the present invention have superior blood glucose -lowering action.

### (3) Ob/ob mouse triglyceride-lowering test

Using 6% 2-hydroxypropyl-β-cyclodextrin as the solvent, a compound liquid was prepared. Triglyceride values were measured under non-fasting using ob/ob male mice of 9 weeks of age, and then arrangement into groups was carried out at random in such a manner that their triglyceride values became uniform. The compound to be tested was repeatedly orally administered (10 mg/kg, bid) twice a day for 9 days, and triglyceride values after 12 hours of the final administration were measured (n = 6). Triglyceride was measured by carrying out colorimetric determination of the amount of triglyceride (mg/dl) in heparin blood plasma obtained by collecting blood in a heparin-coated glass capillary and subsequently centrifuging it.
As a result, Example compound 3 showed a triglyceride-lowering action of 50%, and Example compound 280 that of 42%, so that it was confirmed that the compounds of the present invention have superior triglyceride-lowering action.

As a result of the above-mentioned respective tests, it was confirmed that the compounds of the present invention have the 11β-HSD1-inhibitory activity. Based on this, it is evident that these are useful as therapeutic agents for diseases, such as preventive or therapeutic agents for the diseases in which 11β-HSD1 is concerned, such as hyperglycemia, insulin resistance, obesity, hyperlipidemia, hypertension, osteoporosis, glaucoma, lowering of cognition function and the like, particularly hyperglycemia, insulin resistance and the like.

### BEST MODE FOR CARRYING OUT THE INVENTION

The present invention is described further in detail.
In this description, the terms "lower alkyl", "lower alkenyl", "lower alkylidene" and "lower alkylene" mean respectively hydrocarbon chains having from 1 to 6 carbon atoms which may be straight or branched chains unless otherwise noted.
Accordingly, the "lower alkyl" means a C₁₋₆ alkyl and illustrative examples include methyl, ethyl, propyl, butyl, pentyl or hexyl, or the structural isomer thereof such as isopropyl or tert-butyl, preferably a C₁₋₅ alkyl, more preferably methyl, ethyl, propyl, isopropyl, butyl, isobutyl or tert-butyl.

The "lower alkenyl" means a C₂₋₆ alkenyl, which may contain two or more double bonds. Illustrative examples thereof include ethenyl, propenyl, butenyl, pentenyl, hexenyl, butadienyl and the like, of which a C₂₋₃ alkenyl is preferable, and ethenyl, 1-propenyl, 2-propenyl or 3-propenyl is more preferable.

The "lower alkylidene" means a group in which a free valence as a result of removing one hydrogen from a linking arm-possessing carbon atom of a lower alkyl becomes double bond. Illustratively, it is methylidene, ethylidene, propylidene, butylidene, pentylidene, hexylidene or the like. Preferred is a C₁₋₃ alkylidene, more preferred is methylidene.

The "alkylene" means a divalent group as a result of removing one hydrogen at an optional position of alkyl. The "lower alkylene" means a C₁₋₆ alkylene. Illustratively, it is methylene, ethylene, methylmethylene, dimethylmethylene, propylene, butylene, pentylene, hexylene or the like. preferred is a C₁₋₃ alkylene, more preferred is methylene, ethylene, methylmethylene, dimethylmethylene or propylene.

The "cycloalkyl" means a C₃₋₁₀ non-aromatic hydrocarbon ring which may form a bridged ring or spiro ring. In addition, it may also have a partially unsaturated bond. Illustrative examples thereof include cyclopropyl, cyclobutyl, cyclopentyl, cyclohexyl, cyclooctyl, cyclohexenyl, cyclobutenyl, adamantly, norbornyl and the like, of which a C₃₋₆ cycloalkyl is preferable, and cyclopropyl, cyclobutyl, cyclobutenyl, cyclopentyl or cyclohexyl is more preferable.

The "cycloalkyl ring" which is formed from A and B in combination together with the carbon atom to which these are bonded means a divalent group of a C₃₋₁₀ non-aromatic hydrocarbon ring which may form a bridged ring or spiro ring. In addition, it may also have a partially unsaturated bond. Illustrative examples thereof include cyclopropyl ring (cyclopropane-1,1-diyl), cyclobutyl ring (cyclobutane-1,1-diyl), cyclopentyl ring (cyclopentane-1,1-diyl), cyclohexane ring (cyclohexane-1,1-diyl), cyclobutenyl ring (cyclobut-2-ene-1,1-diyl) and the like, of which a C₃₋₅ cycloalkyl ring is preferable, and cyclopropyl ring, cyclobutyl ring or cyclobutenyl ring is more preferable.

The "halogen" means a halogen atom, and illustrative examples thereof include fluoro, chloro, bromo, iodo and the like, of which fluoro and chloro are preferable.

The "halogeno-lower alkyl" means a group in which at least one optional hydrogen atom of the aforementioned "lower alkyl" is substituted with the aforementioned "halogen" which may be the same or different from each other. Illustrative examples thereof include trifluoromethyl, pentafluoromethyl and the like, of which trifluoromethyl is preferable.

The "aryl" means a monocyclic to tricyclic C₆₋₁₄ aromatic hydrocarbon ring, and illustrative examples thereof include phenyl, naphthyl and the like, of which phenyl is preferable.

The "heterocyclic" group means a cyclic group consisting of i) monocyclic 3-to 8-membered (preferably 5- to 7-membered) hetero ring having from 1 to 4 hetero atoms selected from O, S and N, and ii) a bicyclic 8- to 14-membered (preferably 9- to 11-membered) hetero ring or tricyclic 11- to 20-membered (preferably 12 to 15-membered) hetero ring having from 1 to 5 hetero atoms selected from O, S and N, which is formed by the ring condensation of said monocyclic hetero ring with one or two rings selected from the group consisting of a monocyclic hetero ring, benzene ring and a C₅₋₈ cycloalkyl. An oxide or dioxide may be formed through the oxidation of S or N as the ring atom. Preferred as the "heterocyclic" group is aziridinyl, azetidinyl, pyrrolidinyl, piperidinyl, piperazinyl, homopiperazinyl, oxiranyl, oxetanyl, tetrahydrofuranyl, tetrahydropyranyl, morpholinyl, homomorpholinyl, tetrahydrothiopyrannyl, pyrrolinyl, imidazolyl, triazolyl, tetrazolyl, pyridyl, pyrimidinyl, pyrazinyl, furyl, thienyl, oxazolyl, oxadiazolyl, thiazolyl, thiadiazolyl, indolyl, dihydroisoindolyl, indolizinyl, benzimidazolyl, imidazo[1,2-a]pyridinyl, quinoxalinyl, quinolyl, isiquinolyl, quinazolyl, cinnolinyl, phthalazyl, benzofuranyl, benzothienyl, benzoxazolyl, benzothiazolyl, carbazolyl or quinuclidinyl, and more preferred is piperidinyl, tetrahydrofuranyl or dihydroisoindolyl.

The "saturated heterocyclic" group is the saturated heterocyclic group among the above-mentioned "heterocyclic" groups such as aziridinyl, azetidinyl, pyrrolidinyl, piperidinyl, piperazinyl, homopiperazinyl, oxiranyl, oxetanyl, tetrahydrofuranyl, tetrahydropyranyl, morpholinyl, homomorpholinyl, tetrahydrothiopyrannyl and the like. Pyrrolidinyl, piperidinyl, piperazinyl, tetrahydrofuranyl, tetrahydropyranyl and tetrahydrothiopyrannyl are preferable, and piperidinyl and tetrahydropyranyl are more preferable.

The "aromatic hetero ring" means, among the above-mentioned "heterocyclic" groups, a monocyclic 3- to 8-membered, preferably 5- to 7-membered, monocyclic aromatic hetero ring having from 1 to 4 hetero atoms selected from O, S and N, and a bicyclic or tricyclic hetero ring which is formed by the ring condensation of said aromatic hetero rings or of said aromatic hetero ring with benzene ring. An oxide may be formed through the oxidation of S or N as the ring atom. For example, pyridyl, pyridazinyl, pyrimidinyl, pyrazinyl, furyl, thienyl, pyrrolyl, oxazolyl, isoxazolyl, oxadiazolyl, thiazolyl, thiadiazolyl, imidazolyl, triazolyl, tetrazolyl, benzofuranyl, benzothienyl, benzoxazolyl, benzimidazolyl, benzothiazolyl, quinolinyl, quinazolinyl, quinoxalinyl, cinnolinyl and the like may be cited. Pyridyl, pyrimidinyl, pyrazinyl, thienyl, pyrrolyl, thiazolyl and quinolyl are preferable, and pyridyl and thienyl are more preferable.

The "may be substituted" means "not substituted" or "substituted with the same or different 1 to 5 substituents".

According to this description, as the acceptable substituent regarding the term "may be substituted", it may be any substituent as long as it is a substituent generally used in said technical field as the substituent for respective group.

As the acceptable substituent for the "heterocyclic group" which may be substituted regarding R¹, preferably a group selected from the following group G¹ may be cited, more preferably a group selected from the group consisting of halogen, lower alkyl, halogeno-lower alkyl, cyano, -O-lower alkyl and -O-halogeno-lower alkyl may be cited, further preferably a group selected from the group consisting of halogen, lower alkyl and halogeno-lower alkyl may be cited, and further more preferably a group selected from the group consisting of halogen and halogeno-lower alkyl may be cited. Group G¹: halogen, lower alkyl, lower alkenyl, halogeno-lower alkyl, cyano, -OR⁰, -O-halogeno-lower alkyl, lower alkylene-OR⁰, lower alkylene-O-lower alkylene-cycloalkyl, -C(O)R⁰, -CO₂R⁰ and oxo.

As the acceptable substituent for the "cycloalkyl", "aryl" and "aromatic heterocyclic group" which may be respectively substituted regarding R⁴, a group selected from the group consisting of halogen and lower alkyl may be preferably cited and, more preferably, halogen may be cited.

As the acceptable substituent for the "cycloalkyl ring" which may be substituted and is formed from A and B in combination together with the carbon atom to which these are bonded, a group selected from the group consisting of halogen and -OH may preferably be cited.

As the acceptable substituent for the "aryl" and "aromatic heterocyclic group" which may be substituted regarding R², a group selected from the group consisting of halogen, lower alkyl, -OR⁰ and -O-halogeno-lower alkyl may preferably be cited and, more preferably, halogen may be cited.

As the acceptable substituent for the "cycloalkyl" which may be substituted regarding R², a group selected from the group consisting of halogen, lower alkyl and aryl can be preferably cited, and halogen can be cited more preferably.

As the acceptable substituent for the "cycloalkyl" and "saturated heterocyclic group" which may be substituted regarding R², a group selected from the group consisting of halogen, lower alkyl, -OR⁰,-O-halogeno-lower alkyl, -CO₂R⁰, lower alkylidene and oxo may preferably be cited and, more preferably, lower alkyl may be cited.

Preferred embodiment regarding the compound of the present invention represented by the general formula (I) is shown in the following.
Preferred as R¹ is aromatic heterocyclic group which may be substituted, more preferred is pyridyl, thienyl, pyrrolyl, quinolyl, thiazolyl, pyrimidyl or pyrazyl which may respectively be substituted, further more preferred is pyridyl or thienyl which may be respectively substituted with a group selected from the group consisting of lower alkyl, halogen and halogeno-lower alkyl, and particularly preferred is thienyl which may be substituted with a group selected from the group consisting of lower alkyl, halogen and halogeno-lower alkyl.
Preferred as A and B is methyl.
Alternatively, preferred as the ring formed by A and B in combination together with the carbon atom to which these are bonded is cyclobutyl ring or cyclobutenyl ring which may respectively be substituted, more preferred is cyclobutyl ring or cyclobutenyl ring which may respectively be substituted with halogen or -OH, and further preferred is cyclobutyl ring which may be substituted with halogen or -OH.
Preferred as R² is lower alkyl, cycloalkyl, lower alkylene-(aryl which may be substituted) or lower alkylene-aromatic heterocyclic group, more preferred is lower alkyl, cycloalkyl or lower alkylene-(aryl which may be substituted), further more preferred is methyl, cyclopropyl, -(CH₂)₂-(phenyl which may be substituted with halogen) or -(CH₂)₂-pyridyl, further preferred is cyclopropyl or -(CH₂)₂-(phenyl which may be substituted with halogen), and further more preferred is cyclopropyl.
Preferred as R³ is lower alkyl or cycloalkyl which may be substituted, more preferred is cycloalkyl which may be substituted with lower alkyl, further preferred is cyclopropyl or cyclobutyl which may be substituted with a lower alkyl, further more preferred is cyclopropyl or cyclobutyl which may be substituted with methyl, and particularly preferred is cyclopropyl.
In addition, a compound consisting of a combination of the above-described preferred groups is more desirable.

In addition, other preferred compounds among the compounds of the present invention represented by the general formula (I) are shown below.
(1) The compound described in the formula (I), wherein R³ is lower alkyl, or cycloalkyl which may be substituted.
(2) The compound described in (1), wherein A and B are both methyl; or the ring formed by A and B in combination together with the carbon atom to which these are bonded is cyclobutyl ring or cyclobutenyl ring which may respectively be substituted.
(3) The compound described in (2), wherein R¹ is aromatic heterocyclic ring which may be substituted.
(4) The compound described in (3), wherein R² is lower alkyl, cycloalkyl or lower alkylene-(aryl which may be substituted).
(5) The compound described in (4), wherein R³ is cyclopropyl or cyclobutyl which may be respectively substituted with lower alkyl.
(6) The compound described in (5), wherein A and B are both methyl.
(7) The compound described in (6), wherein R¹ is pyridyl or thienyl which may respectively be substituted with a group selected from the group consisting of halogen, lower alkyl and halogeno-lower alkyl.
(8) The compound described in (7), wherein R² is cyclopropyl or -(CH₂)₂-(phenyl which may be substituted with halogen).
(9) The compound described in the formula (I) selected from the group consisting of
   3-[1-(5-bromo-2-thienyl)-1-methylethyl]-4,5-dicyclopropyl-4H-1,2,4-triazole,
   2-(1-{5-cyclopropyl-4-[2-(2,6-difluorophenyl)ethyl]-4H-1,2,4-triazol-3-yl}-1-methylethyl)pyridine,
   3,4-dicyclopropyl-5-{1-methyl-1-[5-(trifluoromethyl)-2-thienyl]ethyl}-4H-1,2,4-triazole, and
   3,4-dicyclopropyl-5-{1-[3-fluoro-5-(trifluoromethyl)-2-thienyl]-1-methylethyl}-4H-1,2,4-triazole,
or a pharmaceutically acceptable salt thereof.

There is a case in which the triazole derivatives represented by the formula (I) form salts, and such salts are included in the compounds of the present invention as long as they are pharmaceutically acceptable salts. Illustratively, acid addition salts with inorganic acids (e.g., hydrochloric acid, hydrobromic acid, hydriodic acid, sulfuric acid, nitric acid, phosphoric acid and the like), or organic acids (e.g., formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, p-toluenesulfonic acid, aspartic acid, glutamic acid and the like), salts with inorganic bases including metals such as sodium, potassium, calcium, magnesium and the like or with organic bases (e.g., methylamine, ethylamine, ethanolamine, lysine, ornithine and the like), ammonium salts and the like may be exemplified.

Also, the compound of the present invention may have an asymmetric carbon atom in some cases depending on the kind of substituents, and optical isomers based on the can be present. The present invention includes all of the mixtures and separated forms of these optical isomers. Also, tautomers are present in the compounds of the present invention in some cases, and the present invention also includes mixtures and separated forms of these isomers. In addition, a labeled substance, namely a compound in which at least one atom of the compound of the present invention is replaced by a radioisotope or non-radioactive isotope, is also included in the present invention.

In addition, the present invention also includes various types of hydrate and solvate and polymorphism of the compounds of the present invention. In this connection, as a matter of course, the compounds of the present invention are not limited to the compounds described in the Examples which are described later, and all of the derivatives represented by the formula (I) and pharmaceutically acceptable salts thereof are included therein.

In this connection, all of the compounds which are metabolized in the living body and thereby converted into the compounds of the present invention, so-called prodrugs, are also included in the compounds of the present invention. As the groups which can form prodrugs of the compounds of the present invention, the groups described in "Progress in Medicines", Life Science Medica, 1985, vol. 5,pp. 2157 - 2161, and in "Iyakuhin no Kaihatsu (Development of Medicines)" published by Hirokawa Shoten in 1990, vol. 7 Bunshi Sekkei (Molecular Design), pp. 163 - 198, may be exemplified.

### (Production methods)

The compound of the present invention and a pharmaceutically acceptable salt thereof may be produced by employing various known synthesis methods making use of the characteristics based on its basic skeleton or kind of the substituents. In this connection, depending on the kind of functional group, there is an effective case from the production technology point of view to replace said functional group with an appropriate protecting group, namely a group which may be easily converted into said functional group, at the stage of the starting material to the intermediate. Thereafter, the desired compound may be obtained by removing the protecting group as occasion demands. As such a functional group, hydroxyl group, carboxyl group, amino group and the like may for example be cited, and as their protecting groups, the protecting groups described for example in "Protective Groups in Organic Synthesis" edited by Greene and Wuts, (USA), 3rd edition, John Willey & Sons, 1999, may be cited, which may be optionally used in response to the reaction conditions.

### (First production method)

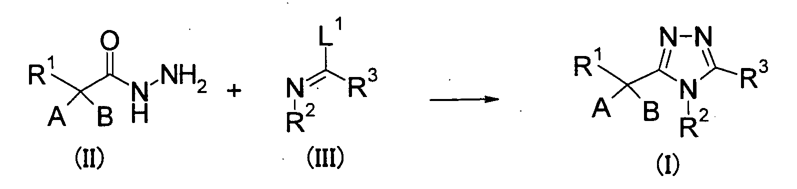

(In the formula, L¹ represents a leaving group.)
This production method is a method in which the compound (I) of the present invention is produced by a cyclization reaction of a compound (II) and a compound (III). In this case, for example, chloro, bromo, methoxy, methylsulfanyl and the like may be cited as the leaving group of L¹. The reaction may be carried out at room temperature or under a heating condition in a solvent such as ethers (e.g., tetrahydrofuran (THF), 1,4-dioxane, diglyme and the like), alcohols (e.g., methanol, ethanol, propanol, butanol and the like) or aprotic polar solvents (e.g., N,N-dimethylformamide (DMF), dimethylimidazolidinone, dimethylacetamide, DMSO and the like), and the like. Depending on the compounds, it is advantageous in some cases to carry out the reaction in the presence of an acid such as an organic acid such as acetic acid, p-toluenesulfonic acid or the like or a minieral acid such as sulfuric acid, hydrochloric acid or the like.

### (Second production method)

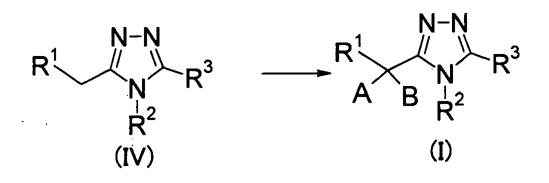

This production method is a method in which the compound (I) of the present invention is produced from a compound (IV) by an alkylation reaction. The alkylation reaction of this process may use sodium hydride, potassium hydride, butyl lithium, lithium diisopropylamide or the like as the base, and corresponding alkyl halide, dihalogenated alkane or the like as the electrophilic reagent. The reaction may be carried out under cooling, under room temperature or under a heating condition in a solvent such as ethers or aprotic polar solvents.
Depending on the compounds, it is advantageous in some cases to carry out the reaction in the presence of a phase-transfer catalyst such as tetra-n-butylammonium iodide or the like.

### (Third production method)

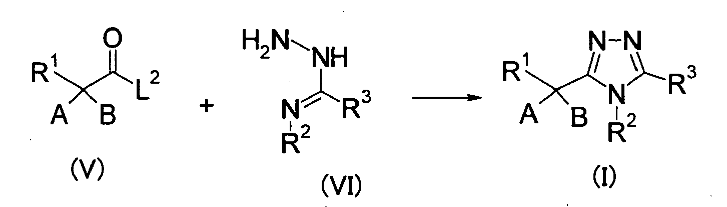

(In the formula, L² represents a leaving group. The same shall apply hereinafter.)
This production method is a method in which the compound (I) of the present invention is produced by a cyclization reaction of a compound (V) as an activated carboxylic acid derivative and a compound (VI). In this case, examples of the leaving group of L² include chloro, bromo, fluoro, acyloxy and the like. The reaction may be carried out under room temperature or under a heating condition in a solvent such as ethers, alcohols or aprotic polar solvents. Depending on the compounds, it is advantageous in some cases to carry out the reaction in the presence of an acid such as organic acid (e.g., acetic acid, p-toluenesulfonic acid or the like) or mineral acid (e.g., sulfuric acid, hydrochloric acid or the like).

### (Fourth production method)

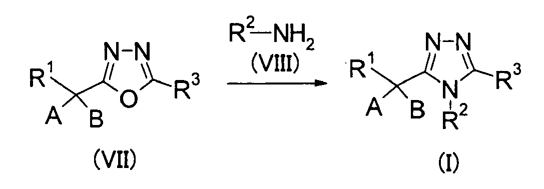

This production method is a method in which the compound (I) of the present invention is obtained by allowing a compound (VII) and a compound (VIII) to undergo the reaction.
The reaction may be carried out using the compound (VII) and compound (VIII) in equivalent amount, or one of them in an excess amount, from under room temperature to under heating, preferably under heating, in a reaction inert solvent such as alcohols, aromatic hydrocarbons (e.g., benzene, toluene, xylene and the like), acetic acid or the like, or under no solvent. Depending on the compounds, it is advantageous in some cases to carry out the reaction in the presence of an acid such as organic acid (e.g., acetic acid, p-toluenesulfonic acid or the like) or mineral acid (e.g., sulfuric acid, hydrochloric acid or the like). Also, it is advantageous in some cases to carry out the reaction using a microwave.

### (Fifth production method)

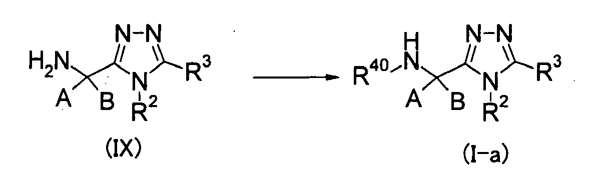

(In the formula, R⁴⁰ represents C₁₋₇ alkyl, halogeno-lower alkyl, lower alkyl substituted with cycloalkyl, cycloalkyl, aryl, lower alkylene-aryl or lower alkylene-aromatic heterocyclic group. The same shall apply hereinafter.)
This production method is a method in which the compound (I-a) of the present invention is obtained by subjecting a compound (IX) to reductive alkylation.
The reductive alkylation reaction may be carried out using the compound (IX) and an aldehyde or ketone which corresponds to R⁴⁰, in equivalent amounts or one of them in an excess amount, in the presence of a reducing agent under from cooling to under reflux with heating in a reaction inert solvent such as alcohols, ethers or the like. As the reducing agent, sodium cyanoborohydride, sodium triacetoxyborohydride, sodium borohydride and the like may be cited. It is desirable in some cases to carry out the reaction in the presence of an dehydrating agent such as molecular sieves or the like or an acid such as acetic acid, hydrochloric acid, titanium(IV) isopropoxide complex or the like. Depending on the reaction, when an imine compound formed as an intermediate in the reaction system may be stably isolated, a reducing reaction may be separately carried out after obtaining said imine compound.

### (Sixth production method)

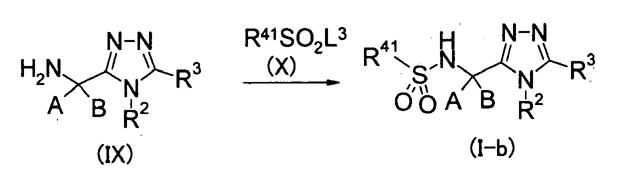

(In the formula, L³ represents a leaving group, and R⁴¹ represents a lower alkyl, aryl or aromatic heterocyclic group. The same shall apply hereinafter.)
This production method is a method in which the compound (I-b) of the present invention is obtained by allowing the compound (IX) and a compound (X) to undergo the reaction. In this case, examples of the leaving group of L³ include chloro, bromo, fluoro and the like.
The reaction may be carried out using the compound (IX) and compound (X) in equivalent amounts, or one of them in an excess amount, from under cooling to under heating, in a reaction inert solvent such as ethers, halogenated hydrocarbons (e.g., dichloromethane, 1,2-dichloroethane, chloroform or the like), an aprotic polar solvent or the like. Depending on the compounds, for effecting smooth progress of the reaction, it is advantageous in some cases to carry out the reaction in the presence of an organic base (e.g., triethylamine, N,N-diisopropylethylamine, N-methylmorpholine or the like) or an inorganic base (e.g., potassium carbonate, sodium carbonate or the like).

In addition, some compounds represented by the formula (I) may also be produced from the compounds of the present invention obtained in the above manner, by optionally combining known steps which may generally be employed by those skilled in the art, such as alkylation, acylation, substitution reaction, oxidation, reduction, hydrolysis and the like.

The starting materials to be used in the production of the compounds of the present invention may be produced, for example, by employing the following methods, the methods described in Reference Examples which are described later, known methods or methods obvious to those skilled in the art or modified methods thereof.

### (Starting material synthesis 1)

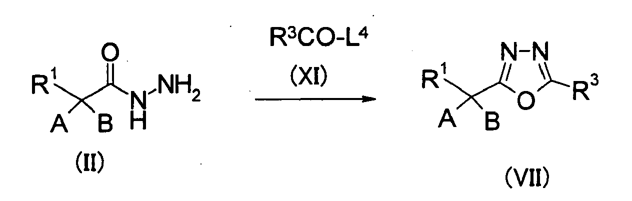

(In the formula, L⁴ represents a leaving group. The same shall apply hereinafter.)
The compound (VII) may be produced by a cyclization reaction of the compound (II) and compound (XI). In this case, examples of the leaving group of L⁴ include chloro, bromo, fluoro, hydroxy and the like.
It may be produced by a reaction in which the (II) and (XI) are allowed to undergo condensation under room temperature or under a heating condition in a solvent such as an aprotic polar solvent (e.g., halogenated hydrocarbons or the like), or the like, and allowing a dehydrating agent (e.g., phosphorus oxychloride, trifluoromethanesulfonic acid anhydride, or the like) to act upon the resulting diacyl compound. Depending on the compounds, for effecting smooth progress of the reaction, it is advantageous in some cases to carry out the reaction in the presence of an organic base (e.g., triethylamine, N,N-diisopropylethylamine, pyridine or the like) or an inorganic base (e.g., potassium carbonate, sodium carbonate or the like).

### (Starting material synthesis 2)

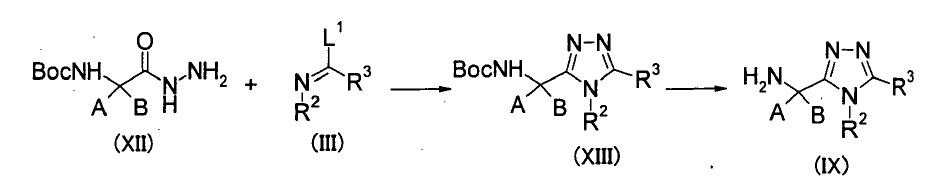

(In the formula, Boc represents tert-butoxycarbonyl group. The same shall apply hereinafter.)
The compound (IX) may be produced by deprotecting a compound (XIII). Deprotection of Boc may be carried out by a method generally used by those skilled in the art. For example, it may be carried out by the method described in the aforementioned "Protective Groups in Organic Synthesis".
The compound (XIII) may be produced from a compound (XII) and the compound (III) in the same manner as in the first production method.

The compound of the present invention produced in this manner is isolated and purified directly as such or as a salt thereof by applying a salt formation treatment in the usual way. The isolation and purification are carried out by employing general chemical operations such as extraction, concentration, evaporation, distillation, crystallization, filtration, recrystallization, various types of chromatography and the like.
Various types of isomers may be isolated in the usual way making use of the difference in the physicochemical properties between isomers. For example, a racemic mixture may be converted into an optically pure isomer by a general racemic resolution such as, for example, a method in which these are converted into diastereomer salts with an optically active organic acid (e.g., tartaric acid or the like) and then subjected to optical resolution. Also, a diastereomer mixture may be separated, for example, by a fractional recrystallization or various types of chromatography. In addition, an optically active compound may also be produced using an appropriate optically active compound as the starting material.

The pharmaceutical composition which contains one or more of the compounds of the present invention or pharmaceutically acceptable salts thereof as the active ingredient is prepared into tablets, powders, fine subtilaes, granules, capsules, pills, solutions, injections, suppositories, ointments, adhesive preparations and the like using generally used pharmaceutical carriers, fillers and other additives pharmaceutical preparation use and orally or parenterally administered.
Clinical dose of the compound of the present invention in human is optionally decided by taking into consideration symptoms, weight, age, sex and the like of the patient to be treated, but the daily dose is usually from about 0.0001 to 50 mg/kg, preferably from about 0.001 to 10 mg/kg body weight, more from about 0.01 to 1 mg/kg, in the case of oral administration, and this is administered in one portion or by dividing into 2 to 4 portions. In the case of intravenous administration, the daily dose is from about 0.0001 to 1 mg/kg body weight, preferably from about 0.0001 to 0.1 mg/kg, and this is administered once a day or by dividing it into two or more times. Since the dose varies under various conditions, there is a case in which a sufficient effect is obtained by a smaller dose than the above-mentioned administration range.

As the solid composition for use in the oral administration according to the present invention, tablets, powders, granules and the like are used. In such a solid composition, one or more active substances are mixed with at least one inert diluent such as lactose, mannitol, glucose, hydroxypropylcellulose, microcrystalline cellulose, starch, polyvinyl pyrrolidone, aluminum magnesium silicate or the like. In the usual way, the composition may contain other additives than the inert diluent, such as a lubricant (e.g., magnesium stearate or the like), a disintegrating agent (calcium cellulose glycolate or the like), a stabilizing agent, solubilizing agent and the like. When necessary, tablets or pills may be coated with a sugar coating or film of a gastric or enteric substance, such as of sucrose, gelatin, hydroxypropylcellulose, hydroxypropylmethylcellulose phthalate or the like.

The liquid composition for oral administration includes pharmaceutically acceptable emulsions, solutions, suspensions, syrups, elixirs and the like and contains a generally used inert diluent such as purified water or ethanol EtOH). In addition to the inert diluent, this composition may contain auxiliary agents such as a moistening agent, a suspending agent and the like, as well as sweeteners, flavors, aromatics and antiseptics.
As the injections for parenteral administration, aseptic aqueous or non-aqueous solutions, suspensions and emulsions are included. As the aqueous solutions and suspensions, for example, distilled water for injection and physiological saline are included. As the non-aqueous solutions and suspensions, for example, there are propylene glycol, polyethylene glycol, plant oil (e.g., olive oil or the like), alcohols (e.g., EtOH or the like), Polysorbate 80 and the like. Such a composition may further contain auxiliary agents such as an antiseptic, a moistening agent, an emulsifying agent, a dispersing agent, a stabilizing agent, a solubilizing agent or the like. These are sterilized by filtration through a bacteria retaining filter, blending of a germicide or irradiation. These may also be used by producing sterile solid compositions and dissolving them in sterile water or a sterile solvent for injection prior to their use.

### EXAMPLES

The present invention is illustratively described by the following Examples, but the present invention is not restricted by these Examples. In this connection, since novel substances are included in the starting compounds to be used in the Examples, production methods of such starting compounds are described as production methods.
In this connection, symbols in the Examples represent the following meanings (the same shall apply hereinafter).
Rf: Production Example number, Ex: Example number, No: compound number, Structure: structural formula, Data: physical data (EI: EI-MS (Pos); ESP: ESI-MS (Pos); ESN: ESI-MS (Neg); FP: FAB-MS (Pos); FN: FAB-MS (Neg); CI: CI-MS (Pos); NMR1: δ (ppm) of characteristic peak of ¹H-NMR in DMSO-d₆; NMR2: δ (ppm) of characteristic peak of ¹H-NMR in CDCl₃; Sal: salt (No description means free form, and the numeral before the salt shows compositional ratio. For example, when 2HCl is described, it shows that the compound is dihydrochloride.)), Me: methyl; Et: ethyl; iPr: isopropyl; cPr: cyclopropyl; iBu: isobutyl; tBu: tert-butyl; cBu: cyclobutyl; iPen: isopentyl; cPen: cyclopentyl; cHex: cyclohexyl; Ph: phenyl; Bn: benzyl; Bz: benzoyl; MOM: methoxymethyl; Boc: tert-butoxycarbonyl; HOBt: 1-hydroxybenzotriazole; WSC: 1-ethyl-3-(3-dimethylaminopropyl)carbodiimide; The numeral before substituent shows substituting position, and therefore for example, 4-Cl-5-F means 4-chloro-5-fluoro.), Syn: production method (The numeral shows that, similar to Example compound having the number as its Example number, it was produced using the corresponding starting material.), RSyn: production method (The numeral shows that, similar to the production example compound having the number as its production example number, it was produced using the corresponding starting material.).

### Production Example 1

3-Chloro-2-thiophene-carboxylic acid and 1,1'-carbonylbis-1H-imidazole were allowed to undergo reaction in THF under ice-cooling, and then sodium borohydride and water were added to this mixed liquid, followed by reaction at room temperature to obtain (3-chloro-2-thienyl)methanol.

### Production Example 2

By adding a catalytically effective amount of pyridine, (3-chloro-2-thienyl)methanol and thionyl chloride were allowed to undergo reaction at room temperature in dichloromethane to obtain 3-chloro-2-(chloromethyl)-thiophene. By allowing 3-chloro-2-(chloromethyl)-thiophene to react with sodium cyanide in DMSO, (3-chloro-2-thienyl)acetonitrile was obtained.

### Production Example 3

A DMF solution of (3-chloro-2-thienyl)acetonitrile and methyl iodide was added to a DMF solution of sodium hydride, which was allowed to undergo reaction at room temperature to obtain 2-(3-chloro-2-thienyl)-2-methyl-propanenitrile.

### Production Example 4

By allowing 2-(3-chloro-2-thienyl)-2-methylpropanenitrile and potassium hydroxide in ethylene glycol to undergo the reaction under heating, 2-(3-chloro-2-thienyl)-2-methylpropanecarboxylic acid was obtained.

### Production Example 5

By allowing 2-(3-chloro-2-thienyl)-2-methylpropanecarboxylic acid to react with hydrazine monohydrate, HOBt monohydrate and WSC monohydrochloride in dichloromethane at room temperature, 2-(3-chloro-2-thienyl)-2-methylpropanohydrazide was obtained.

### Production Example 6

By allowing ethyl 2-methyl-2-(2-thienyl)propanoate and hydrazine monohydrate in ethanol to undergo the reaction under heating, 2-methyl-2-(2-thienyl)propanohydrazide was obtained.

### Production Example 7

Ethyl pyridin-4-ylacetate and metachloroperbenzoic acid in dichloromethane and in a saturated sodium bicarbonate aqueous solution were allowed to undergo the reaction at room temperature to obtain ethyl (1-oxidopyridine-4-yl)acetate.

### Production Example 8

1,3-Dibromo-2-propanol, dimethoxymethane and boron trifluoride diethyl ether complex in dichloromethane were allowed to undergo the reaction at room temperature to obtain 1,3-dibromo-2-(methoxymethoxy)propane.

### Production Example 9

Thiophene-2-acetonitrile and 1,3-dibromo-2-(methoxymethoxy)propane were added to a DMF solution of sodium hydride, which was allowed to undergo the reaction at room temperature to obtain 3-(methoxymethoxy)-1-(2-thienyl)cyclobutanecarbonitrile.

### Production Example 10

A DMF solution of ethyl (1-oxidopyridin-4-yl)acetate and methyl iodide was added to a DMF solution of sodium hydride, which was allowed to undergo the reaction under ice-cooling to obtain ethyl 2-methyl-2-(1-oxidopyridin-4-yl)propanoate.

### Production Example 11

By allowing ethyl 2-methyl-2-(1-oxidopyridin-4-yl)propanoate and 10% palladium carbon to undergo the reaction in an acetic acid-ethyl acetate solution under 3 atmospheric pressure of hydrogen to obtain ethyl 2-methyl-2-pyridin-4-ylpropanoate.

### Production Example 12

By allowing 3-(methoxymethoxy)-1-(2-thienyl)cyclobutanecarbonitrile and potassium hydroxide to undergo the reaction under heating in ethylene glycol, 3-(methoxymethoxy)-1-(2-thienyl)cyclobutanecarboxylic acid was obtained.

### Production Example 13, 14

After heating a mixture of N-cyclopropylcyclopropanecarboxamide and methyl trifluoromethanesulfonic acid at 60°C, toluene, triethylamine and 3-(methoxymethoxy)-1-(2-thienyl)cyclobutanecarbohydrazide were added thereto, followed by reaction by heating to 60°C and then to 110°C. Then, purification was carried out by silica gel column chromatography to obtain 3 ,4-dicyclopropyl-5-[cis-3-(methoxymethoxy)-1-(2-thienyl)cyclobutyl]-4H-1,2,4-triazole (Production Example 13) and 3,4-dicyclopropyl-5-[trans-3-(methoxymethoxy)-1-(2-thienyl)cyclobutyl]-4H-1,2,4-triazole (Production Example 14)

### Production Example 15

By allowing 3,4-dicyclopropyl-5-[cis-3-(methoxymethoxy)-1-(2-thienyl)cyclobutyl]-4H-1,2,4-triazole and N-chlorosuccinimide to undergo reaction in acetic acid at 80°C, 3-[cis-1-(5-chloro-2-thienyl)-3-(methoxymethoxy)cyclobutyl]-4,5-dicyclopropyl-4H-1,2,4-triazole was obtained.

### Production Example 16

By allowing 3,4-dicyclopropyl-5-[cis-3-(methoxymethoxy)-1-(2-thienyl)cyclobutyl]-4H-1,2,4-triazole and N-bromosuccinimide to undergo reaction in acetic acid at room temperature, 3-[cis-1-(5-bromo-2-thienyl)-3-(methoxymethoxy)cyclobutyl]-4,5-dicyclopropyl-4H-1,2,4-triazole was obtained.

### Production Example 17

By heating N-(2-methyl-2-pyridin-2-ylpropanoyl)cyclopropanecarbohydrazide and phosphoryl chloride, 2-[1-(5-cyclopropyl-1,3,4-oxadiazol-2-yl)-1-methylethyl]pyridine was obtained.

### Production Example 18

In the presence of N,N-diisopropylethylamine, pivaloyl chloride and cyclopropanamine were allowed to undergo reaction in dichloromethane at room temperature to obtain N-cyclopropyl-2,2-dimethylpropanamide.

### Production Example 19

After heating a mixture of N-cyclopropyl-2,2-dimethylpropanamide and methyl trifluoromethanesulfonate at 60°C, toluene, triethylamine and 3-(methoxymethoxy)-1-(2-thienyl)cyclobutanecarbohydrazide were added thereto, followed by reaction by heating to 60°C and then to 110°C, thereby obtaining 3-tert-butyl-4-cyclopropyl-5-[3-(methoxymethoxy)-1-(2-thienyl)cyclobutyl]-4H-1,2,4-triazole.

### Production Example 20

By allowing 3-tert-butyl-4-cyclopropyl-5-[3-(methoxymethoxy)-1-(2-thienyl)cyclobutyl]-4H-1,2,4-triazole to react with 6 M hydrochloric acid aqueous solution in THF at room temperature, 3-(5-tert-butyl-4-cyclopropyl-4H-1,2,4-triazol-3-yl)-3-(2-thienyl)cyclobutanol was obtained.
By allowing 3-(5-tert-butyl-4-cyclopropyl-4H-1,2,4-triazol-3-yl)-3-(2-thienyl)cyclobutanol to react with pyridine and benzoyl chloride at room temperature in dichloromethane, trans-3-(5-tert-butyl-4-cyclopropyl-4H-1,2,4-triazol-3-yl)-3-(2-thienyl)cyclobutyl benzoate was obtained.

### Production Example 21

After heating a mixture of N,2-dimethylpropanamide and methyl trifluoromethane sulfonate at 60°C, toluene and 2-methyl-2-pyridin-2-ylpropanohydrazide were added thereto, followed by heating at 60°C. Triethylamine was further added thereto, followed by heating at 60°C to effect the reaction, thereby obtaining 2-[1-(5-isopropyl-1,3,4-oxadiazol-2-yl)-1-methylethyl]pyridine.

### Production Example 22

By allowing aniline, ethyl 2-bromoisobutyrate and potassium carbonate to undergo reaction in DMF at 90°C, ethyl 2-anilino-2-methylpropanoate was obtained.

### Production Example 23

By allowing 1-[(tert-butoxycarbonyl)amino]cyclobutanecarboxylic acid to react with potassium carbonate and iodomethane in DMF at room temperature, methyl 1-[(tert-butoxycarbonyl)amino]cyclobutanecarboxylate was obtained.

### Production Example 24

By allowing methyl 1-[(tert-butoxycarbonyl)amino]cyclobutanecarboxylate and hydrazine monohydrate to undergo the reaction in methanol under heating, 1-[(tert-butoxycarbonyl)amino]cyclobutanecarbohydrazide was obtained.

### Production Example 25

After heating a mixture of N-cyclopropylcyclopropanecarboxamide and methyl trifluoromethanesulfonate at 60°C, toluene, triethylamine and 1-[(tert-butoxycarbonyl)amino]cyclobutanecarbohydrazide were added thereto, followed by reaction by heating at 60°C and further at 100°C to obtain tert-butyl [1-(4,5-dicyclopropyl-4H-1,2,4-triazol-3-yl)cyclobutyl]carbamate.

### Production Example 26

By heating tert-butyl [1-(4,5-dicyclopropyl-4H-1,2,4-triazol-3-yl)cyclobutyl]carbamate in a 4 M hydrogen chloride-ethyl acetate solution and ethanol, 1-(4,5-dicyclopropyl-4H-1,2,4-triazol-3-yl)cyclobutanamine was obtained.

### Production Example 27

By allowing (6-chloropyridin-2-yl)acetonitrile to undergo the reaction in a saturated solution of hydrogen chloride in methanol in a stream of nitrogen under a heating reflex condition, methyl (6-chloropyridin-2-yl)acetate was obtained.

### Production Example 28

By allowing methyl (6-chloropyridin-2-yl)acetate to react with sodium hydride and iodomethane in DMF at room temperature, methyl (6-chloropyridin-2-yl)-2-methylpropanoate was obtained.

### Production Example 29

By allowing methyl 2-methyl-2-pyridin-2-ylpropanoate and hydrazine monohydrate to undergo the reaction in ethanol under heating, 2-methyl-2-pyridin-2-ylpropanohydrazide was obtained.

### Production Example 30

By allowing 2-methyl-2-pyridin-2-ylpropanohydrazide and cyclopropanoyl chloride to undergo the reaction in dichloromethane at room temperature in the presence of triethylamine, N-(2-methyl-2-pyridin-2-ylpropanoyl)cyclopropanecarbohydrazide was obtained.

### Production Example 31]

By allowing 2-methyl-2-(2-thienyl)propanohydrazide to react with cyclopropanoyl chloride and triethylamine in dichloromethane at room temperature, N'-[2-methyl-2-(2-thienyl)propanoyl]cyclopropanecarbohydrazide was obtained.

### Production Example 32

By allowing N'-[2-methyl-2-(2-thienyl)propanoyl]cyclopropanecarbohydrazide to react with pyridine and trifluoromethanesulfonic anhydride in dichloromethane at room temperature, 2-cycloplopyl-5-[1-methyl-1-(2-thienyl)ethyl]-1,3,4-oxadiazole was obtained.

### Production Example 33

By allowing 5-fluorothiophene-2-carboxylic acid to react with lithium aluminum hydride in THF under heating reflux, (5-fluoro-2-thienyl)methanol was obtained.

### Production Example 34

By allowing 3-thienylacetonitrile to react with sodium hydride and 1,3-dibromo-2-(methoxymethoxy)propane in DMF, 3-(methoxymethoxy)-1-(3-thienyl)cyclobutanecarbonitrile was obtained.
By allowing the resulting 3-(methoxymethoxy)-1-(2-thienyl)cyclobutanecarbonitrile to react with potassium hydroxide in ethylene glycol at 190°C, 3-(methoxymethoxy)-1-(3-thienyl)cyclobutanecarboxylic acid was obtained.

### Production Example 35

By allowing 3,4-dicyclopropyl-5-[cis-3-(methoxymethoxy)-1-(3-thienyl)cyclobutyl]-4H-1,2,4-triazole to react with a 6 M hydrochloric acid aqueous solution in THF at room temperature, cis-3-(4,5-dicyclopropyl-4H-1,2,4-triazol-3-yl)-3-(3-thienyl)cyclobutanol was obtained.

### Production Example 36

By allowing a mixture of a lithium amide obtained from a THF solution of dicyclohexylamine and a 1.6 M n-butyl lithium/hexane solution, a toluene solution of ethyl cyclobutanecarboxylate, bis(dibenzylideneacetone)palladium, 2-bromopyridine and a 10% tert-butylphosphine/hexane solution to undergo reaction at room temperature, ethyl 1-pyridin-2-yl-cyclobutanecarboxylate was obtained.

### Production Example 37

By allowing ethyl 2-methyl-(2-thienyl)propanoate to react with N-iodosuccinimide in an acetic acid-chloroform mixed solution at room temperature, ethyl 2-(5-iodo-2-thienyl)-2-methylpropanoate was obtained.

### Production Example 38

Ethyl 2-(5-iodo-2-thienyl)-2-methylpropanoate was stirred with methyl difluoro(fluorosulfonyl)acetate and copper iodide in DMF while heating at 95°C, thereby obtaining ethyl 2-methyl-2-[5-(trifluoromethyl)-2-thienyl]propanoate.

### Production Example 39

A 1 M sodium hydroxide aqueous solution was added to an ethanol solution of ethyl 2-methyl-2-[5-(trifluoromethyl)-2-thienyl]propanoate, followed by reaction at room temperature to obtain 2-methyl-2-[5-(trifluoromethyl)-2-thienyl]propanecarboxylic acid.

### Production Example 40

An n-hexane solution of n-butyl lithium was added to a THF solution of 2-methyl-2-[5-(trifluoromethyl)-2-thienyl]propanecarboxylic acid, followed by reaction with N-fluorobenzenesulfonimide to obtain 2-[3-fluoro-5-(trifluoromethyl)-2-thienyl]-2-methylpropanecarboxylic acid.

### Production Example 41

By allowing 2-[3-fluoro-5-(trifluoromethyl)-2-thienyl]-2-methyl-propanecarboxylic acid to react with potassium carbonate and methyl iodide in DMF at room temperature, methyl 2-[3-fluoro-5-(trifluoromethyl)-2-thienyl]-2-methylpropanoate was obtained.

### Production Example 42

By allowing cyclopropylacetic acid to react with cyclopropylamine, HOBt monohydrate and WSC monohydrochloride in dichloromethane at room temperature, N,2-dicyclopropylacetamide was obtained.

### Production Example 43

By allowing 3-cyclobutyl-4-cyclopropyl-5-[trans-(methoxymethoxy)-1-(2-thienyl)cyclobutyl]-4H-1,2,4-triazole to react with N-iodosuccinimide'in acetic acid at room temperature, 3-cyclobutyl-4-cyclopropyl-5-[trans-1-(5-iodo-2-thienyl)-3-(methoxymethoxy)cyclobutyl]-4H-1,2,4-triazole was obtained.
By allowing the resulting 3-cyclobutyl-4-cyclopropyl-5-[trans-1-(5-iodo-2-thienyl)-3-(methoxymethoxy)cyclobutyl]-4H-1,2,4-triazole to react with copper cyanide in pyridine under heating with refluxing, 5-[trans-1-(5-cyclobutyl-4-cyclopropyl-4H-1,2,4-triazol-3-yl)-3-(methoxymethoxy)cyclobutyl]-thiophene-2-carbonitrile was obtained.

In the same manner as in the above-mentioned Production Examples 1 to 43, Reference Examples 44 to 163 which are shown later in Tables 2 to 21 were produced using respectively corresponding starting materials. Structures and physicochemical data of the production example compounds are shown in Tables 2 to 21.

### Example 1

Methyl trifluoromethanesulfonate (1.61 ml) was added to N-cyclopropylcyclopropanecarboxamide (1.67 g), followed by heating at 60°C for 30 minutes. Toluene (25 ml), triethylamine (3.97 ml) and 2-methyl-2-(2-thienyl)propanohydrazide (1.64 g) were added to the resulting mixture, followed by stirring at 60°C for 10 hours and at 100°C for 12 hours. The reaction solution was diluted with chloroform (100 ml) and washed with a saturated sodium bicarbonate aqueous solution (100 ml) and saturated brine (50 ml) in that order. The organic layer was dried and then concentrated under a reduced pressure. The resulting residue was purified by silica gel column chromatography (chloroform:methanol). By washing the resulting solid with diethyl ether, 813 mg of 3,4-dicyclopropyl-5-[1-methyl-1-(2-thienyl)ethyl]-4H-1,2,4-triazole (colorless solid) was obtained.

### Example 2

3,4-Dicyclopropyl-5-[1-methyl-1-(2-thienyl)ethyl]-4H-1,2,4-triazole (1.0 g) was dissolved in acetic acid (25 ml), and N-chlorosuccinimide (513 mg) was added, followed by stirring at 80°C for 2 hours. The reaction liquid was evaporated under a reduced pressure, diluted with chloroform, and washed with a saturated sodium bicarbonate aqueous solution and saturated brine in that order. The organic layer was dried and then concentrated under a reduced pressure. The residue was purified by silica gel column chromatography and then the resulting solid was washed with diisopropyl ether to obtain 968 mg of 3-[1-(5-chloro-2-thienyl)-1-methylethyl]-4,5-dicyclopropyl-4H-1,2,4-triazole (white solid).

### Example 3

3,4-Dicyclopropyl-5-[1-methyl-1-(2-thienyl)ethyl]-4H-1,2,4-triazole (1.0 g) was dissolved in acetic acid (25 ml), and N-bromosuccinimide (684 mg) was added, followed by stirring at 80°C for 2 hours. The reaction liquid was evaporated under a reduced pressure, diluted with chloroform, and washed with a saturated sodium bicarbonate aqueous solution and saturated brine. The organic layer was dried and then concentrated under a reduced pressure. The residue was purified by silica gel column chromatography and then the resulting solid was washed with diisopropyl ether to obtain 1.13 g of 3-[1-(5-bromo-2-thienyl)-1-methylethyl]-4,5-dicyclopropyl-4H-1,2,4-triazole (white solid).

### Example 4

3,4-Dicyclopropyl-5-[1-methyl-1-(2-thienyl)ethyl]-4H-1,2,4-triazole (331 mg) was dissolved in acetic acid (5 ml), N-iodosuccinimide (286 mg) was added, followed by overnight stirring at room temperature. The reaction liquid was evaporated under a reduced pressure, diluted with chloroform, and washed with a saturated sodium bicarbonate aqueous solution and saturated brine in that order. The organic layer was dried and then concentrated under a reduced pressure. The residue was purified by silica gel column chromatography to obtain 437 mg of 3-[1-(5-iodo-2-thienyl)-1-methylethyl]-4,5-dicyclopropyl-4H-1,2,4-triazole (white solid). 3-[1-(5-Iodo-2-thienyl)-1-methylethyl]-4,5-dicyclopiopyl-4H-1,2,4-triazole (437 mg) was dissolved in pyridine (10 ml), and copper cyanide (196 mg) was added, followed by stirring at 115°C. The reaction liquid was concentrated under a reduced pressure. The residue was purified by silica gel column chromatography, and the resulting solid was washed with diethyl ether and recrystallized from toluene to obtain 170 mg of 5-[1-(4,5-dicyclopropyl-4H-1,2,4-triazol-3-yl)-1-methylethyl]thiophene-2-carbonitrile (pale yellow crystals).

### Example 5

2-[1-(5-Cyclopropyl-1,3,4-oxadiazol-2-yl)-1-methylethyl]pyridine (300 mg) was dissolved in acetic acid (3 ml), and cyclopropylamine (0.9 ml) was slowly added thereto at 0°C. After 40 minutes of reaction by a microwave at 175°C and subsequent neutralization with a 1 M sodium hydroxide aqueous solution and extraction with chloroform, the organic layer was washed with saturated brine and dried over anhydrous magnesium sulfate, and the solvent was evaporated under a reduced pressure. By purifying the residue by silica gel column chromatography (methanol/ethyl acetate = 10%, and then methanol/chloroform = 10%), 2-[1-(4,5-dicyclopropyl-4H-1,2,4-triazol-3-yl)-1-methylethyl]pyridine was obtained as a pale yellow oily substance. 4 M hydrogen chloride-dioxane (0.24 ml) was added at 0°C to an ethyl acetate (2.7 ml) solution of 2-[1-(4,5-dicyclopropyl-4H-1,2,4-triazol-3-yl)-1-methylethyl]pyridine, followed by stirring at room temperature for 1 hour. The crystals precipitated were collected by filtration, washed with ethyl acetate (2 ml) and then dried under a reduced pressure to obtain 224 mg of 2-[1-(4,5-dicyclopropyl-4H-1,2,4-triazol-3-yl)-1-methylethyl]pyridine monohydrochloride as white crystals.

### Example 6

Sodium methoxide (240 mg) was added to a methanol (2.7 ml) solution of 2-chloro-6-[1-(4,5-dicyclopropyl-4H-1,2,4-triazol-3-yl)-1-methylethyl]pyridine (135 mg), followed by reaction at 120°C for 7 hours using a microwave reaction device (manufactured by Biotage). After extraction with chloroform by adding a saturated sodium bicarbonate aqueous solution, the organic layer was washed with saturated brine and dried, and then the solvent was evaporated under a reduced pressure. 4 M Hydrogen chloride-dioxane (0.074 ml) was added at 0°C to an ethyl acetate (0.88 ml) solution of the residue (88 mg), followed by stirring at room temperature for 1 hour. The crystals precipitated were collected by filtration and washed with ethyl acetate to obtain 99 mg of 2-[1-(4,5-dicyclopropyl-4H-1,2,4-triazol-3-yl)-1-methylethyl]-6-methoxypyridine monohydrochloride as white crystals.

### Example 7

After stirring a mixture of 2-(4,5-dicyclopropyl-4H-1,2,4-triazol-3-yl)propane-2-amine (1.02 g), 2,5-dimethoxytetrahydrofuran (0.78 g), chloroform (5 ml) and acetic acid (5 ml) at 100°C for 2 hours using a microwave reaction device (manufactured by Biotage), chloroform, a 1 M sodium hydroxide aqueous solution and water were added, followed by layer separation operation. The organic layer was washed with a saturated sodium chloride aqueous solution, dried over anhydrous magnesium sulfate and then evaporated under a reduced pressure. By purifying the residue by silica gel column chromatography and washing the resulting solid with hexane, 1.08 g of 3,4-dicyclopropyl-5-[1-methyl-1-(1H-pyrrol-1-yl)ethyl]-4H-1,2,4-triazole was obtained.

### Example 8

N-bromosuccinimide (139 mg) was added at 0°C to a mixture of 3,4-dicyclopropyl-5-[1-methyl-1-(1H-pyrrol-1-yl)ethyl]-4H-1,2,4-triazole (200 mg) and THF (10 ml), followed by stirring at 0°C for 3 hours. Then, sodium sulfite (200 mg) was added to the reaction liquid, followed by evaporation under a reduced pressure. The residue was purified by silica gel column chromatography and recrystallized from hexane to obtain 21 mg of 3-[1-(3-bromo-1H-pyrrol-1-yl)-1-methylethyl]-4,5-dicyclopropyl-4H-1,2,4-triazole as a colorless solid.

### Example 9

N-chlorosuccinimide (473 mg) was added at room temperature to a mixture of 3,4-dicyclopropyl-5-[1-methyl-1-(1H-pyrrol-1-yl)ethyl]-4H-1,2,4-triazole (864 mg) and THF (40 ml), followed by stirring at 60°C for 1 hour and then concentration under a reduced pressure. The residue was purified by silica gel column chromatography, and the resulting solid was washed with hexane to obtain 398 mg of 3-[1-(2-chloro-1H-pyrrol-1-yl)-1-methylethyl]-4,5-dicyclopropyl-4H-1,2,4-triazole as a colorless solid.

### Example 10

Sodium triacetoxyborohydride (1.54 g) was added to a mixed solution of 2-(4,5-dicyclopropyl-4H-1,2,4-triazol-3-yl)propane-2-amine (300 mg), 37% formalin (0.59 ml) and acetonitrile (20 ml), followed by stirred at room temperature for 6 hours. Chloroform, a 1 M sodium hydroxide aqueous solution and water were added to the reaction solution and the layers were separated. The organic layer was washed with a saturated sodium chloride aqueous solution, dried over anhydrous magnesium sulfate and evaporated under a reduced pressure. The residue was purified by silica gel column chromatography. 4 M Hydrogen chloride-ethyl acetate was added to an ether solution of this product, followed by stirring for 30 minutes. The solid formed was collected by filtration to obtain 316 mg of 2-(4,5-dicyclopropyl-4H-1,2,4-triazol-3-yl)-N,N-dimethylpropane-2-amine hydrochloride as a colorless solid.

### Example 11

A mixture of 2-(4,5-dicyclopropyl-4H-1,2,4-triazol-3-yl)propane-2-amine (600 mg), chloroform (20 ml), acetone (2.14 ml) and sodium triacetoxyborohydride (0.92 g) was stirred at 50°C for 16 hours. Chloroform, a saturated sodium bicarbonate aqueous solution and water were added, and the layers were separated. The organic layer was washed with a saturated sodium chloride aqueous solution, dried over anhydrous magnesium sulfate, and evaporated under a reduced pressure. The residue was purified by silica gel column chromatography (chloroform:methanol = 50:1) to obtain a product (360 mg). 4 M Hydrogen chloride-ethyl acetate was added to an ether (24 ml) solution of this product, followed by stirring for 30 minutes. The solid formed was collected by filtration to obtain 331 mg of 2-(4,5-dicyclopropyl-4H-1,2,4-triazol-3-yl)-N-isopropylpropane-2-amine hydrochloride as a colorless solid.

### Example 12

After converting 2-(4,5-dicyclopropyl-4H-1,2,4-triazol-3-yl)-N-isopropylpropane-2-amine hydrochloride into free amine by a layer separation operation, 37% formalin (0.26 ml), acetonitrile (15 ml) and sodium triacetoxyborohydride (0.67 g) were added, followed by stirring at room temperature for 7 hours. Chloroform, a saturated sodium hydroxide aqueous solution and water were added to the reaction solution, and layers were separated. The organic layer was washed with a saturated sodium chloride aqueous solution, dried over anhydrous magnesium sulfate, and then evaporated under a reduced pressure. The residue was purified by silica gel column chromatography (chloroform:methanol = 50:1). 4 M Hydrogen chloride-ethyl acetate (0.24 ml) was added to an ether (17 ml) solution of this product, followed by stirring for 30 minutes. The solid formed was collected by filtration to obtain 248 mg of 2-(4,5-dicyclopropyl-4H-1,2,4-triazol-3-yl)-N-isopropyl-N-methylpropane-2-amine hydrochloride as a colorless solid.

### Example 13

Pyridine (0.59 ml) and 2-thiophenesulfonylchloride (398 mg) were added to a dichloromethane (6 ml) solution of 2-(4,5-dicyclopropyl-4H-1,2,4-triazol-3-yl)propane-2-amine (300 mg), followed by stirring at room temperature for 3 days. Then, N,N-dimethylpropanediamine (0.14 ml) was added thereto at 0°C. After stirring at room temperature for 30 minutes, this was diluted with ethyl acetate and washed with a 0.1 M hydrochloric acid aqueous solution, a saturated sodium bicarbonate aqueous solution and saturated brine in that order. The organic layer was dried and concentrated, and the residue was purified by silica gel column chromatography to obtain 275 mg of N-[1-(4,5-dicyclopropyl-4H-1,2,4-triazol-3-yl)-1-methylethyl]thiophene-2-sulfonamide as a solid.

### Example 14

Sodium hydride (31 mg) washed with hexane was suspended in DMF (5.5 ml), and a DMF solution (22 ml) of N-[1-(4,5-dicyclopropyl-4H-1,2,4-triazol-3-yl)-1-methylethyl]thiophene-2-sulfonamide (275 mg) and iodomethane (0.049 ml) were added thereto. After 2 days of stirring at room temperature, ethyl acetate and a 0.5 M hydrochloric acid aqueous solution were added thereto. The organic layer was washed with a saturated sodium bicarbonate aqueous solution and saturated brine in that order, dried and then concentrated under a reduced pressure. By purifying the residue by silica gel column chromatography, 104 mg of N-[1-(4,5-dicyclopropyl-4H-1,2,4-triazol-3-yl)-1-methylethyl]-N-methylthiophene-2-sulfonamide was obtained as a white solid.

### Example 15

3-[Cis-1-(5-chloro-2-thienyl)-3-(methoxymethoxy)cyclobutyl]-5-cyclobutyl-4-cyclopropyl-4H-1,2,4-triazole (348 mg) was dissolved in THF (4 ml), and a 6 M hydrochloric acid aqueous solution (2 ml) was added thereto, followed by stirring at room temperature for 15 hours. The reaction liquid was diluted with a 1 M sodium hydroxide aqueous solution (12 ml), followed by extraction with chloroform (10 ml x 3). The organic layer was dried over anhydrous sodium sulfate and then concentrated under a reduced pressure. The resulting solid was washed with hexane-ethyl acetate (1:1) to obtain 242 mg of cis-3-(5-chloro-2-thienyl)-3-(5-cyclobutyl-4-cyclopropyl-4H-1,2,4-triazol-3-yl)cyclobutanol as a white solid.

### Example 16, 17

Trans-3-(5-chloro-2-thienyl)-3-(4,5-dicyclopropyl-4H-1,2,4-triazol-3-yl)cyclobutanol (700 mg) was dissolved in dichloromethane (20 ml), and under ice-cooling, pyridine (0.51 ml) and trifluoromethanesulfonic anhydride(420 µl) were added thereto, followed by stirring under ice-cooling for 1 hour. The reaction liquid was diluted with dichloromethane (30 ml) and washed with a saturated copper sulfate aqueous solution (30 ml x 2) and saturated brine (30 ml) in that order. The organic layer was dried over anhydrous sodium sulfate and then concentrated under a reduced pressure. The residue was purified by silica gel column chromatography. The resulting residue was dissolved in dichloromethane (20 ml), and tris(dimethylamino)sulfur (trimethylsilyl)difluoride (861 mg) was added thereto, followed by stirring room temperature for 15 hours. The reaction liquid was diluted with a saturated sodium bicarbonate aqueous solution, followed by extraction with chloroform (20 ml x 2). The organic layer was dried over anhydrous sodium sulfate and then concentrated under a reduced pressure, and the residue was purified by silica gel column chromatography. By respectively washing the resulting solids with diethyl ether, 423 mg of 3-[cis-1-(5-chloro-2-thienyl)-3-fluorocyclobutyl]-4,5-dicyclopropyl-4H-1,2,4-triazole (Example 16) and 80 mg of 3-[1-(5-chloro-2-thienyl)cyclobut-2-ene-1-yl]-4,5-dicyclopropyl-4H-1,2,4-triazole (Example 17) were obtained as white solids.

### Example 18

Trans-3-(5-tert-butyl-4-cyclopropyl-4H-1,2,4-triazol-3-yl)-3-(5-chloro-2-thienyl)cyclobutyl benzoate (785 mg) was dissolved in methanol (20 ml), and a 1.0 M sodium methoxide-methanol solution (0.86 ml) was added thereto, followed by stirring at 40°C for 15 hours. AMBERLYST (registered trademark) A-26 was added to the reaction liquid and the resin was separated by filtration, followed by washing with methanol. By concentrating the filtrate under a reduced pressure and purifying the residue by silica gel column chromatography (chloroform-methanol = 50:1), 440 mg of trans-3-(5-tert-butyl-4-cyclopropyl-4H-1,2,4-triazol-3-yl)-3-(5-chloro-2-thienyl)cyclobutanol was obtained as a white solid.

### Example 19

A mixture of 3-[1-(4-bromo-2-thienyl)-1-methylethyl]-4,5-dicyclopropyl-4H-1,2,4-triazole (300 mg), zinc cyanide (100 mg), zinc powder (33 mg), 1,1'-bis(diphenylphosphino)ferrocene (94 mg), tris(dibenzylideneacetone)dipalladium(0)(82 mg) and N,N-dimethylacetamide (3 ml) was stirred at 80°C for 80 hours and then cooled to room temperature. Chloroform and an aqueous ammonia solution were added thereto to carry out layer separation operation. The organic layer was dried and then concentrated. The residue was purified by silica gel column chromatography (ethyl acetate-chloroform = 30:70 to 70:30), and the resulting solid was washed with ether, thereby obtaining 40 mg of 5-[1-(4,5-dicyclopropyl-4H-1,2,4-triazol-3-yl)-1-methylethyl]thiophene-3-carbonitrile as a white solid.

### Example 20

N-Chlorosuccinimide (230 mg) was added at room temperature to a mixture of 3,4-dicyclopropyl-5-[1-methyl-1-(1H-pyrrol-1-yl)ethyl]-4H-1,2,4-triazole (210 mg ) and THF (20 ml), followed by stirring at 60°C for 3 hours and concentration under a reduced pressure. The residue was purified by silica gel column chromatography (chloroform-methanol = 200:1), and 4 M hydrogen chloride-dioxane was added to an ether solution of the resulting residue, followed by stirring for 30 minutes. The solid precipitated was collected by filtration to obtain 47 mg of 3,4-dicyclopropyl-5-[1-(2,5-dichloro-1H-pyrrol-1-yl)-1-methylethyl]-4H-1,2,4-triazole hydrochloride as a colorless solid.

### Example 21

N-Chlorosuccinimide (820 mg) was added at room temperature to a mixture of 3,4-dicyclopropyl-5-[1-methyl-1-(1H-pyrrol-1-yl)ethyl]-4H-1,2,4-triazole (500 mg) and THF (20 ml), followed by stirring at 60°C for 3 hours and concentration under a reduced pressure. The residue was purified by silica gel column chromatography (chloroform-methanol = 200:1), and a 4 M hydrogen chloride-dioxane was added to an ether solution of the resulting residue, followed by stirring for 20 minutes. The solid precipitated was collected by filtration to obtain 18 mg of 3,4-dicyclopropyl-5-[1-(2,5-dichloro-1H-pyrrol-1-yl)-1-methylethyl]-4H-1,2,4-triazole hydrochloride as a colorless solid.

### Example 22

In an atmosphere of nitrogen, triethylamine (0.59 ml), potassium trifluoro(vinyl) borate (456 mg) and [1,1'-bis(diphenylphosphino)ferrocene]dichloropalladium(II) dichloromethane complex (116 mg) were added to an n-propanol solution (20 ml) of 3-[1-(5-bromo-2-thienyl)-1-methylethyl]-4,5-dicyclopropyl-4H-1,2,4-triazole (1.0 g), followed by stirring at 110°C for 15 hours. After confirming completion of the reaction, the solvent was evaporated under a reduced pressure, and water was added, followed by extraction with chloroform. The organic layer was washed with a saturated sodium chloride aqueous solution and then dried over anhydrous magnesium sulfate. The solvent was evaporated under a reduced pressure. The residue was purified by silica gel column chromatography (chloroform-methanol = 100:0 to 95:5) and washed with diisopropyl ether to obtain 3,4-dicyclopropyl-5-[1-methyl-1-(5-vinyl-2-thienyl)ethyl]-4H-1,2,4-triazole (591 mg) as a pale yellow solid.

### Example 23

In an atmosphere of nitrogen, N,N,N',N'-tetramethylethylenediamine (2.4 ml) was added to a THF solution (30 ml) of 3,4-dicyclopropyl-5-[1-methyl-1-(2-thienyl)ethyl]-4H-1,2,4-triazole (2.82 g), and n-butyl lithium-n-hexane solution (1.6 M, 7.5 ml) was added dropwise thereto at -78°C, followed by stirring as such for 1 hour. A THF solution of DMF (1.86 ml) was added dropwise to the reaction solution, followed by stirring for 1 hour as it is. The reaction solution was poured into water, followed by extraction with chloroform. The organic layer was washed with a saturated sodium chloride aqueous solution and dried over anhydrous magnesium sulfate. The solvent was evaporated under a reduced pressure. The residue was purified by silica gel column chromatography (chloroform-methanol = 100:0 to 95:5) and washed with diisopropyl ether to obtain 5-[1-(4,5-dicyclopropyl-4H-1,2,4-triazol-3-yl)-1-methylethyl]thiophene-2-carbaldehyde (1.61 g) as a colorless solid.

### Example 24

3-[1-(5-Bromo-2-thienyl)-1-methylethyl]-4-cyclopropyl-5-(3-methylenecyclobutyl)-4H-1,2,4-triazole (101 mg) was dissolved in dichloromethane (20 ml), and ozone was blown therein at -78°C for 5 minutes using an ozonizer. When the reaction solution changed to pale blue, oxygen was blown therein for 5 minutes, and nitrogen for 10 minutes, and dimethyl sulfoxide (60 µl) was added thereto, followed by stirring at room temperature for 30 minutes. The reaction solution was washed with saturated brine. The organic layer was dried over anhydrous sodium sulfate and then concentrated under a reduced pressure. The residue was purified by silica gel column chromatography (methanol-chloroform = 2:98). The resulting solid was washed with diisopropyl ether to obtain 3-{5-[1-(5-bromo-2-thienyl)-1-methylethyl]-4-cyclopropyl-4H-1,2,4-triazol-3-yl}cyclobutanone (43.4 mg) as a pale yellow solid.

### Example 25

A sulfur trifluoride dimethoxyethylamino complex (175 µl) was dissolved in dichloromethane (10 ml), and under ice-cooling, 3-{5-[1-(5-bromo-2-thienyl)-1-methylethyl]-4-cyclopropyl-4H-1,2,4-triazol-3-yl}cyclobutanone (150.5 mg) was added thereto, followed by stirring at room temperature for 3 days. The reaction solution was diluted with a saturated sodium bicarbonate aqueous solution, followed by extraction with chloroform. The organic layer was dried over anhydrous sodium sulfate and then concentrated under a reduced pressure. The residue was purified by silica gel column chromatography (chloroform-methanol = 99: 1). The resulting solid was dissolved in ethyl acetate (3 ml), and a 4 M hydrogen chloride-ethyl acetate (0.1 ml) was added thereto, followed by stirring at room temperature for 30 minutes. The reaction solution was concentrated under a reduced pressure, and the resulting solid was washed with ethyl acetate to obtain 3-[1-(5-bromo-2-thienyl)-1-methylethyl]-4-cyclopropyl-5-(3,3-difluorocyclobutyl)-4H-1,2,4-triazole (43.5 mg) as a white solid.

### Example 26

10% Palladium-carbon powder (30 mg) was added to an ethanol solution (5 ml) of 3,4-dicyclopropyl-5-[1-methyl-1-(5-vinyl-2-thienyl)ethyl]-4H-1,2,4-triazol (278 mg) in an atmosphere of nitrogen, followed by vigorous stirring for 4 hours in an atmosphere of hydrogen. After completion of the reaction, the reaction liquid was filtered through celite, and the solvent was evaporated under a reduced pressure. By recrystallizing the residue with isopropyl ether, 3,4-dicyclopropyl-5-[1-(5-ethyl-2-thienyl)-1-methylethyl]-4H-1,2,4-triazole (207 mg) was obtained as pale yellow crystals.

### Example 27

Thionyl chloride (0.125 ml) was added at 0°C to a dichloroethane solution (10 ml) of DMF (0.13 ml), followed by stirring at room temperature for 15 minutes. Then, a dichloroethane solution (5 ml) of 3,4-dicyclopropyl-5-[1-methyl-1-(1H-pyrrol-1-yl)ethyl]-4H-1,2,4-triazol (400 mg) was added thereto at 0°C, followed by stirring at 70°C for 3 hours. Chloroform, a 1 M sodium hydroxide aqueous solution and water were added to the reaction liquid to carry out layer separation operation. The organic layer was washed with a saturated sodium chloride aqueous solution, dried over anhydrous magnesium sulfate, and then concentrated under a reduced pressure. The residue was purified by silica gel column chromatography (chloroform-methanol = 100:1) and made into powder in diethyl ether, thereby obtaining 1-[1-(4,5-dicyclopropyl-4H-1,2,4-triazol-3-yl)-1-methylethyl]-1H-pyrrole-2-carbaldehyde (225 mg) as a colorless solid.

### Example 28

Triethylsilane (0.37 ml) was added to a trifluoroacetic acid (4 ml) solution of 1-[1-(4,5-dicyclopropyl-4H-1,2,4-triazol-3-yl)-1-methylethyl]-1H-pyrrole-2-carbaldehyde (220 mg), followed by stirring at room temperature for 16 hours. The reaction liquid was poured into a mixture of chloroform, a 1 M sodium hydroxide aqueous solution and water to carry out a layer separation operation. The organic layer was washed with a saturated sodium chloride aqueous solution, dried over anhydrous magnesium sulfate, and then concentrated under a reduced pressure. The residue was purified by silica gel column chromatography (chloroform-methanol = 100:1) and made into powder in diethyl ether, thereby obtaining {1-[1-(4,5-dicyclopropyl-4H-1,2,4-triazol-3-yl)-1-methylethyl]-1H-pyrrol-2-yl}methanol (38 mg) as a colorless solid.

### Example 29

A mixture of 1-[1-(4,5-dicyclopropyl-4H-1,2,4-triazol-3-yl)-1-methylethyl]-1H-pyrrole-2-carbaldehyde (200 mg), diethylene glycol (10 ml) and hydrazine monohydrate (0.1 ml) was stirred at 130°C for 1 hour and 30 minutes, and then potassium hydroxide (118 mg) was added thereto, followed by stirring at 170°C for 2 hours. The reaction solution was added to chloroform and water to carry out layer separation operation. The organic layer was washed with a saturated sodium chloride aqueous solution, dried over anhydrous magnesium sulfate, and then concentrated under a reduced pressure. The residue was purified by silica gel column chromatography (chloroform-methanol = 100:1) and made into powder and washed with n-hexane, thereby obtaining 3,4-dicyclopropyl-5-[1-methyl-1-(2-methyl-1H-pyrrol-1-yl)ethyl]-4H-1,2,4-triazole (80 mg) as a colorless solid.

### Example 30

A mixture of 1-[1-(4,5-dicyclopropyl-4H-1,2,4-triazol-3-yl)-1-methylethyl]-1H-pyrrole-2-carbaldehyde (900 mg), hydroxylamine hydrochloride (242 mg), potassium carbonate (481 mg) and DMF (20 ml) was stirred at 120°C for 20 hours, and then acetic anhydride (1.31 ml) was added thereto, followed by stirring at 120°C for 5 hours. The reaction liquid was poured into a mixture of chloroform, a 1 M sodium hydroxide aqueous solution and water to carry out layer separation operation. The organic layer was washed with a saturated sodium chloride aqueous solution, dried over anhydrous magnesium sulfate, and then concentrated under a reduced pressure. The residue was purified by silica gel column chromatography (chloroform-methanol = 100:1), and made into powder and washed with n-hexane, thereby obtaining 1-[1-(4,5-dicyclopropyl-4H-1,2,4-triazol-3-yl)-1-methylethyl]-1H-pyrrole-2-carbonitrile (476 mg) as a pale yellow solid.

### Example 31 and 32

2-Methoxy-N-(2-methoxyethyl)-N-(trifluoro-λ⁴-sulfanyl)ethanamine was added under ice-cooling to a dichloromethane solution of 5-[1-(4,5-dicyclopropyl-4H-1,2,4-triazol-3-yl)-1-methylethyl]thiophene-2-carbaldehyde (250 mg), followed by stirring at room temperature for 25 hours. Dichloroethane (5 ml) was added thereto, followed by stirring at 80°C for 15 hours. A saturated sodium bicarbonate aqueous solution was added to the reaction solution, followed by extraction with chloroform. The organic layer was washed with a saturated sodium chloride aqueous solution, dried over anhydrous magnesium sulfate, and then the solvent was evaporated under a reduced pressure. The residue was purified by silica gel column chromatography (chloroform-methanol = 100:0 to 95:5) and washed with diisopropyl ether. The resulting solid (189 mg) was dissolved in methanol (5 ml), and sodium borohydride (20 mg) was added thereto under ice-cooling, followed by stirring at 0°C for 45 minutes. After confirmation of the completion of the reaction, a 1 M hydrochloric acid aqueous solution (5 ml) was added thereto, and the solvent was evaporated under a reduced pressure. A 1 M sodium hydroxide aqueous solution (5 ml) was added to the reside, followed by extraction with chloroform. The organic layer was washed with a saturated sodium chloride aqueous solution, dried over anhydrous magnesium sulfate, and then the solvent was evaporated under a reduced pressure. The residue was purified by silica gel column chromatography (chloroform-methanol = 100:0 to 95:5) to obtain low polarity and high polarity products. By washing respective products with diisopropyl ether, 3,4-dicyclopropyl-5-{ 1-[5-(difluoromethyl)-2-thienyl]-1-methylethyl}-4H-1,2,4-triazole (87 mg) (Example 31) was obtained from the low polarity product, and {5-[1-(4,5-dicyclopropyl-4H-1,2,4-triazol-3-yl)-1-methylethyl]-2-thienyl}methanol (23 mg) (Example 32) from the high polarity product.

### Example 33

Sodium borohydride (45 mg) was added to a mixed solution of 1-[1-(5-cycloheptyl-4-methyl-4H-1,2,4-triazol-3-yl)-1-methylethyl]-1H-pyrrole-3-carbaldehyde (370 mg) and methanol (5 ml), followed by stirring at room temperature for 1 hour. A 1 M hydrochloric acid aqueous solution (2 ml) was added to the reaction solution, followed by stirring for 10 minutes,. A 1 M sodium hydroxide aqueous solution (2 ml), chloroform and water were added thereto to carry out layer separation operation. The organic layer was washed with a saturated sodium chloride aqueous solution, dried over anhydrous magnesium sulfate, and then the solvent was evaporated under a reduced pressure. The residue was purified by silica gel column chromatography (chloroform-methanol = 50:1) to obtain an oily product. A 4 M hydrogen chloride-ethyl acetate was added to an ethyl acetate solution of this product, followed by stirring for 30 minutes. The solid formed was collected by filtration to obtain 1-[1-(5-cycloheptyl-4-methyl-4H-1,2,4-triazol-3-yl)-1-methylethyl]-1H-pyrrol-3-yl}methanol hydrochloride (301 mg) as a colorless solid.

### Example 34

Iodomethane (16 µl) was added to a mixture of {1-[1-(5-cycloheptyl-4-methyl-4H-1,2,4-triazol-3-yl)-1-methylethyl]-1H-pyrrol-3-yl}methanol hydrochloride (100 mg), sodium hydride (27 mg) and DMF (6.7 ml), followed by stirring at room temperature for 5 hours. The reaction solution was poured into a mixture of chloroform and water to carry out layer separation operation. The organic layer was washed with a saturated sodium chloride aqueous solution, dried over anhydrous magnesium sulfate, and then concentrated under a reduced pressure. The residue was purified by silica gel column chromatography (chloroform-methanol = 50:1) to obtain an oily product (60 mg). A 4 M hydrogen chloride-ethyl acetate was added to an ethyl acetate solution of this product, followed by stirring for 30 minutes. The solid formed was collected by filtration to obtain 3-cycloheptyl-5-{1-[3-(methoxymethyl)-1H-pyrrol-1-yl]-1-methylethyl}-4-methyl-4H-1,2,4-triazole hydrochloride (37 mg) as a colorless solid.

### Example 35

3-[1-(5-Bromo-2-thienyl)-1-methylethyl]-5-cyclopropyl-4-(2-phenylethyl)-4H-1,2,4-triazole (88 mg) and copper cyanide(I) (95 mg) were suspended in N-methylpyrrolidone (0.5 ml), followed by stirring at 200°C for 60 minutes using a microwave reaction device (manufactured by Biotage). The reaction liquid was cooled to room temperature, and water and a 1 M sodium hydroxide aqueous solution were added, followed by extraction with ethyl acetate. The organic layer was dried over anhydrous magnesium sulfate, and the solvent was evaporated under a reduced pressure. The residue was purified by silica gel column chromatography (chloroform-methanol = 98:2), and the resulting oily substance was again purified by silica gel column chromatography (ethyl acetate) to obtain an oily substance. The resulting oily substance was dissolved in ethyl acetate, a 4 M hydrogen chloride-ethyl acetate (1 ml) was added thereto, and the solvent was evaporated under a reduced pressure. By washing the resulting solid with diisopropyl ether, 5-{1-[cyclopropyl-4-(2-phenylethyl)-4H-1,2,4-triazol-3-yl-1-methylethyl}thiophene-2-carbonitrile hydrochloride (15 mg) was obtained as a white solid.

### Example 36

A mixture of 2-(4,5-dicyclopropyl-4H-1,2,4-triazol-3-yl)propane-2-amine (300 mg), 3-bromophthalic anhydride (363 mg), chloroform (3 ml) and acetic acid (3 ml) was stirred at 100°C for 2 hours using a microwave reaction device (manufactured by Biotage). Chloroform, a 1 M sodium hydroxide aqueous solution and water were added to the reaction solution to carry out layer separation operation. The organic layer was washed with a saturated sodium chloride aqueous solution, dried over anhydrous magnesium sulfate, and then concentrated under a reduced pressure. The residue was purified by silica gel column chromatography (chloroform-methanol = 100:1) to obtain a product. By washing this product with hexane, 5-bromo-2-[1-(4,5-dicyclopropyl-4H-1,2,4-triazol-3-yl)-1-methylethyl]-1H-isoindole-1,3(2H)dione (362 mg) was obtained as a colorless solid.

### Example 37

3,4-Dicyclopropyl-5-{1-methyl-1-[5-(trifluoromethyl)-2-thienyl]ethyl]-4H-1,2,4-triazole (231 mg) was dissolved in THF (5 ml), and 1.42 M tert-butyl lithium-n-pentane solution (0.57 ml) was added thereto at -78°C, followed by stirring at -78°C for 30 minutes. DMF (80 µl) was added to the reaction solution, followed by stirring for 15 hours with spontaneously rising to room temperature. The reaction solution was diluted with water, followed by extraction with ethyl acetate. The residue was purified by a preparative HPLC (column name: Mightysil RP-18 GP 250-20 (5 mm); eluent: 0.08% formic acid-acetonitrile-water = 0.08:50:50), and then diluted with a saturated sodium bicarbonate aqueous solution, followed by extraction with ethyl acetate. The organic layer was dried over anhydrous sodium sulfate and then concentrated under a reduced pressure to obtain 5-[1-(4,5-dicyclopropyl-4H-1,2,4-triazol-3-yl)-1-methylethyl]-2-(trifluoromethyl)thiophene-3-carbaldehyde (40.9 mg) as a colorless syrup.
5-[1-(4,5-Dicyclopropyl-4H-1,2,4-triazol-3-yl)-1-methylethyl]-2-(trifluoromethyl)thiophene-3-carbaldehyde (40.9 mg) was dissolved in ethanol (3 ml), and hydroxylamine hydrochloride (31.5 mg) and triethylamine (62 µl) were added, followed by heating under reflux for 15 hours while stirring. The reaction solution was concentrated under a reduced pressure, diluted with a saturated sodium bicarbonate aqueous solution, followed by extractions three times with chloroform. The organic layer was dried over anhydrous sodium sulfate and then concentrated under a reduced pressure. The resulting residue was dissolved in dichloromethane (3 ml), and triethylamine (62 µl) and trifluoromethanesulfonic anhydride (36 µl) were added at -78°C, spontaneously risen to room temperature, followed by stirring for 5 hours. The reaction solution was diluted with a saturated sodium bicarbonate aqueous solution, followed by extraction with chloroform. The organic layer was dried over anhydrous sodium sulfate and then concentrated under a reduced pressure. The residue was purified by silica gel column chromatography (chloroform-methanol = 98:2). The resulting solid was diluted with ethyl acetate, and 4 M hydrogen chloride-ethyl acetate (0.2 ml) was added, followed by concentration under a reduced pressure. By washing the resulting solid with diisopropyl ether, 5-[1-(4,5-dicyclopropyl-4H-1,2,4-triazol-3-yl)-1-methylethyl]-2-(trifluoromethyl)thiophene-3-carbonitrile hydrochloride (35.4 mg) was obtained as a white solid.

### Example 38

2-(5-Chloro-2-thienyl)-N-cyclopropyl-2-methylpropanamide (1.33 g) was dissolved in chloroform (20 ml), and thionyl chloride (2.00 ml) and DMF (50 µl) were added, followed by stirring at 60°C for 1 hour, and then subjected to toluene azeotropy. The residue was dissolved in toluene (20 ml), 1-hydroxycyclopropanecarbohydrazide (575 mg) was added, followed by overnight stirring at 80°C. Then, DMF (3 ml) was added, followed by stirring at 110°C for 3 hours. The reaction liquid was cooled to room temperature, diluted with ethyl acetate, washed with water, a 1 M sodium hydroxide aqueous solution, a citric acid aqueous solution and saturated brine in that order, and then dried over anhydrous magnesium sulfate. The solvent was concentrated under a reduced pressure, and the resulting residue was washed with diisopropyl ether to obtain 1-{5-[1-(5-chloro-2-thienyl)-1-methylethyl]-4-cyclopropyl-4H-1,2,4-triazol-3-yl}cyclopropanol (532 mg) as a white solid.

### Example 39

1-{5-[1-(5-Chloro-2-thienyl)-1-methylethyl]-4-cyclopropyl-4H-1,2,4-triazol-3-yl}cyclopropanol (495 mg) was dissolved in dichloroethane (10 ml), and 2-methoxy-N-(2-methoxyethyl)-N-(trifluoro-λ⁴-sulfanyl)ethanamine (0.845 ml) was added under ice-cooling, followed by stirring at room temperature for 3 days. AIM sodium hydroxide aqueous solution was added to the reaction solution, followed by extraction with chloroform. The organic layer was dried over anhydrous magnesium sulfate, the solvent was concentrated under a reduced pressure, and the residue was purified by silica gel column chromatography (n-hexane-ethyl acetate = 2:1). By washing the resulting solid with hexane, 3-[1-(5-chloro-2-thienyl)-1-methylethyl]-4-cyclopropyl-5-(1-fluorocyclopropyl)-4H-1,2,4-triazole (42 mg) was obtained as a white solid.

### Example 40

3-[1-(4-Bromo-2-thienyl)-1-methylethyl]-4,5-dicyclopropyl-4H-1,2,4-triazole (1.00 g), sodium trifluoroacetate (3.86 g) and copper(I) iodide (2.70 g) were dissolved in 1-methyl-2-pyrrolidone (40 ml), followed by stirring at 180°C for 2 hours in an atmosphere of nitrogen. The reaction liquid was cooled to room temperature, and water was added, followed by extraction with ethyl acetate. The resulting oily substance was purified by silica gel column chromatography, and the resulting solid was washed with hexane and further recrystallized from hexane-ethyl acetate to obtain 3,4-dicyclopropyl-5-{1-methyl-1-[4-(trifluoromethyl)-2-thienyl]ethyl]-4H-1,2,4-triazole (130 mg) as a white solid.

### Example 41

Thionyl chloride (3.5 ml) and DMF (74 µl) were added at room temperature to a chloroform solution (30 ml) of N-cyclopropyl-2-methyl-2-(2-thienyl)propanamide (2 g), followed by stirring at 60°C for 1 hour. The reaction liquid was evaporated under a reduced pressure, and then toluene (40 ml) and formic hydrazide (630 mg) were added to the residue. After stirring the reaction liquid at 70°C for 20 hours, chloroform and 1 M sodium hydroxide aqueous solution were added thereto to carry out layer separation operation. The organic layer was washed with a saturated sodium chloride aqueous solution, dried over anhydrous magnesium sulfate, and then evaporated under a reduced pressure. The residue was purified by silica gel column chromatography (chloroform-methanol = 100:1) to obtain the product. By washing this product with hexane, 565 mg of 4-cyclopropyl-3-[1-methyl-1-(2-thienyl)ethyl]-4H-1,2,4-triazole was obtained as a colorless solid.

### Example 42

A 1.42 M tert-butyl lithium-n-pentane solution (0.31 ml) was added dropwise at -70°C to a THF solution of 3,4-dicyclopropyl-5-[1-methyl-1-(3-thienyl)ethyl]-4H-1,2,4-triazole (100 mg), followed by stirring at the same temperature for 30 minute. Then, DMF (42 µl) was added, followed by gradual temperature rising to room temperature. The reaction liquid was cooled using an ice bath, the reaction was terminated by adding water, and diethyl ether was added, followed by layer separation operation. The organic layer was washed with saturated brine, dried over anhydrous magnesium sulfate, and then concentrated under a reduced pressure. By purifying the residue by a thin layer chromatography, 5-[1-(4,5-dicyclopropyl-4H-1,2,4-triazol-3-yl)-1-methylethyl]-thiophene-2-carbaldehyde (33 mg) was obtained.
2-Methoxy-N-(2-methoxyethyl)-N-(trifluoro-λ⁴-sulfanyl)ethanamine (0.1 ml) was added to a dichloroethane solution of 5-[1-(4,5-dicyclopropyl-4H-1,2,4-triazol-3-yl)-1-methylethyl]-thiophene-2-carbaldehyde (33 mg), followed by stirring at 60°C for 18 hours. The reaction solution was added to an ice-cooled saturated sodium bicarbonate aqueous solution, followed by extraction with chloroform. The organic layer was washed with a saturated sodium bicarbonate aqueous solution and a saturated sodium chloride aqueous solution and dried over anhydrous magnesium sulfate. The solvent was evaporated under a reduced pressure. The residue was purified by a thin layer chromatography (chloroform-methanol = 95:5). The resulting solid (11 mg) was dissolved in ethyl acetate (0.2 ml), 4 M hydrogen chloride-ethyl acetate (10 µl) and diisopropyl ether were added thereto, and the crystals precipitated were collected by filtration and washed with diisopropyl ether to obtain 4.9 mg of 3,4-dicyclopropyl-5-{1-[5-(difluoromethyl)-3-thienyl]-1-methylethyl}-4H-1,2,4-triazole hydrochloride as a colorless solid.

### Example 43

3-Bromo-5-[1-(5-bromo-2-thienyl)-1-methylethyl]-4-cyclopropyl-4H-1,2,4-triazole (60 mg) and sodium ethoxide (52 mg) were dissolved in ethanol (2 ml), followed by stirring at 150°C for 30 minutes (inner pressure 7 bar) using a microwave reaction device (manufactured by Biotage). The reaction solution was diluted with ethanol (10 ml), and neutralized with AMBERLYST (registered trademark) A-26, followed by separation by filtration. The filtrate was concentrated under a reduced pressure, and the residue was diluted with chloroform and washed with saturated brine. The organic layer was dried over anhydrous sodium sulfate and then concentrated under a reduced pressure, and the residue was purified by a preparative thin layer chromatography (chloroform-methanol = 19:1) to obtain 4H-1,2,4-triazole, 3-[1-(5-bromo-2-thienyl)-1-methylethyl]-4-cyclopropyl-5-ethoxy- (30.6 mg) as a white solid.

### Example 44

A mixture of 3,4-dicyclopropyl-5-[1-methyl-1-(1H-pyrrol-1-yl)ethyl]-4H-1,2,4-triazole (600 mg), DMF (10 ml), potassium carbonate (1.21 g) and 1,2-bis(bromomethyl)benzene (768 mg) was stirred at 60°C for 2 hours and at 100°C for 16 hours. Chloroform and water were added to the reaction liquid, followed by a layer separation operation. The organic layer was washed with a saturated sodium chloride aqueous solution. The organic layer was dried over anhydrous magnesium sulfate and then concentrated under a reduced pressure. The residue was purified by silica gel column chromatography (chloroform-methanol = 100:1) to obtain the product. By washing the resulting product with hexane, 2-[1-(4,5-dicyclopropyl-4H-1,2,4-triazol-3-yl)-1-methylethyl]isoindolin-1-one (205 mg) was obtained.

### Example 45

Ethyl 3-{3-[1-(5-bromo-2-thienyl)-1-methylethyl]-5-cyclopropyl-4H-1,2,4-triazol-4-yl}propanoate hydrochloride (150 mg) was suspended in ethanol (3 ml), a 1 M sodium hydroxide (0.84 ml) was added, followed by stirring at room temperature for 1 hour. The reaction liquid was concentrated under a reduced pressure and neutralized with a 1 M hydrochloric acid aqueous solution, followed by extraction with chloroform. The organic layer was dried over anhydrous magnesium sulfate, and the solvent was evaporated under a reduced pressure. The resulting solid was washed with diisopropyl ether, suspended in ethyl acetate, and a 4 M hydrogen chloride-ethyl acetate (2 ml) was added, followed by concentration under a reduced pressure. By washing the resulting solid with ethyl acetate and diethyl ether in that order, 3-{3-[1-(5-bromo-2-thienyl)-1-methylethyl]-5-cyclopropyl-4H-1,2,4-triazol-4-yl}propanoic acid hydrochloride (60 mg) was obtained as a white solid.

### Example 46

2-Methyl-2-butene (282 µl), sodium dihydrogenphosphate (103 mg) and sodium chlorite (98 mg) were added in that order to a tert-butanol-water (2:1, 7.5 ml) mixed solution of 5-[1-(4,5-dicyclopropyl-4H-1,2,4-triazol-3-yl)-1-methylethyl]thiophene-2-carbaldehyde (200 mg), followed by stirring at room temperature for 2 days. Then, 2-methyl-2-butene (141 µl), sodium dihydrogenphosphate (56 mg) and sodium chlorite (60 mg) were further added thereto, followed by stirring at room temperature for 1 day. The solvent was evaporated under a reduced pressure, a saturated sodium bicarbonate aqueous solution was added, followed by washing with diethyl ether. A 1 M hydrochloric acid aqueous solution and sodium chloride were added to the aqueous layer, the pH was adjusted to pH = 5, followed by extraction with chloroform. The extraction was carried out 4 times until carboxylic acid disappeared from the water layer. The organic layer was washed with a saturated sodium chloride aqueous solution and dried over anhydrous magnesium sulfate. Then, the solvent was evaporated under a reduced pressure. The resulting solid was washed with diisopropyl ether, and ethyl acetate (5 ml) and a 4 M hydrogen chloride-ethyl acetate (250 µl) were added. The crystals precipitated were collected by filtration and washed with ethyl acetate to obtain 5-[1-(4,5-dicyclopropyl-4H-1,2,4-triazol-3-yl)-1-methylethyl]thiophene-2-carboxylic acid hydrochloride (186 mg) as colorless crystals.

### Example 47

Tert-butyl 4-(4-methyl-5-{ 1-methyl-1-[5-(trifluoromethyl)-2-thienyl]ethyl}-4H-1,2,4-triazol-3-yl)piperidine-1-carboxylate (402 mg) was dissolved in ethanol (10 ml), a 4 M hydrogen chloride-ethyl acetate solution (1.0 ml) was added, followed by stirring at room temperature for 15 hours. The stirring was further carried out at 60°C for 4 hours. The reaction liquid was concentrated under a reduced pressure, and the resulting solid was washed with ethyl acetate to obtain 4-(4-methyl-5-{1-methyl-1-[5-(trifluoromethyl)-2-thienyl]ethyl}-4H-1,2,4-triazol-3-yl)piperidine dihydrochloride (377.5 mg) as a white solid.

In the same manner as in the above-mentioned Examples 1 to 47, Examples 48 to 284 which are shown later in Tables 22 to 59 were produced using corresponding starting materials. Structures and physicochemical data of Example compounds are shown in Tables 22 to 74.

In addition, structures of other compounds of the present invention are shown in Tables 75 to 82 which are described later. These may be easily produced by the use of the above-mentioned production methods and the methods described in Examples, or the methods obvious to those skilled in the art, or modified methods thereof.

**[Table 2]**

| Rf | RSyn | Structure | Data |
|---|---|---|---|
| 1 | 1 | 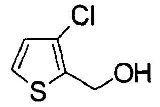 | NMR2 : 1.97-2.07 (1H, m), 4.81 (2H, d), 6.91 (1H, d), 725 (1H, d) |
| 2 | 2 | 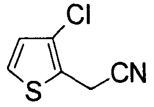 | NMR2 : 3.88(2H,s), 6.93(1H,d), 7.28(1H,d) |
| 44 | 1 | 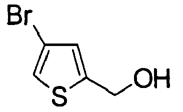 | EI : 192 |
| 45 | 2 | 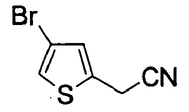 | EI : 201 |
| 46 | 1 | 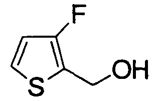 | NMR2 : 1.93 (1H, br), 4.77 (2H,s), 6.77 (1H, d), 7.16 (1H, dd) |
| 47 | 2 | 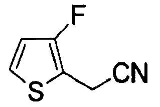 | NMR2 : 3.82 (2H, s), 6.80 (1H, d), 7.18 (1H, dd) |
| 8 | 8 | 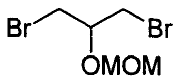 | EI : 261 |
| 27 | 27 | 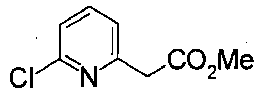 | NMR2 : 3.02 (3H, s), 3.85 (2H, s), 7.2-7.3 (2H, m), 7.64 (1H, t) |
| 7 | 7 | 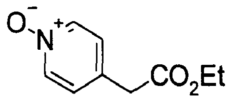 | ESP : 182 |
| 33 | 33 | 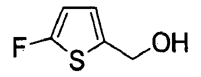 | NMR1 : 4.49-4.52 (2H, m), 5.48 (1H, m), 6.52-6.53 (1H, m), 6.61-6.64 (1H, m) |
| 48 | 2 | 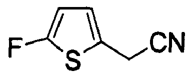 | NMR1 : 4.19-4.20 (2H, m), 6.62-6.64 (1H, m), 6.77-6.79 (1H, m) |
| 49 | 5 | 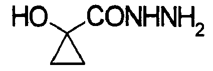 | CI : 117 |
| 50 | 2 | 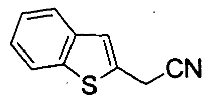 | NMR2 : 4.00 (2H, d), 7.32-7.40 (3H, m), 7.74-7.80 (2H,m) |

**[Table 3]**

| | | | |
|---|---|---|---|
| 34 | 34 | 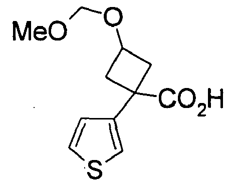 | NMR2 : 2.43-2.52 (2Hx04, m), 2.73-2.87 (4Hx0.6, m), 3.12-3.22 (2Hx0.4, m), 3.36 (3H, s), 4.05-4.14 (1Hx0.6, m), 429-4.37 (1Hx0.4, m), 4.59 (2Hx0.6, s), 4.60 (2Hx0.4, s), 6.99-7.33 (3H, m) |
| 51 | 5 | 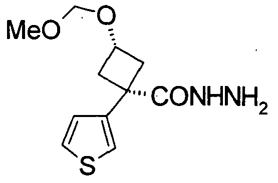 | FP : 257 |
| 52 | 14 | 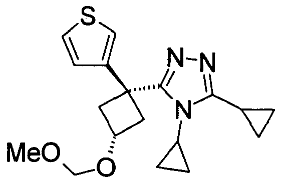 | ESP : 346 |
| 35 | 35 | 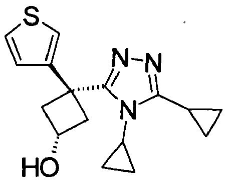 | ESP : 302 |

**[Table 4]**

| | | | | |
|---|---|---|---|---|
| 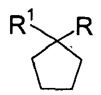 | | | | |

| Rf | RSyn | R¹ | R | Data |
|---|---|---|---|---|
| 53 | 9 | 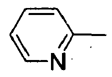 | -CO₂Me | ESP : 206 |
| 54 | 28 | | -C(O)NHNH₂ | NMR2 : 220 - 2.55 (4H, m), 3.77 (2H, d), 7.35 (1H, d) |

**[Table 5]**

| | | | | |
|---|---|---|---|---|
| 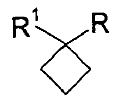 | | | | |

| Rf | RSyn | R¹ | R | Data |
|---|---|---|---|---|
| 23 | 23 | BocNH- | -CO₂Me -C(O)NHNH₂ | FP : 230 |
| 24 | 24 | | | FP : 230 |
| 36 | 36 | 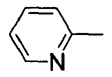 | -CO₂Et -C(O)NHNH₂ | ESP : 206 |
| 55 | 28 | | | ESP : 192 |
| 56 | 36 | 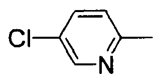 | -CO₂Et | NMR2 : 1.19 (3H, t), 2.03-2.13 (1H, m), 2.19-229 (1H, m), 2.56-2.67 (2H, m), 2.74-2.85 (2H, m), 724 (1H, d), 7.62 (1H, t), 8.52 (1H, d) |
| 57 | 28 | | -C(O)NHNH₂ | NMR2 : 1.80-1.90 (1H, m), 2.00-2.10 (1H, m), 2.53-2.60 (2H, m), 2.78-2.88 (2H, m), 7.30 (1H, d), 7.65 (1H, dd), 3.52 (1H, d) |

**[Table 6]**

| | | | | |
|---|---|---|---|---|
| 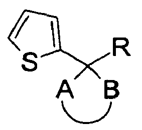 | | | | |

| Rf | RSyn | 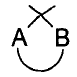 | R | Data |
|---|---|---|---|---|
| 9 | 9 | 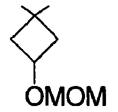 | -CN | EI : 223 |
| 12 | 12 | | -CO₂H | FN : 241 |
| 58 | 5 | | -C(O)NHNH₂ | FP : 257 |
| 59 | 22 | 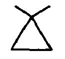 | -CO₂Et | EI : 196 |
| 60 | 28 | | -C(O)NHNH₂ | EI : 182 |

**[Table 7]**

| | | | | |
|---|---|---|---|---|
| 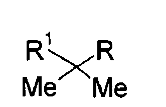 | | | | |

| Rf | RSyn | R¹ | R | Data |
|---|---|---|---|---|
| 6 | 6 | 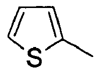 | -C(O)NHNH₂ | ESP : 185 |
| 3 | 3 | 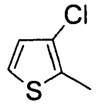 | -CN | NMR2 : 1.86 (6H, s), 6.96 (1H, d), 7.20 (1H, d) |
| 4 | 4 | | -CO₂H | ESN : 203 |
| 5 | 5 | | -C(O)NHNH₂ | ESN : 217 |
| 61 | 3 | 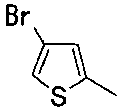 | -CN | EI : 229 |
| 62 | 4 | | -CO₂H | EI : 248 |
| 63 | 5 | | -C(O)NHNH₂ | FP : 263 |
| 22 | 22 | PhNH- | CO₂Et | ESP : 208 |
| 64 | 6 | | -C(O)NHNH₂ | ESP : 194 |
| 29 | 29 | 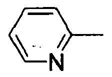 | -C(O)NHNH₂ | ESP : 180 |
| 28 | 28 | 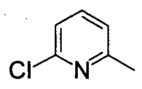 | -CO₂Me | NMR2 : 1.60 (6H, s), 3.69 (3H, s), 7.22 (2H, d), 7.60 (1H, t) |
| 65 | 29 | | -C(O)NHNH₂ | NMR2 : 1.63 (6H, s), 3.84 (2H, brs), 722 (1H, d), 732 (1H, d), 7.64 (1H, t), 7.66 (1H, brs) |
| 10 | 10 | 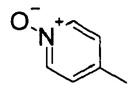 | -CO₂Et | ESP : 210 |
| 11 | 11 | 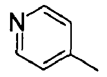 | CO₂Et | ESP : 194 |
| 66 | 6 | | -C(O)NHNH₂ | ESP : 180 |
| 67 | 28 | 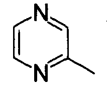 | -CO₂Me | ESP : 181 |
| 68 | 29 | | -C(O)NHNH₂ | EI : 180 |
| 69 | 28 | 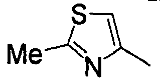 | -CO₂Et | NMR2 : 1.21 (3H, t), 1.60 (6H, s), 2.68 (3H, s), 4.15 (2H, q), 6.87 (1H, s) |
| 70 | 29 | | -C(O)NHNH₂ | EI : 199 |
| 71 | 28 | 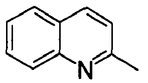 | -CO₂Me | ESP : 230 |
| 72 | 29 | | -C(O)NHNH₂ | ESP : 230 |
| 73 | 15 | 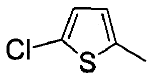 | -CO₂Et | EI : 232 |
| 74 | 6 | | -C(O)NHNH₂ | EI : 218 |

**[Table 8]**

| | | | | |
|---|---|---|---|---|
| 75 | 16 | 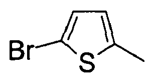 | -CO₂Et | NMR2 : 1.24 (3H, t), 1.61 (6H, s), 4.14 (2H, q), 6.70 (1H, d), 6.88 (1H, d) |
| 76 | 6 | | -C(O)NHNH₂ | NMR1 : 1.50 (6H, s), 4.22-4.26 (2H, br), 6.77 (1H, d), 7.03 (1H, d), 8.94-8.98 (1H, br) |
| 37 | 37 | 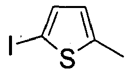 | -CO₂Et | FP : 324 |
| 77 | 28 | 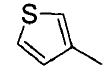 | -CO₂Et | EI : 198 |
| 78 | 6 | | -C(O)NHNH₂ | FP : 185 |
| 38 | 38 | 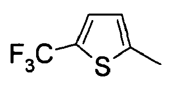 | -CO₂Et | NMR1 : 1.16 (3H, t), 1.61 (6H, s) 4.11 (2H, q), 7.12-7.14 (1H, m), 7.57-7.59 (1H, m) |
| 79 | 6 | | -C(O)NHNH₂ | NMR1 : 1.56 (6H, s), 427 (2H, brs), 7.04-7.06 (1H, m), 7.52-7.53 (1H, m), 9.08 (1H, brs) |
| 39 | 39 | | -CO₂H | NMR1 : 1.60 (6H, s), 7.12-7.14 (1H, m), 7.56-7.59 (1H, m) |
| 40 | 40 | 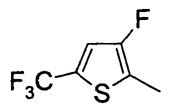 | -CO₂H | NMR1 : 1.70 (6H, s), 7.14 (1H,brs) |
| 41 | 41 | | -CO₂Me | NMR1 : 1.66 (6H, s), 3.74 (3H, s), 7.12 (1H,brs) |
| 80 | 6 | | -C(O)NHNH₂ | NMR1 : 1.67 (6H, s), 6.83-6.99 (1H, br), 7.17 (1H,brs) |
| 81 | 3 | 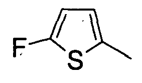 | -CN | NMR1 : 1.72 (6H, s), 6.66-6.68 (1H, m), 6.88-6.90 (1H, m) |
| 82 | 4 | | -CO₂H | NMR1 : 1.49 (6H, s), 6.53-6.55(1H, m), 6.64-6.66 (1H, m), 12.66 (1H, brs) |
| 83 | 5 | | -C(O)NHNH₂ | NMR1 : 1.47 (6H, s), 423 (2H, brs), 6.49-6.52 (1H, m), 6.58-6.60 (1H, m), 8.96 (1H, brs) |
| 84 | 3 | 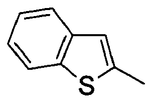 | -CN | NMR2 : 1.86 (6H, s), 732-7.39 (3H, m), 7.73-7.81 (2H, m) |
| 85 | 4 | | -CO₂H | NMR2 : 1.74 (6H, s), 7.23-7.34 (3H, m), 7.69-7.78 (2H, m) |
| 86 | 5 | | -C(O)NHNH₂ | NMR2 : 1.71 (6H, s), 327 (2H, brs), 7.22-7.37 (3H, m), 7.71-7.73 (1H, m), 7.78-7.79 (1H, m) |

**[Table 9]**

| | | | | |
|---|---|---|---|---|
| 87 | 3 | 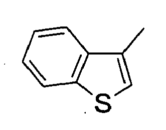 | -CN | NMR1 : 1.84 (6H, s), 7.42-7.54 (2H, m), 7.78 (1H, s), 8.06-8.12 (2H, m) |
| 88 | 4 | | -CO₂H | NMR1 : 1.59 (6H, s), 7.32-7.40 (2H, m), 7.58 (1H, s), 7.68-7.71 (1H, m), 7.97-8.00 (1H, m), 12.48 (1H, brs) |
| 89 | 5 | | -C(O)NHNH₂ | NMR1 : 1.55 (6H, s), 4.15 (2H, brs), 7.30-734 (2H, m), 7.53 (1H, s), 7.65-7.68 (1H, m), 7.95-7.97 (1H, m), 8.69 (1H, brs) |
| 90 | 36 | 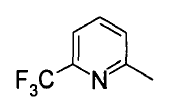 | -CO₂Me | NMR2 : 1.63 (6H, s), 3.69 (3H, s), 7.46 (1H, d), 7.53 (1H, d), 7.82 (1H, t) |
| 91 | 28 | | -C(O)NHNH₂ | NMR2 : 1.68 (6H, s), 3.82 (2H, s), 7.57 (1H, d), 7.61 (1H, d), 7.73 (1H, s), 7.87 (1H, t) |

**[Table 10]**

| | | | |
|---|---|---|---|
| 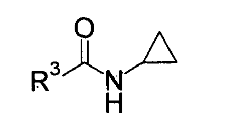 | | | |

| Rf | RSyn | R³ | Data |
|---|---|---|---|
| 92 | 18 | cBu | ESP : 140 |
| 18 | 18 | tBu | ESP : 142 |
| 93 | 18 | cPen | ESP : 154 |
| 94 | 18 | cHex | ESP : 168 |
| 95 | 18 | cHep | ESP : 182 |
| 42 | 42 | 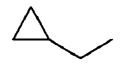 | NMR2 : 0.15-0.21 (2H, m), 0.48-0.54 (2H, m), 0.56-0.63 (2H, m), 0.76-0.83 (2H, m), 0.87-0.98 (1H, m), 2.12 (2H, d), 2.69-2.77 (1H, m), 6.03 (1H, brs) |
| 96 | 42 | 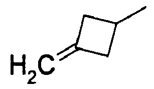 | EI : 151 |
| 97 | 42 | CF₃CH₂- | NMR1 : 0.36-0.41 (2H, m), 0.61-0.68 (2H, m), 2.61-2.67 (1H, m), 3.16 (2H, dd), 8.31 (1H, brs) |
| 98 | 42 | CF₃-(CH₂)₂- | NMR1 : 0.34-0.39 (2H, m), 0.57-0.64 (2H, m), 2.25-2.30 (2H, m), 2.39-2.63 (3H, m), 8.06 (1H, brs) |
| 99 | 42 | 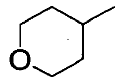 | EI : 169 |
| 100 | 42 | 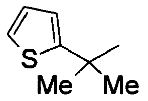 | ESP : 210 |
| 101 | 42 | 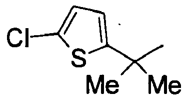 | FP : 244 |

**[Table 11]**

| | | | |
|---|---|---|---|
| 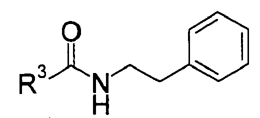 | | | |

| Rf | RSyn | R³ | Data |
|---|---|---|---|
| 102 | 18 | cPr | FP : 190 |
| 103 | 42 | cBu | NMR1 : 1.68-1.76 (1H, m), 1,79-2.01 (3H, m), 2.03-2.13 (2H, m), 2.67-2.71 (2H, m), 2.90-2.99 (1H, m), 321-326 (2H, m), 7.17-720 (3H, m), 7.26-7.30 (2H, m), 7.72(1H, m) |
| 104 | 18 | Me | FP : 164 |
| 105 | 42 | CF₃CH₂- | NMR2 : 2.80-2.87 (2H, t), 2.95-3.07 (2H, q), 3.54-3.60 (2H, dt), 5.76 (1H, brs), 7.17-721 (2H, m), 722-727 (1H, m), 7.29-7.35 (2H, m) MH-003 |
| 106 | 42 | 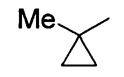 | NMR1 : 0.46-0.48 (2H, m), 0.90-0.93 (2H, m), 1.23 (3H, s), 2.69-2.73 (2H, m), 323-328 (2H, m), 7.17-720 (3H, m), 7.27-7.31 (2H, m), 7.58 (1H, m) |
| 107 | 42 | 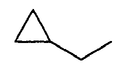 | ESP : 204 |

**[Table 12]**

| | | | |
|---|---|---|---|
| 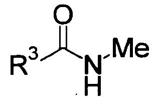 | | | |

| Rf | RSyn | R³ | Data |
|---|---|---|---|
| 108 | 42 | 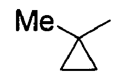 | CI : 114 |
| 109 | 42 | 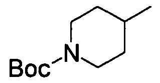 | FP : 243 |

**[Table 13]**

| | | | |
|---|---|---|---|
| 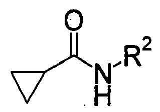 | | | |

| Rf | RSyn | R² | Data |
|---|---|---|---|
| 110 | 18 | -CH₂CFH₂ | EI : 131 |
| 111 | 18 | -(CH₂)₂CO₂Et | FP:186 |
| 112 | 18 | 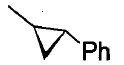 | CI : 202 |

**[Table 14]**

| | | | |
|---|---|---|---|
| 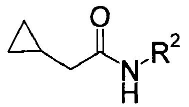 | | | |

| Rf | RSyn | R² | Data |
|---|---|---|---|
| 113 | 42 | Pr | NMR1 : 0.08-0.12 (2H, m), 039-0.42 (2H, m), 0.83 (3H, t), 0.90-0.99 (1H, m), 1.35-1.44 (2H, m), 1.95 (2H, d), 2.99 (2H, q), 7.67 (1H, brs) |
| 114 | 42 | 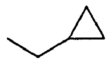 | FP : 154 |

**[Table 15]**

| | | | |
|---|---|---|---|
| 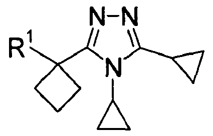 | | | |

| Rf | RSyn | R¹ | Data |
|---|---|---|---|
| 25 | 25 | BocHN- | ESP : 319 |
| 26 | 26 | H₂N- | ESP : 219 |

**[Table 16]**

| | | | | | |
|---|---|---|---|---|---|
| 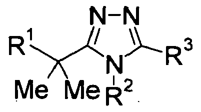 | | | | | |

| Rf | RSyn | R¹ | R² | R³ | Data |
|---|---|---|---|---|---|
| 115 | 25 | BocHN- | Me | cPr | ESP : 281 |
| 116 | 26 | H₂N- | Me | | ESP : 181 |
| 117 | 25 | BocHN- | cPr | | ESP : 307 |
| 118 | 26 | H₂N- | | | ESP : 207 |
| 119 120 | 25 | BocHN- | | cBu | FP : 321 |
| | 26 | H₂N- | | | FP : 221 |
| 121 | 25 | BocHN- | | 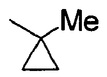 | ESP : 321 |
| 122 | 26 | H₂N- | | | FP : 221 |
| 123 | 25 | BocHN- | Me | cHep | ESP : 337 |
| 124 | 26 | H₂N- | | | FP : 237 |
| 125 | 25 | BocHN- | -(CH₂)₂Ph | cPr | ESP : 371 |
| 126 | 26 | H₂N- | | | FP : 371 |
| 127 | 25 | BocHN- | cPr | cHep | ESP : 363 |
| 128 | 26 | H₂N- | | | ESP : 263 |

**[Table 17]**

| | | | | |
|---|---|---|---|---|
| 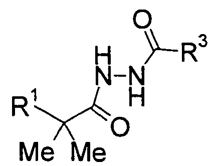 | | | | |

| Rf | RSyn | R¹ | R³ | Data |
|---|---|---|---|---|
| 30 | 30 | 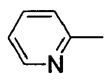 | cPr | NMR2 : 1.68 (6H, s), 720 (1H, dd), 7.41 (1H, d), 7.69 (1H, dt), 8.55 (1H, d) |
| 129 | 30 | | Me | NMR2 : 1.71 (6H, s), 2.03 (3H, s), 7.30 (1H, dd), 7.50 (1H, d), 7.79 (1H, dt), 8.65 (1H, d) |
| 130 | 30 | | Et | NMR2 : 1.17 (3H, t), 1.69 (6H, s), 2.27 (2H, q), 724 (1H, dd), 7.44 (1H, d), 7.73 (1H, dt), 8.64 (1H, d) |
| 131 | 30 | | Pr | NMR2 : 0.95 (3H, t), 1.70 (2H, sext), 1.71 (6H, s), 2.22 (2H, t), 727 (1H, dd), 7.48 (1H, d), 7.77 (1H, dt), 8.65 (1H, d) |
| 132 | 30 | | Bu | NMR2 : 0.91 (3H, t), 1.39 (2H, sext), 1.64 (2H, quint), 1.71 (6H, s), 225 (2H, t), 727 (1H, dd), 7.47 (1H, d), 7.76 (1H, dt), 8.66 (1H, d) |
| 133 | 30 | 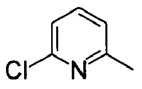 | cPr | ESP : 282 |
| 134 | 30 | | cBu | NMR2 : 1.67 (6H, s), 3.07 (1H, quint), 7.34 (1H, d), 7.65 (1H, t), 8.02 (1H, d) |
| 135 | 30 | 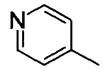 | cPr | ESP : 248 |
| 136 | 30 | 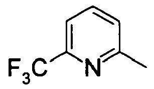 | cPr | NMR2 : 0.79-0.84 (2H, m), 1.00-1.07 (2H, m), 1.45 (1H, dt), 1.72 (6H, s), 7.59 (1H, d), 7.63 (1H, d), 7.88 (1H, t) |
| 31 | 31 | 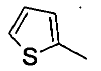 | cPr | EI : 252 |
| 137 | 31 | 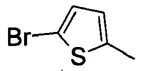 | tBu | ESP : 349 |

**[Table 18]**

| | | | | |
|---|---|---|---|---|
| 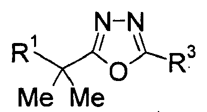 | | | | |

| Rf | RSyn | R¹ | R³ | Data |
|---|---|---|---|---|
| 17 | 17 | 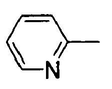 | cPr | ESP : 230 |
| 138 | 21 | | 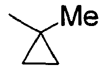 | ESP : 244 |
| 21 | 21 | | iPr | ESP : 232 |
| 139 | 21 | | cBu | ESP : 244 |
| 140 | 21 | | cPen | ESP : 258 |
| 141 | 17 | | cHex | ESP : 272 |
| 142 | 17 | | Me | ESP : 204 |
| 143 | 17 | | Et | ESP : 218 |
| 144 | 17 | | Pr | NMR2 : 0.98 (3H, t), 1.77 (2H, sext), 1.87 (6H, s), 2.79 (2H, t), 2.18-234 (2H, m), 7.69 (1H, t), 8.59 (1H, d) |
| 145 | 17 | | Bu | ESP : 246 |
| 146 | 17 | 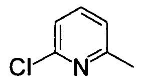 | cPr | ESP : 264 |
| 147 | 17 | | cBu | ESP : 278 |
| 148 | 17 | 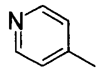 | cPr | ESP : 230 |
| 149 | 17 | 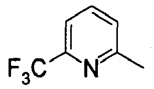 | cPr | ESP : 298 |
| 32 | 32 | 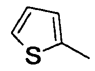 | cPr | EI : 234 |
| 150 | 16 | 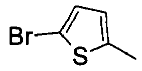 | cPr | FP : 315 |
| 151 | 32 | 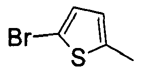 | tBu cPr | ESP : 331 ESP : 264 |
| 152 | 17 | 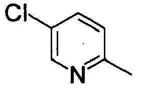 | | |

**[Table 19]**

| | | | |
|---|---|---|---|
| 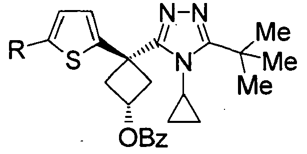 | | | |

| Rf | RSyn | R | Data |
|---|---|---|---|
| 20 | 20 | H | ESP : 422 |
| 153 | 15 | Cl | ESP : 456 |

**[Table 20]**

| | | | | |
|---|---|---|---|---|
| 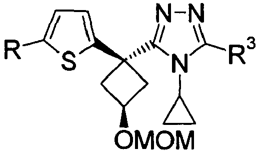 | | | | |

| Rf | RSyn | R | R³ | Data |
|---|---|---|---|---|
| 13 | 13 | H | cPr | ESP : 346 |
| 154 | 13 | | 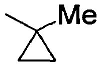 | ESP : 360 |
| 155 | 13 | | cBu | ESP : 360 |
| 15 | 15 | Cl | cPr | ESP : 380 |
| 156 | 15 | | cBu | ESP : 394 |
| 16 | 16 | Br | cPr | ESP : 426 |
| 157 | 16 | | cBu | ESP : 440 |

**[Table 21]**

| | | | | |
|---|---|---|---|---|
| 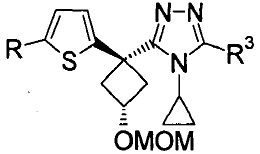 | | | | |

| Rf | RSyn | R | R³ | Data |
|---|---|---|---|---|
| 14 | 14 | H | cPr | ESP : 346 |
| 158 | 14 | | 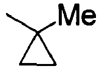 | ESP : 360 |
| 159 | 14 | | cBu | ESP : 360 |
| 19 | 19 | | tBu | ESP : 362 |
| 160 | 15 | Cl | cPr | ESP : 380 |
| 161 | 15 | | cBu | ESP : 394 |
| 162 | 16 | Br | cPr | ESP : 426 |
| 163 | 16 | | cBu | ESP : 440 |
| 43 | 43 | CN | cBu | ESP : 385 |

**[Table 22]**

| Ex | Structure | Sal |
|---|---|---|
| 48 | 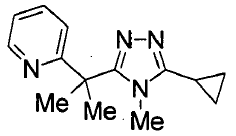 | |
| 49 | 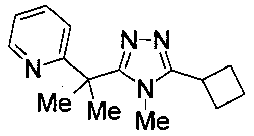 | |
| 50 | 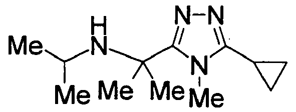 | HCl |
| 51 | 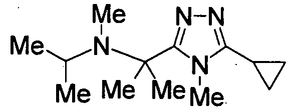 | HCl |
| 52 | 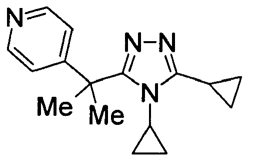 | HCl |
| 53 | 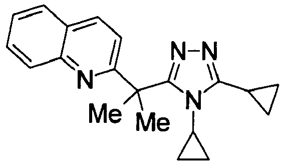 | |
| 7 | 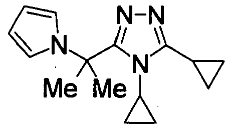 | HCl |
| 8 | 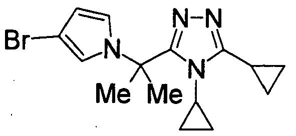 | |
| 9 | 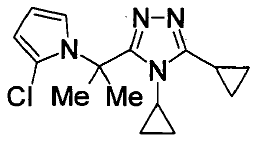 | |

**[Table 23]**

| | | |
|---|---|---|
| 20 | 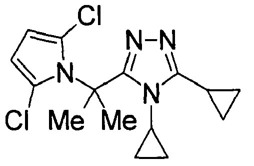 | HCl |
| 21 | 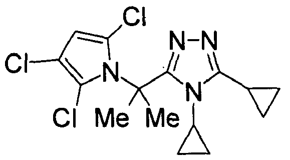 | HCl |
| 10 | 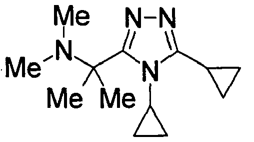 | HCl |
| 11 | 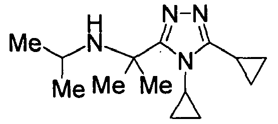 | HCl |
| 12 | 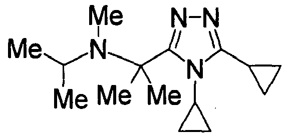 | HCl |
| 54 | 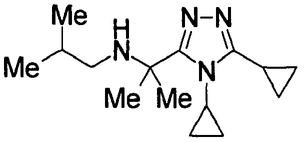 | 2HCl |
| 55 | 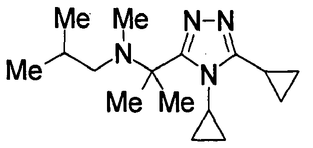 | 2HCl |
| 56 | 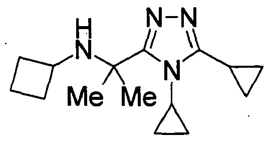 | HCl |
| 57 | 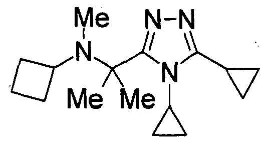 | HCl |

**[Table 24]**

| | | |
|---|---|---|
| 58 | 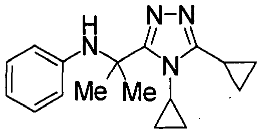 | |
| 59 | 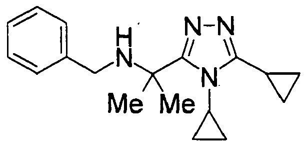 | |
| 60 | 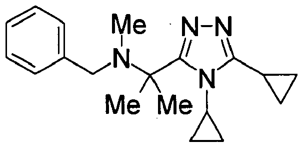 | 2HCl |
| 61 | 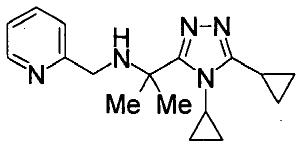 | HCl |
| 62 | 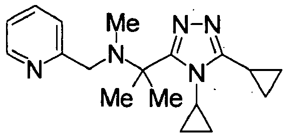 | HCl |
| 13 | 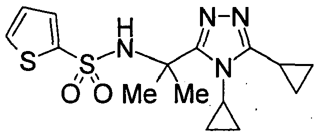 | |
| 63 | 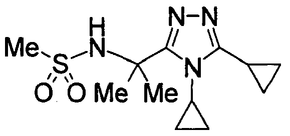 | |
| 64 | 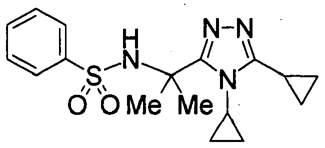 | |
| 65 | 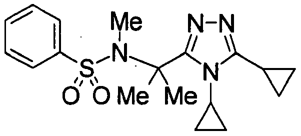 | |

**[Table 25]**

| | | |
|---|---|---|
| 14 | 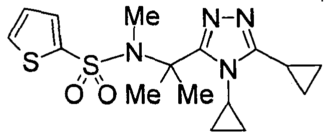 | |
| 66 | 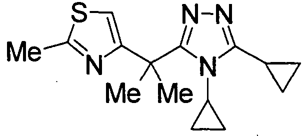 | |
| 67 | 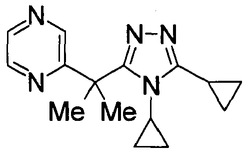 | |
| 68 | 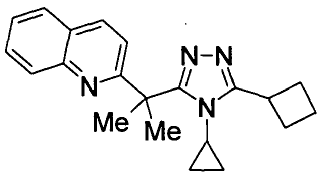 | |
| 5 | 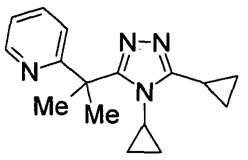 | HCl |
| 69 | 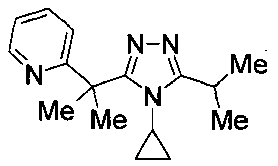 | HCl |
| 70 | 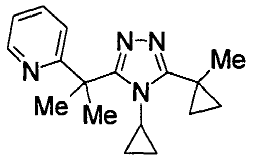 | HCl |
| 71 | 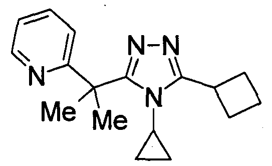 | HCl |

**[Table 26]**

| | | |
|---|---|---|
| 72 | 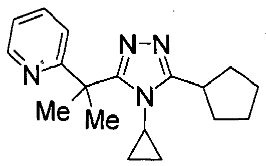 | HCl |
| 73 | 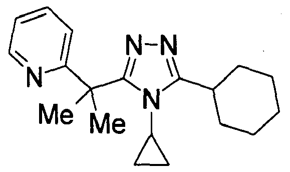 | HCl |
| 74 | 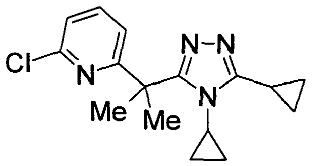 | HCl |
| 75 | 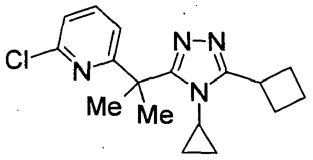 | HCl |
| 6 | 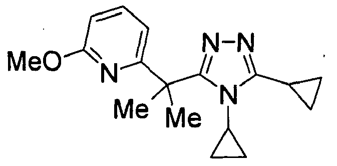 | HCl |
| 1 | 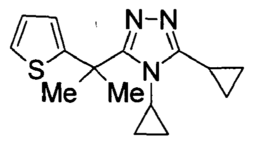 | |
| 2 | 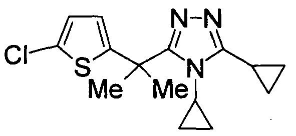 | |
| 3 | 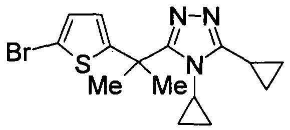 | |

**[Table 27]**

| | | |
|---|---|---|
| 4 | 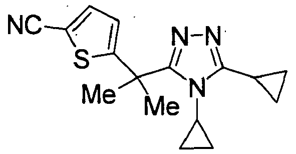 | |
| 76 | 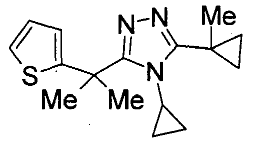 | |
| 77 | 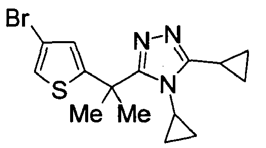 | |
| 78 | 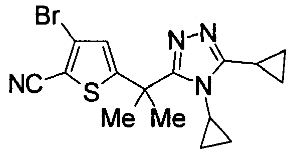 | |
| 19 | 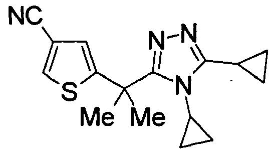 | |
| 79 | 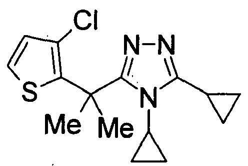 | |
| 80 | 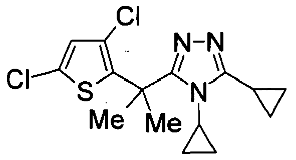 | |
| 81 | 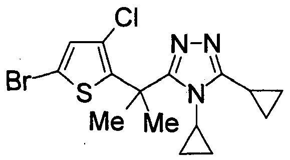 | |

**[Table 28]**

| | | |
|---|---|---|
| 82 | 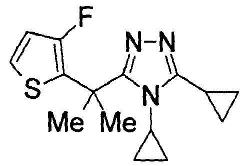 | |
| 83 | 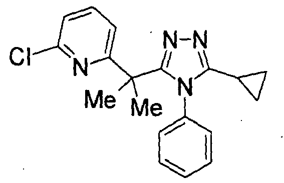 | |
| 84 | 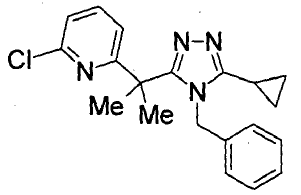 | |
| 85 | 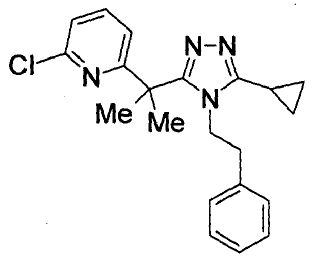 | |
| 86 | 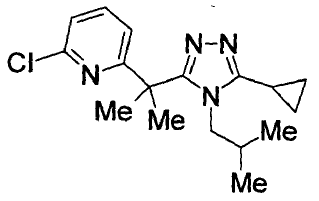 | |
| 87 | 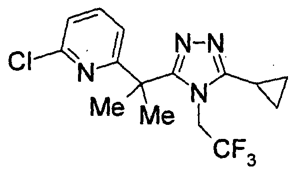 | |

**[Table 29]**

| | | |
|---|---|---|
| 88 | 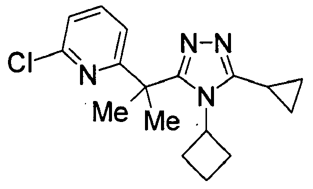 | HCl |
| 89 | 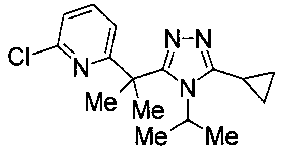 | |
| 90 | 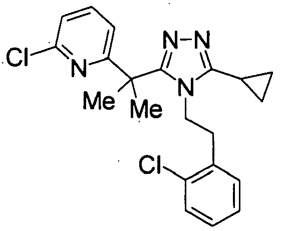 | |
| 91 | 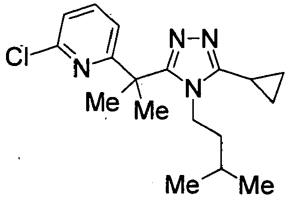 | |
| 92 | 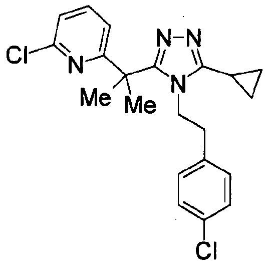 | |
| 93 | 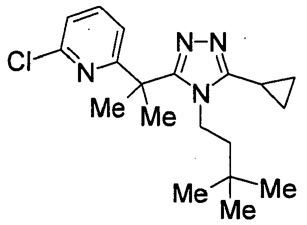 | |

**[Table 30]**

| | | |
|---|---|---|
| 94 | 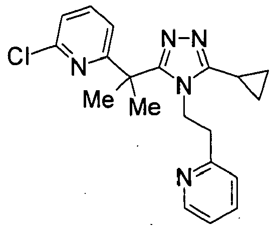 | |
| 95 | 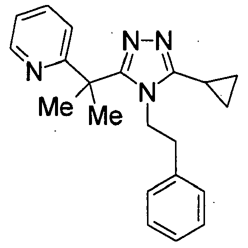 | |
| 96 | 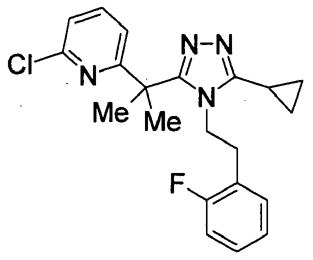 | |
| 97 | 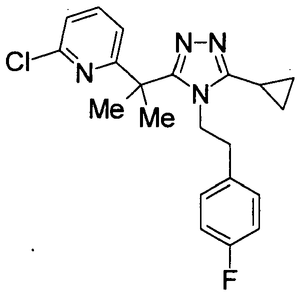 | |
| 98 | 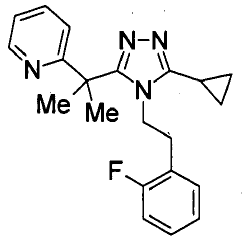 | |

**[Table 31]**

| | | |
|---|---|---|
| 99 | 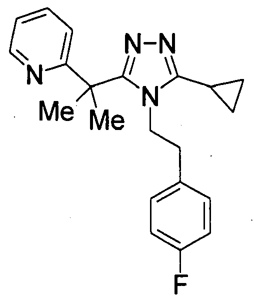 | |
| 100 | 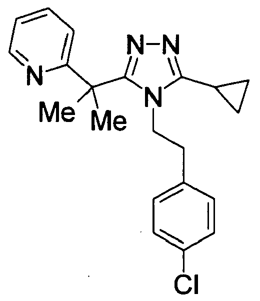 | |
| 101 | 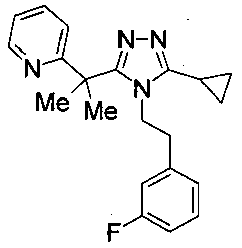 | |
| 102 | 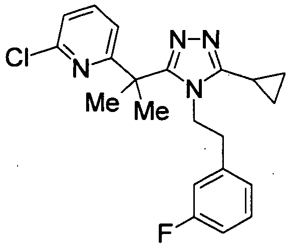 | |
| 103 | 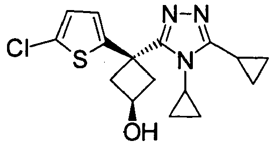 | |

**[Table 32]**

| | | |
|---|---|---|
| 104 | 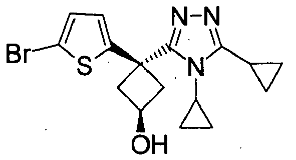 | |
| 105 | 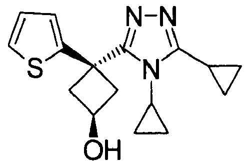 | |
| 106 | 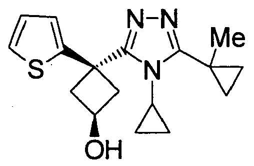 | |
| 107 | 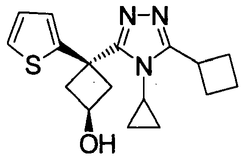 | |
| 15 | 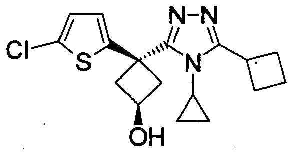 | |
| 108 | 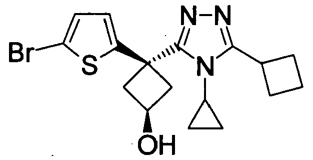 | |
| 109 | 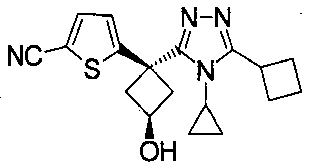 | |
| 110 | 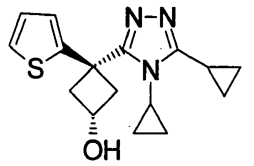 | |

**[Table 33]**

| | | |
|---|---|---|
| 111 | 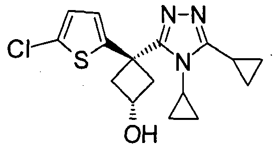 | |
| 112 | 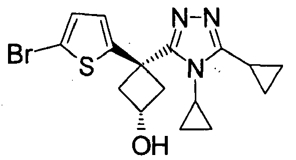 | |
| 113 | 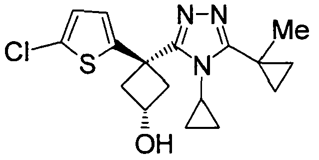 | |
| 114 | 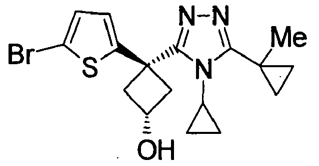 | |
| 115 | 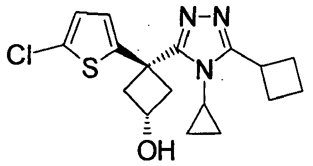 | |
| 116 | 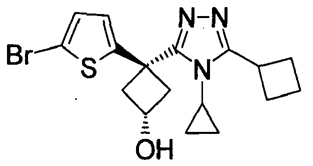 | |
| 18 | 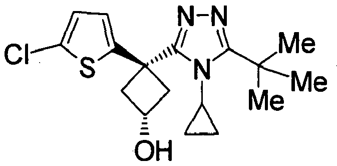 | |
| 117 | 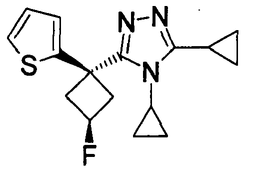 | |

**[Table 34]**

| | | |
|---|---|---|
| 16 | 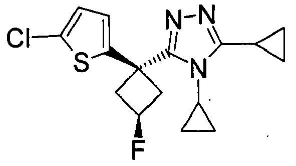 | |
| 118 | 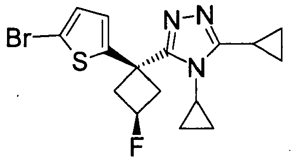 | |
| 119 | 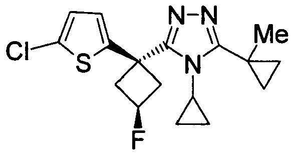 | |
| 120 | 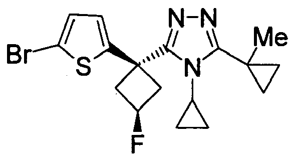 | |
| 121 | 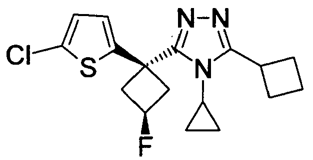 | |
| 122 | 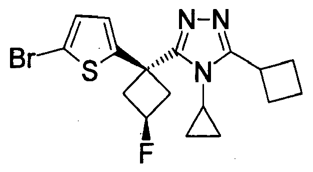 | |
| 123 | 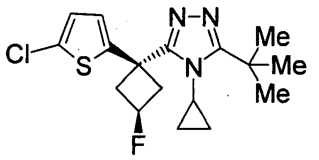 | |
| 17 | 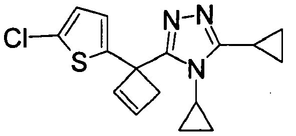 | |

**[Table 35]**

| | | |
|---|---|---|
| 124 | 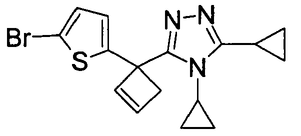 | |
| 125 | 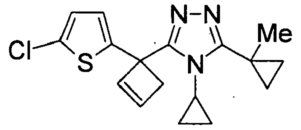 | |
| 126 | 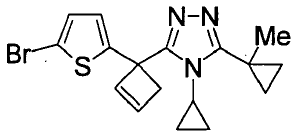 | |
| 127 | 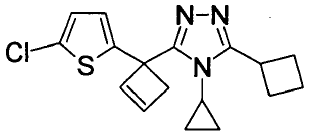 | |
| 128 | 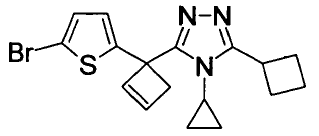 | |
| 129 | 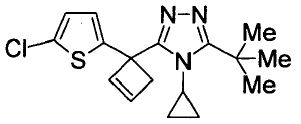 | |
| 130 | 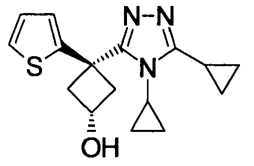 | |
| 131 | 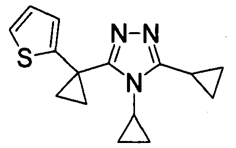 | |
| 132 | 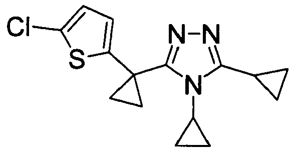 | |

**[Table 36]**

| | | |
|---|---|---|
| 133 | 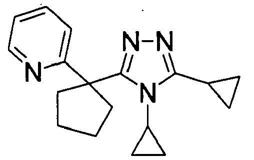 | HCl |
| 134 | 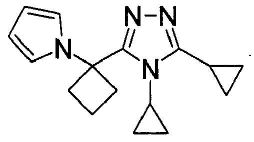 | HCl |
| 135 | 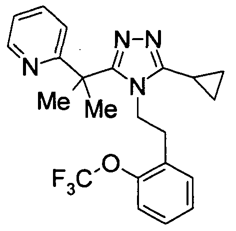 | |
| 136 | 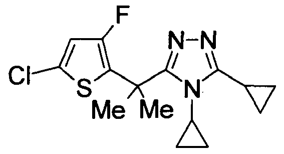 | |
| 137 | 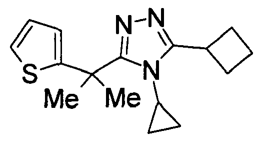 | |
| 138 | 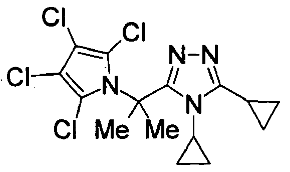 | |
| 28 | 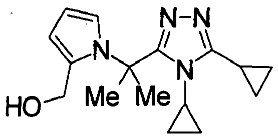 | |
| 139 | 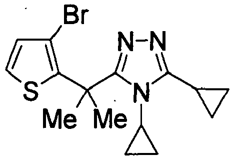 | |

**[Table 37]**

| | | |
|---|---|---|
| 140 | 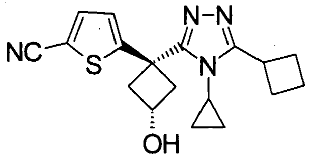 | |
| 141 | 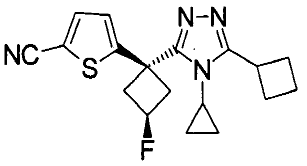 | |
| 142 | 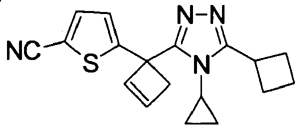 | |
| 143 | 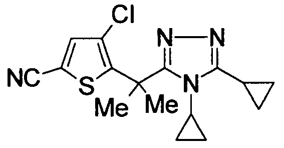 | |
| 144 | 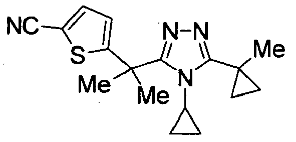 | |
| 145 | 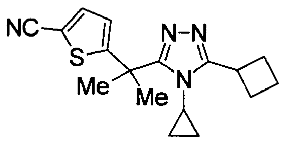 | |
| 146 | 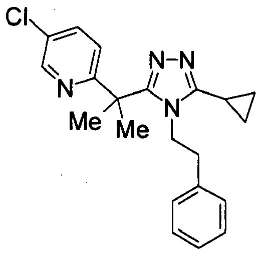 | |
| 147 | 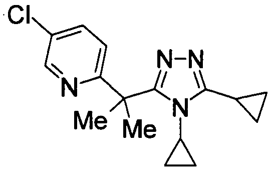 | |

**[Table 38]**

| | | |
|---|---|---|
| 148 | 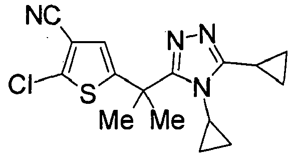 | |
| 30 | 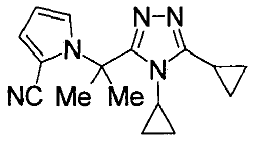 | |
| 149 | 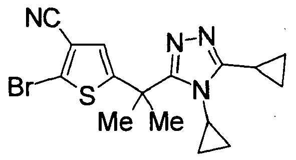 | |
| 150 | 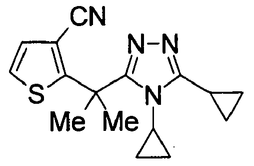 | |
| 29 | 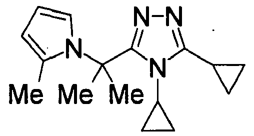 | |
| 151 | 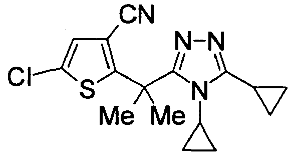 | |
| 152 | 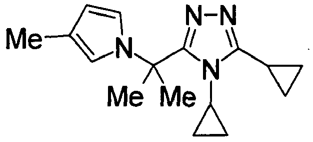 | |
| 153 | 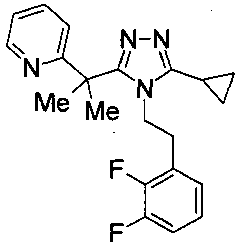 | |

**[Table 39]**

| | | |
|---|---|---|
| 154 | 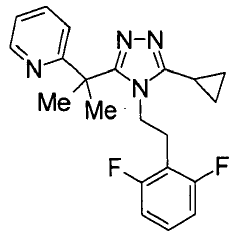 | |
| 155 | 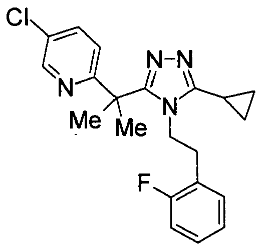 | |
| 156 | 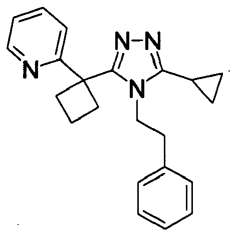 | |
| 40 | 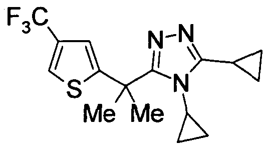 | |
| 157 | 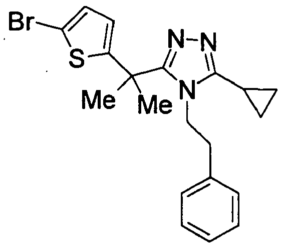 | |
| 158 | 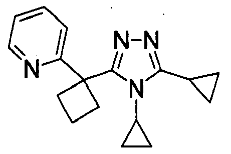 | |

**[Table 40]**

| | | |
|---|---|---|
| 159 | 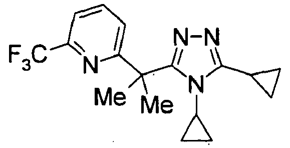 | |
| 160 | 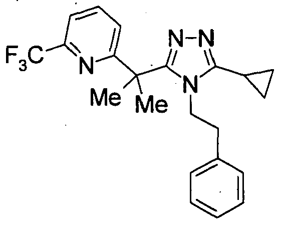 | |
| 161 | 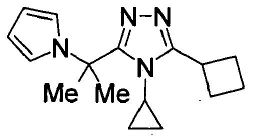 | |
| 162 | 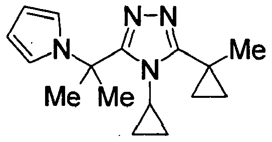 | |
| 163 | 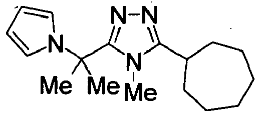 | |
| 164 | 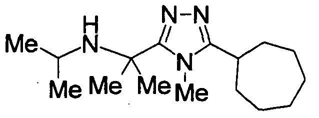 | |
| 165 | 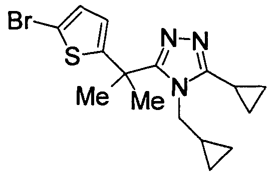 | |
| 166 | 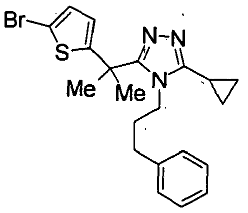 | |

**[Table 41]**

| | | |
|---|---|---|
| 167 | 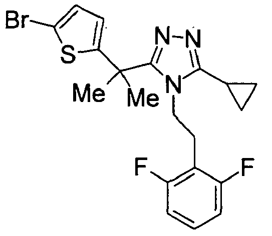 | |
| 168 | 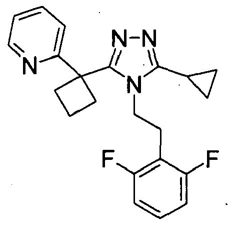 | |
| 169 | 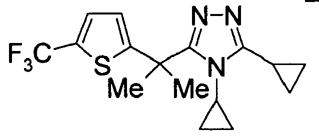 | |
| 170 | 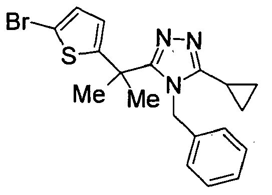 | HCl |
| 171 | 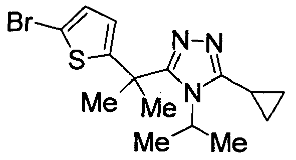 | HCl |
| 172 | 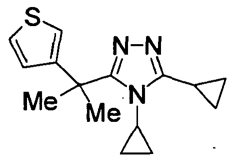 | |
| 173 | 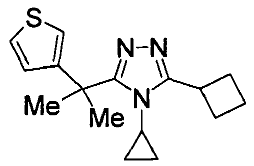 | |

**[Table 42]**

| | | |
|---|---|---|
| 174 | 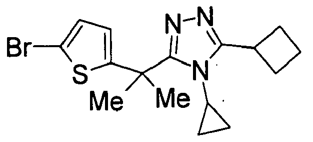 | |
| 175 | 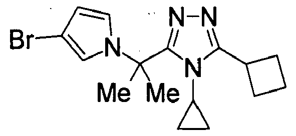 | |
| 176 | 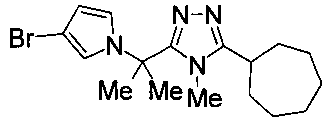 | |
| 177 | 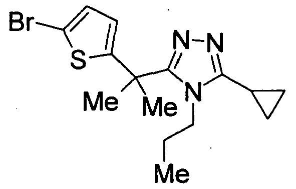 | HCl |
| 178 | 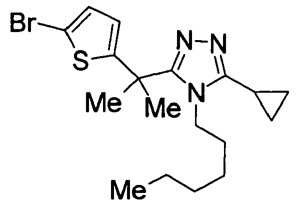 | HCl |
| 179 | 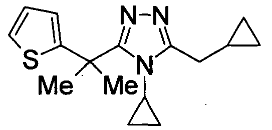 | |
| 180 | 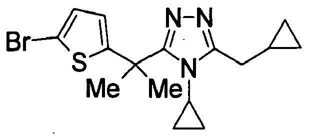 | |
| 181 | 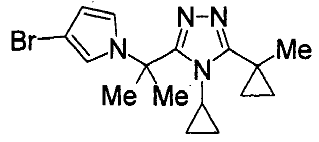 | |

**[Table 43]**

| | | |
|---|---|---|
| 182 | 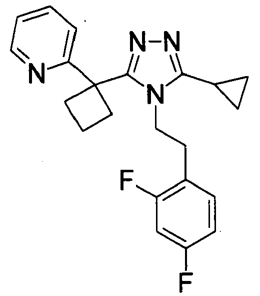 | |
| 22 | 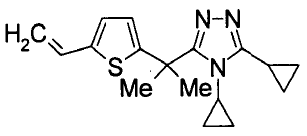 | |
| 183 | 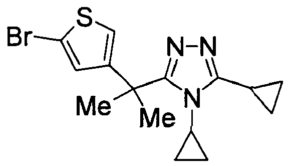 | |
| 184 | 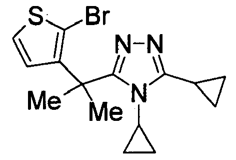 | |
| 185 | 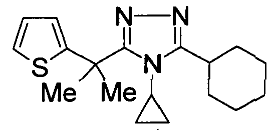 | |
| 41 | 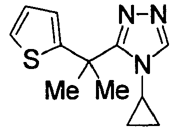 | |
| 186 | 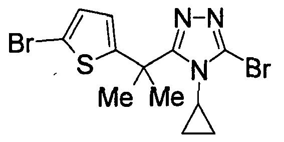 | |
| 187 | 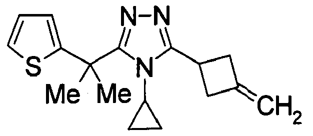 | |

**[Table 44]**

| | | |
|---|---|---|
| 188 | 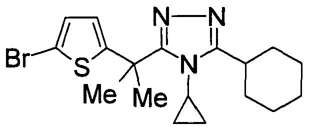 | |
| 189 | 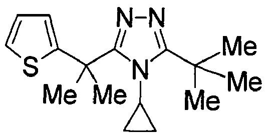 | |
| 190 | 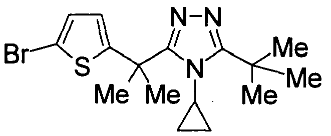 | |
| 191 | 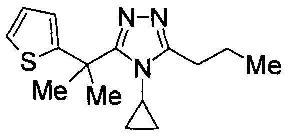 | HCl |
| 192 | 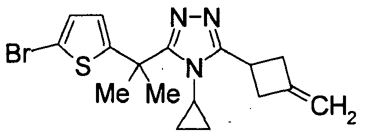 | |
| 193 | 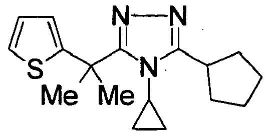 | |
| 23 | 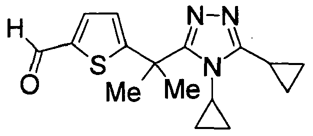 | |
| 194 | 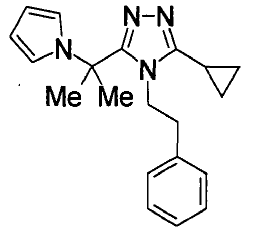 | |
| 24 | 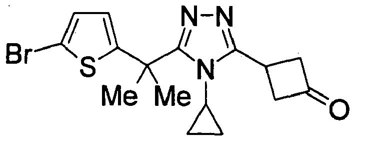 | |

**[Table 45]**

| | | |
|---|---|---|
| 195 | 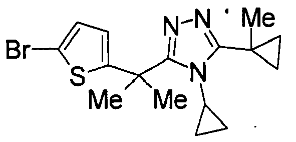 | |
| 196 | 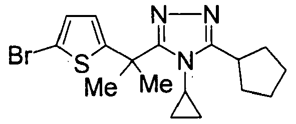 | |
| 197 | 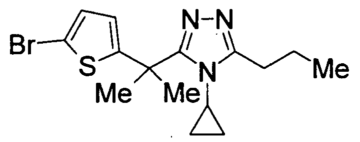 | |
| 198 | 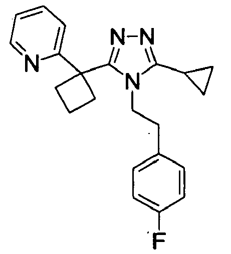 | |
| 199 | 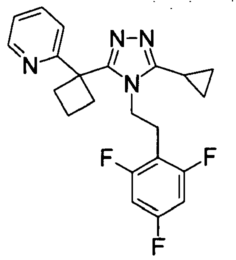 | |
| 25 | 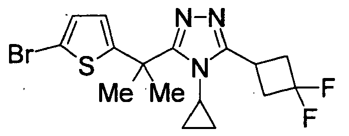 | HCl |
| 200 | 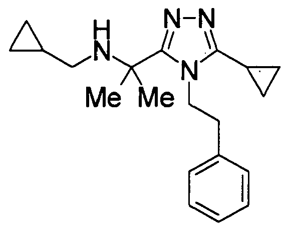 | |

**[Table 46]**

| | | |
|---|---|---|
| 201 | 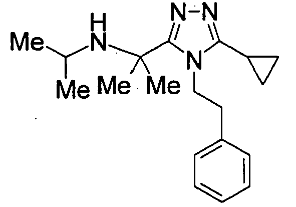 | |
| 202 | 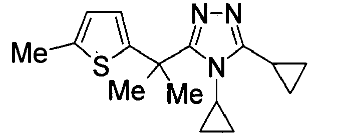 | |
| 26 | 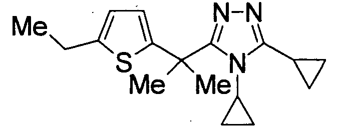 | |
| 203 | 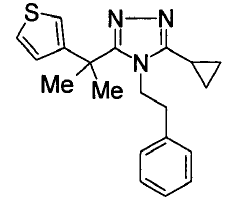 | HCl |
| 204 | 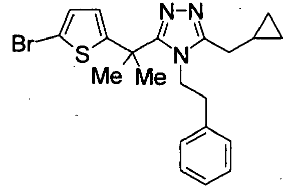 | HCl |
| 205 | 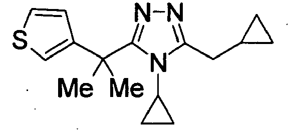 | HCl |
| 206 | 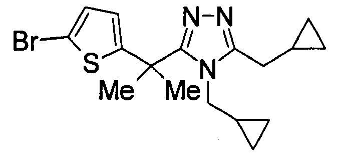 | HCl |
| 207 | 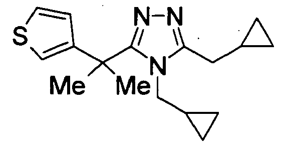 | HCl |

**[Table 47]**

| | | |
|---|---|---|
| 208 | 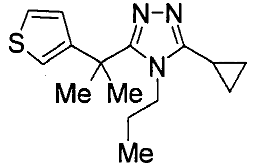 | HCl |
| 209 | 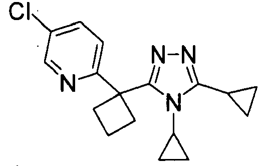 | |
| 210 | 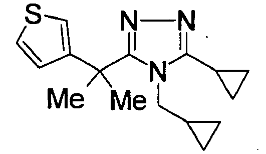 | HCl |
| 211 | 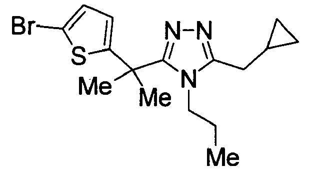 | HCl |
| 31 | 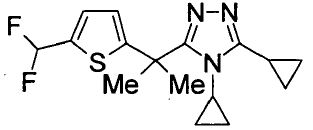 | |
| 32 | 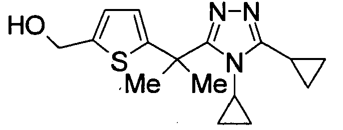 | |
| 212 | 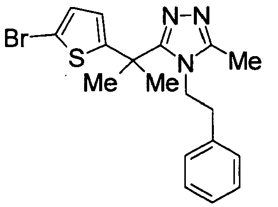 | HCl |
| 213 | 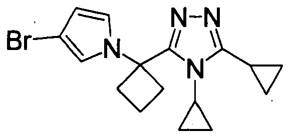 | |

**[Table 48]**

| | | |
|---|---|---|
| 214 | 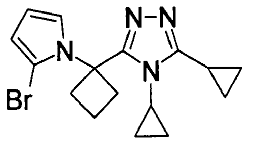 | |
| 215 | 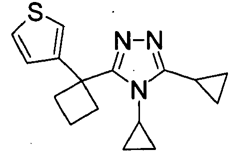 | |
| 216 | 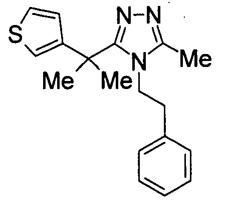 | HCl |
| 217 | 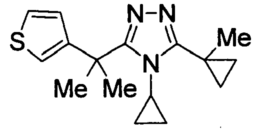 | HCl |
| 218 | 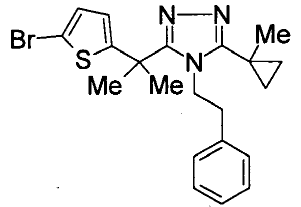 | HCl |
| 43 | 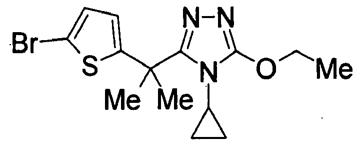 | |
| 219 | 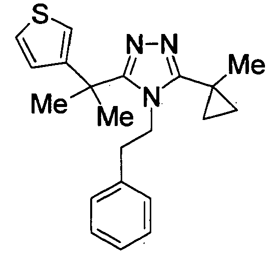 | HCl |

**[Table 49]**

| | | |
|---|---|---|
| 220 | 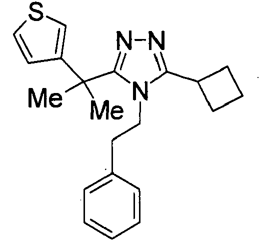 | HCl |
| 44 | 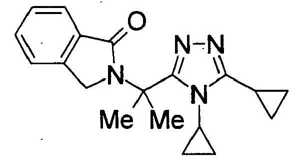 | |
| 221 | 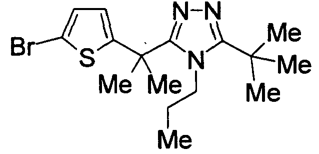 | HCl |
| 222 | 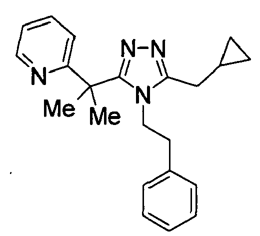 | |
| 223 | 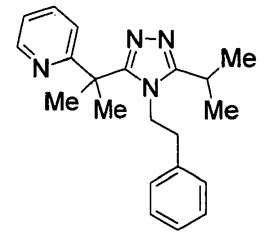 | |
| 224 | 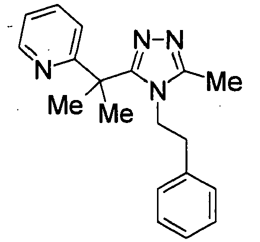 | |

**[Table 50]**

| | | |
|---|---|---|
| 225 | 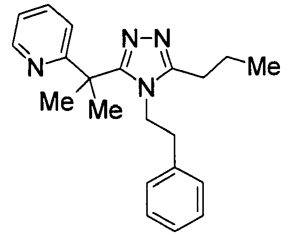 | |
| 226 | 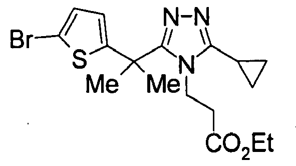 | HCl |
| 227 | 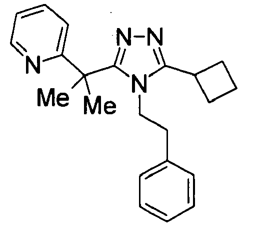 | |
| 45 | 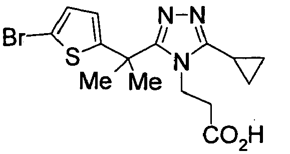 | HCl |
| 228 | 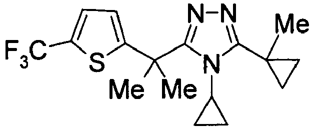 | HCl |
| 229 | 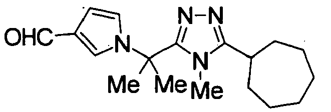 | |
| 33 | 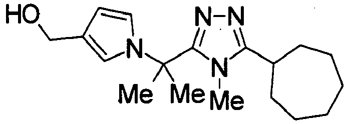 | HCl |
| 34 | 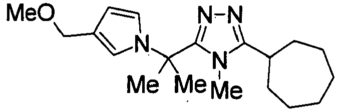 | HCl |

**[Table 51]**

| | | |
|---|---|---|
| 230 | 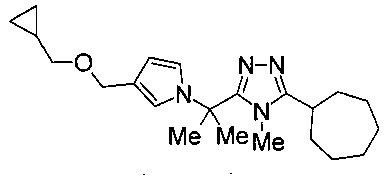 | HCl |
| 231 | 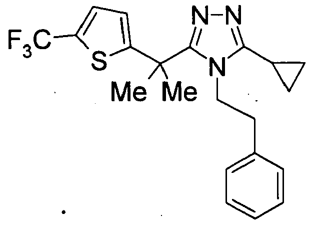 | HCl |
| 35 | 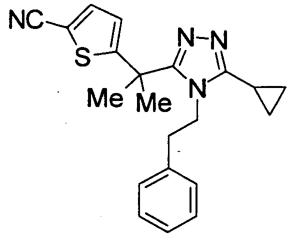 | HCl |
| 232 | 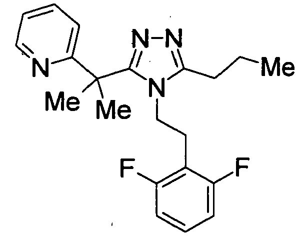 | |
| 233 | 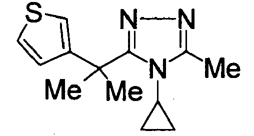 | HCl |
| 234 | 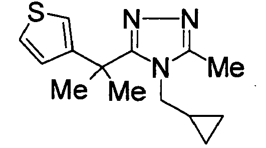 | HCl |

**[Table 52]**

| | | |
|---|---|---|
| 235 | 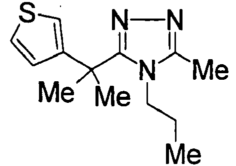 | HCl |
| 236 | 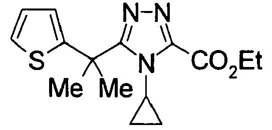 | |
| 237 | 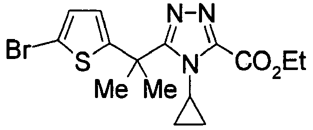 | |
| 238 | 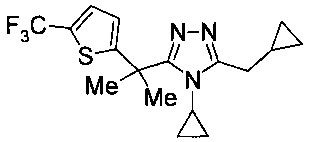 | HCl |
| 239 | 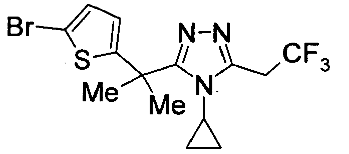 | HCl |
| 240 | 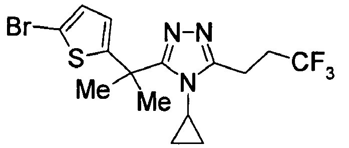 | HCl |
| 241 | 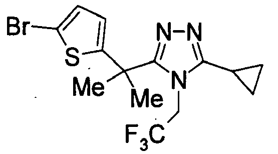 | HCl |
| 242 | 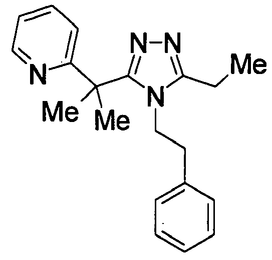 | |

**[Table 53]**

| | | |
|---|---|---|
| 243 | 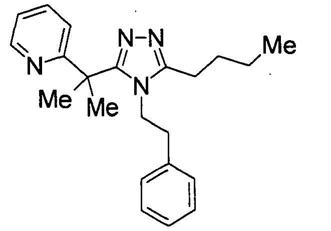 | |
| 244 | 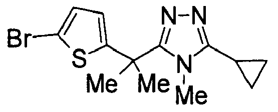 | HCl |
| 245 | 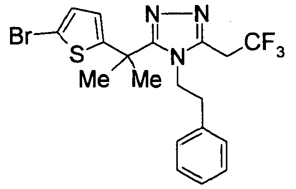 | HCl |
| 46 | 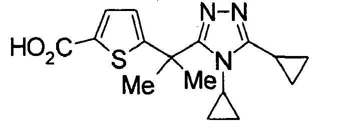 | HCl |
| 246 | 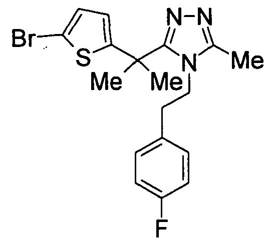 | HCl |
| 247 | 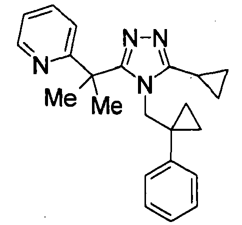 | |
| 248 | 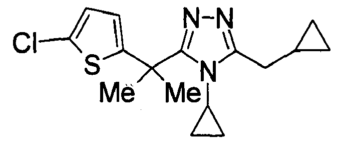 | HCl |

**[Table 54]**

| | | |
|---|---|---|
| 249 | 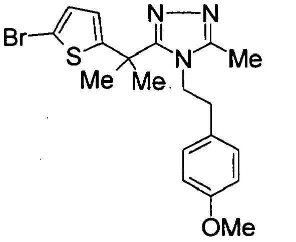 | HCl |
| 250 | 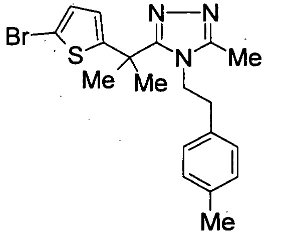 | HCl |
| 251 | 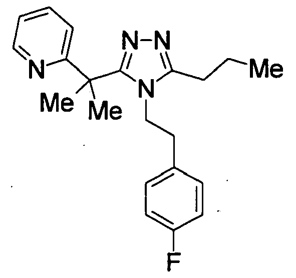 | |
| 252 | 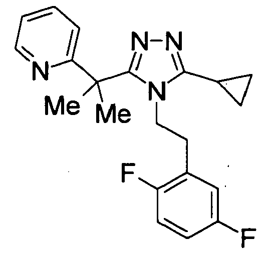 | |
| 253 | 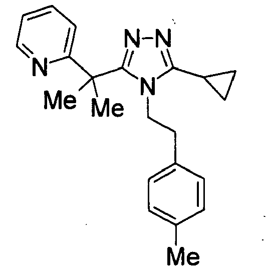 | |

**[Table 55]**

| | | |
|---|---|---|
| 254 | 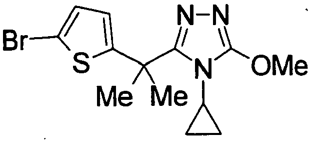 | |
| 255 | 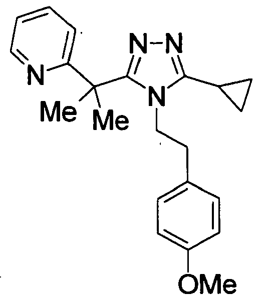 | |
| 256 | 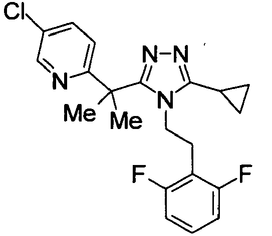 | |
| 257 | 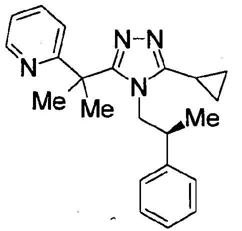 | |
| 258 | 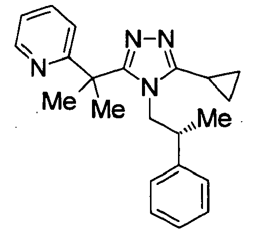 | |
| 259 | 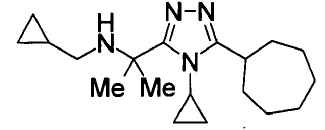 | HCl |

**[Table 56]**

| | | |
|---|---|---|
| 260 | 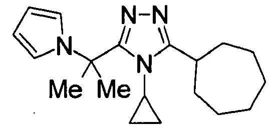 | |
| 261 | 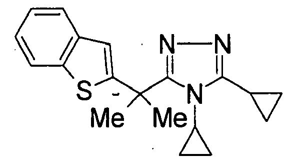 | HCl |
| 262 | 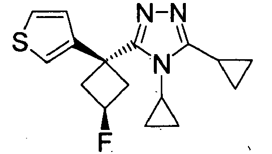 | HCl |
| 36 | 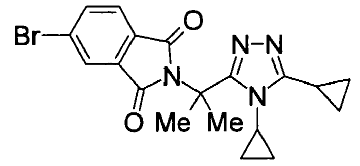 | |
| 263 | 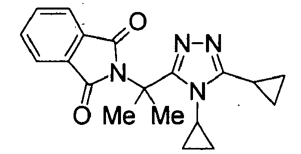 | |
| 264 | 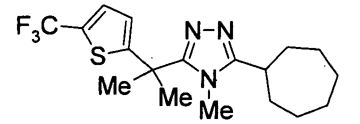 | HCl |
| 265 | 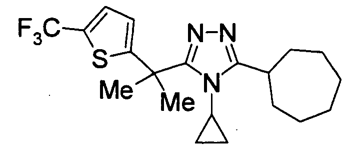 | HCl |
| 266 | 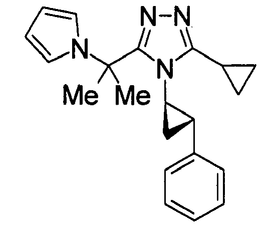 | |

**[Table 57]**

| | | |
|---|---|---|
| 267 | 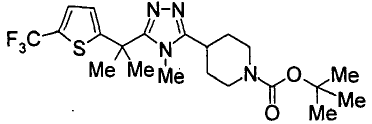 | |
| 268 | 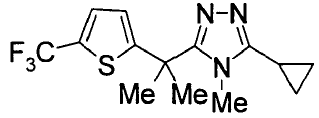 | HCl |
| 269 | 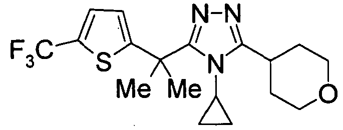 | HCl |
| 270 | 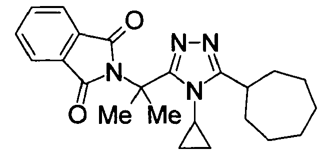 | |
| 271 | 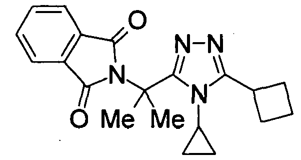 | |
| 272 | 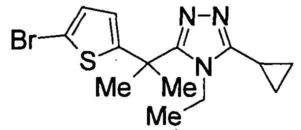 | HCl |
| 273 | 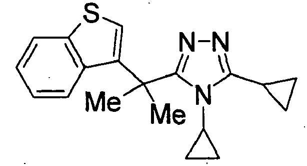 | HCl |
| 47 | 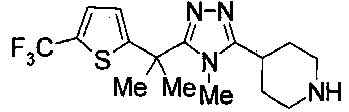 | 2HCl |
| 274 | 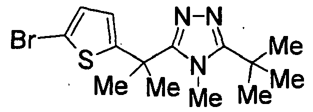 | HCl |

**[Table 58]**

| | | |
|---|---|---|
| 275 | 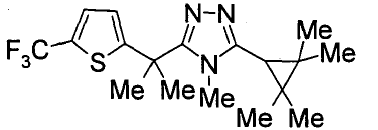 | HCl |
| 276 | 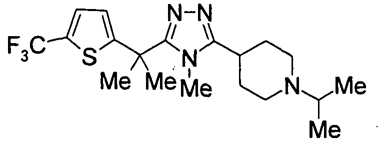 | HCl |
| 277 | 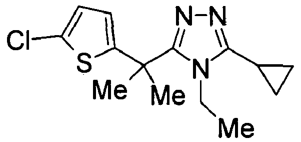 | HCl |
| 42 | 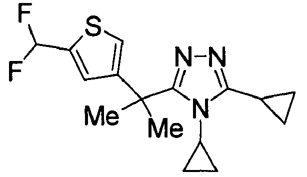 | HCl |
| 278 | 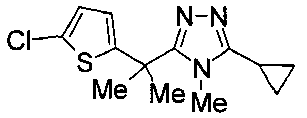 | HCl |
| 279 | 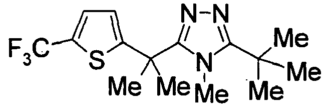 | HCl |
| 280 | 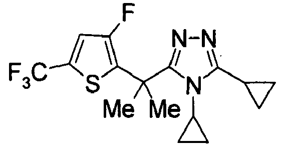 | HCl |
| 37 | 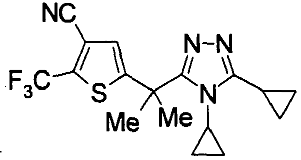 | HCl |
| 281 | 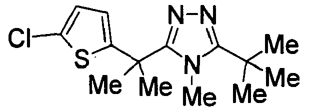 | HCl |

**[Table 59]**

| | | |
|---|---|---|
| 38 | 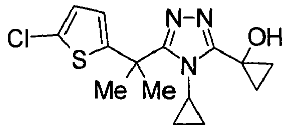 | |
| 39 | 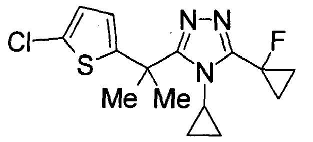 | |
| 282 | 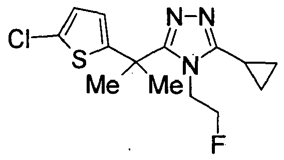 | HCl |
| 283 | 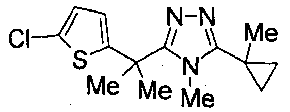 | HCl |
| 284 | 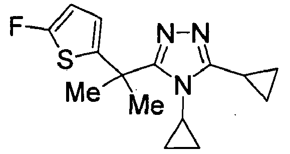 | HCl |
| 27 | 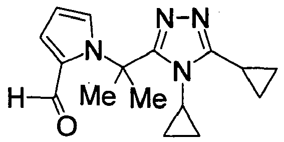 | |

**[Table 60]**

| Ex | Syn | Data |
|---|---|---|
| 48 | 5 | ESP : 243 |
| 49 | 5 | ESP : 257 |
| 50 | 11 | FP : 223 |
| 51 | 12 | FP : 237 |
| 52 | 5 | ESP : 269 |
| 53 | 5 | ESP : 319 |
| 7 | | FP : 257 ; NMR1 : 0.39-0.43 (2H, m), 0.88-0.93 (2H, m), 1.21-1.35 (4H, m), 1.99 (6H, s), 224-2.31 (1H, m), 3.07-3.13 (1H, m), 6.08(2H, t), 6.75(2H, t) |
| 8 | | FP : 336 ; NMR1 : 0.28-0.32 (2H, m), 0.86-1.02 (6H, m), 1.95 (6H, s), 2.01-2.08 (1H, m), 2.98-3.03 (1H, m), 6.12 (1H, dd), 6.61 (1H, t), 6.81 (1H, t) |
| 9 | | FP : 291 ; NMR1 : 0.66-0.80 (4H, m), 0.88-1.00 (4H, m),1.98 (6H, s), 1.98-2.06 (1H, m), 2.50-2.57 (1H, m), 6.11 (2H, d), 7.12 (1H, t) |
| 20 | | FP : 325 ; NMR1 : 0.92-0.99 (4H, m), 1.20-1.28 (4H, m), 2.18 (6H, s), 227-2.34 (1H, m), 2.80-2.86 (1H, m), 629 (2H, s) |
| 21 | | FP : 359 ; NMR2 : 1.03-1.09 (2H, m), 1.23-1.34 (4H, m), 1.67-1.76 (2H, m), 2.12-2.24 (1H, m), 231 (6H, s), 2.73-2.84 (1H, m), 6.20 (1H, s) |
| 10 | 10 | FP : 235 |
| 11 | 11 | FP : 249 |
| 12 | | FP : 263 ; NMR1 : 0.80-2.10 (14H, m), 1.08 (6H, d), 2.10-2.16 (1H, m), 2.68 (3H, s), 3.28-3.69 (2H, m) |
| 54 | 11 | ESN : 261 |
| 55 | 12 | FP : 277 |
| 56 | 11 | FP : 261 |
| 57 | 12 | FP : 275 ; NMR1 : 0.98-1.15 (4H, m), 1.21-1.36 (4H, m),1.50-1.66 (2H, m), 1.74-2.03 (2H, m), 1.86 (6H, s), 2.11-2.18 (1H, m), 2.22-2.43 (2H, m), 2.59 (3H, s), 3.58-3.66 (1H, m), 3.89-4.02 (1H, m) |
| 58 | 1 | ESP : 283 |
| 59 | 11 | FP : 297 |
| 60 | 12 | FP : 311 |
| 61 | 11 | FP : 298 |
| 62 | 12 | FP : 312 |
| 13 | 13 | ESP : 353 |
| 63 | 13 | ESP : 285 |
| 64 | 13 | ESP : 347 |
| 65 | 14 | ESP : 361 |
| 14 | 14 | ESP : 367 |
| 66 | 1 | ESP : 289 |

**[Table 61]**

| | | |
|---|---|---|
| 67 | 1 | ESP : 270 |
| 68 | 5 | ESP : 333 |
| 5 | 5 | ESP : 269 ; NMR1 : 0.40-0.47 (2H, m), 0.74-0.82 (2H, m), 124-128 (4H, m), 1.85 (6H, s), 2.31 (1H, tt), 3.09 (1H, tt), 7.37 (1H, dd), 7.52 (1H, d), 7.89 (1H, dt), 8.49 (1H, dd) |
| 69 | 5 | ESP : 271 |
| 70 | 5 | ESP : 283 ; NMR1 : 0.54-0.58 (2H, m), 0.74-0.81 (2H, m), 0.98-1.02 (2H, dd), 1.36-1.40 (2H, dd), 1.48 (3H, s), 1.86 (6H, s), 3.08 (1H, tt), 737 (1H, dd), 7.52 (1H, d), 7.90 (1H, dt), 8.49 (1H, dd) |
| 71 | 5 | ESP : 283 ; NMR1 : 027-0.33 (2H, m), 0.68-0.75 (2H, m), 1.86 (6H s), 1.87-1.94 (1H, m), 2.05-2.15 (1H, m), 2.35-2.55 (4H, m), 2.99 (1H, tt), 3.97 (1H, quint), 737 (1H, dd), 7.52 (1H, d), 7.89 (1H, dt), 8.47 (1H, dd) |
| 72 | 5 | ESP : 297 ; NMR1 : 0.36-0.42 (2H, m), 0.75-0.82 (2H, m), 1.66-1.99 (6H, m), 1.88 (6H, s), 2.13-2.18 (2H, m), 3.10 (1H, tt), 3.53 (1H, quint), 7.39 (1H, dd), 7.56 (1H, d), 7.92 (1H, dt), 8.48 (1H, dd) |
| 73 | 5 | ESP : 311 ; NMR1 : 0.37-0.40 (2H, m), 0.74-0.82 (2H, m), 1.24-1.86 (8H, m), 1.87 (6H, s), 2.04-2.09 (2H, m), 3.10-3.19 (2H, m), 7.37 (1H, dd), 7.54 (1H, d), 7.90 (1H, dt), 8.47 (1H, dd) |
| 74 | 5 | ESP : 303 ; NMR2 : 0.74-0.80 (2H, m), 0.90-0.96 (2H, m), 136-1.41 (2H, m), 1.78-1.88 (2H, m), 1.95 (6H, s), 2.16-224 (1H, m), 2.96-3.00 (1H, m), 7.10-720 (2H, m), 7.70 (1H, t) |
| 75 | 5 | ESP : 317 ; NMR2 : 0.67-0.71 (2H, m), 0.80-0.87 (2H,m), 1.95 (6H, s), 2.10-2.18 (2H, m), 2.50-2.55 (2H, m), 2.80-2.93 (3H, m), 3.95 (1H, quint), 726 (1H, d), 7.31 (1H, d), 7.70 (1H, t) |
| 6 | 6 | ESP : 299 ; NMR2 : 0.69-0.75 (2H, m), 0.87-0.95 (2H, m), 136 (2H, dt), 1.78-1.82 (2H, m), 1.94 (6H, s), 2.18 (1H, tt), 2.92 (1H, dt), 3.75 (3H, s), 6.66 (1H, d), 6.87 (1H, d), 7.60 (1H, dd) |
| 1 | 1 | FP : 274 ; NMR1 : 0.44-0.48 (2H, m), 0.82-0.99 (6H, m), 1.85 (6H, s), 1.99-2.05 (1H, m), 2.94-3.00 (1H, m), 6.80 (1H, d), 6.95(1H, dd), 7.38 (1H, d) |
| 2 | 2 | FP : 308 ; NMR1 : 0.56-0.61 (2H, m), 0.90-1.00 (6H, m), 1.82 (6H, s), 2.00-2.06 (1H, m), 3.01-3.06 (1H, m), 6.70 (1H, d), 6.96(1H, d) |
| 3 | 3 | FP : 354 ; NMR1 : 0.55-0.59 (2H, m), 0.90-1.00 (6H, m), 1.83 (6H, s), 1.99-2.06 (1H, m), 3.00-3.06 (1H, m), 6.67 (1H, d), 7.06 (1H, d) |
| 4 | 4 | FP : 299 ; NMR1 : 0.49-0.53 (2H, m), 0.89-1.01 (6H, m), 1.89 (6H, s), 2.00-2.06 (1H, m), 3.01-3.07 (1H, m), 7.02 (1H, d), 7.84 (1H, d) |
| 76 | 1 | ESP : 288 ; NMR1 : 0.56-0.63 (2H, m), 0.74-0.79 (2H, m), 0.82-0.90 (2H, m), 1.08-1.13 (2H, m), 1.40 (3H, s), 1.85 (6H, s), 2.94-3.02 (2H, m), 6.80 (1H, d), 6.94 (1H, dd), 7.38 (1H, d) |
| 77 | 1 | ESP : 352 ; NMR1 : 0.45-0.56 (2H, m), 0.85-1.05 (6H, m), 1.83 (6H, s), 1.94-2.10 (1H, m), 2.95-3.10 (1H, m), 6.88 (1H, d), 7.54 (1H, d) |

**[Table 62]**

| | | |
|---|---|---|
| 78 | 4 | ESP : 377 ; NMR1 : 0.50-0.64 (2H, m), 0.86-1.08 (6H, m),1.88 (6H, s), 1.98-2.10 (1H, m), 3.00-3.14 (1H, m), 728 (1H, s) |
| 19 | 19 | ESP : 299 ; NMR1 : 0.46-0.54 (2H, m), 0.85-1.05 (6H, m), 1.87 (6H, s), 1.98-2.07 (1H, m), 2.98-3.06 (1H, m), 729 (1H, s), 8.43 (1H, s) |
| 79 | 1 | ESP : 308 ; NMR1 : 0.75-0.95 (4H, m), 0.96-1.05 (2H, m), 1.12-120 (2H, m), 1.85-1.97 (1H, m), 1.93 (6H, s), 2.49-2.58 (1H, m), 6.85 (1H, d), 7.16 (1H, d) |
| 80 | 2 | ESP : 342 ; NMR1 : 0.82-1.04 (6H, m), 1.11-120 (2H, m), 1.77-1.97 (1H, m), 1.89 (6H, s), 2.55-2.67 (1H, m), 6.70 (1H, s) |
| 81 | 3 | ESP : 388 ; NMR1 : 0.88-1.11 (6H, m), 1.12-1.30 (2H, m), 1.88-2.03 (1H, m), 1.90 (6H, s), 2.56-2.70 (1H, m), 6.84 (1H, s) |
| 82 | 1 | ESP : 292 ; NMR2 : 0.78-1.08 (6H, m), 1.14-124 (2H, m), 1.86-1.99 (1H, m), 1.93 (6H, s), 2.68-2.80 (1H, m), 6.72 (1H, d), 7.03 (1H, dd) |
| 83 | 5 | ESP : 339 |
| 84 | 5 | ESP : 353 |
| 85 | 5 | ESP : 367 ; NMR2 : 1.00-1.07 (2H, m), 1.17-123 (2H, m), 1.60-1.70 (1H, m), 1.87 (6H, s), 2.47-2.53 (2H, m), 3.76-3.82 (2H, m), 6.94 (2H, brd), 7.08 (1H, d), 721-732 (4H, m), 7.59 (1H, dd) |
| 86 | 5 | ESP : 319 |
| 87 | 5 | ESP : 345 |
| 88 | 5 | ESP : 317 |
| 89 | 5 | ESP : 305 |
| 90 | 5 | ESP : 401 ; NMR2 : 1.00-1.08 (2H, m), 1.18-124 (2H, m), 1.72-1.83 (1H, m), 1.88 (6H, s), 2.70-2.77 (2H, m), 3.77-3.84 (2H, m), 6.82-6.85 (1H, m), 7.06 (1H, dd), 7.17-735 (4H, m), 7.60 (1H, dd) |
| 91 | 5 | ESP : 333 |
| 92 | 5 | ESP : 401 ; NMR2 : 1.00-1.07 (2H, m), 1.14-125 (2H, m), 1.60-1.65 (1H, m), 1.87 (6H, s), 2.43-2.50 (2H, m), 3.74-3.80 (2H, m), 6.88 (2H, d), 7.09 (1H, d), 722-728 (3H, m), 7.59 (1H, dd) |
| 93 | 5 | ESP : 347 |
| 94 | 5 | ESP : 368 |
| 95 | 5 | ESP : 333 ; NMR2 : 1.00-1.07 (2H, m), 1.18-123 (2H, m), 1.61-1.71 (1H, m), 1.89 (6H, s), 2.40-2.47 (2H, m), 3.72-3.79 (2H, m), 6.84-6.91 (2H, m), 7.18-729 (5H, m), 7.65 (1H, ddd), 8.65 (1H, brd) |
| 96 | 5 | ESP : 385 ; NMR2 : 1.02-1.09 (2H, m), 1.17-1.23 (2H, m), 1.67-1.80 (1H, m), 1.87 (6H, s), 2.54-2.60 (2H, m), 3.76-3.83 (2H, m), 6.86 (1H, t), 6.97-7.10 (3H, m), 7.19-727 (2H, m), 7.60 (1H, t) |
| 97 | 5 | ESP : 385 ; NMR2 : 1.00-1.07 (2H, m), 1.17-1.23 (2H, m), 157-1.69 (1H, m), 1.87 (6H, s), 2.44-2.50 (2H, m), 3.74-3.80 (2H, m), 6.88-7.10 (4H, m), 7.19 (1H, d), 724 (1H, d), 7.60 (1H, t) |
| 98 | 5 | ESP : 351 ; NMR2 : 1.01-1.08 (2H, m), 1.18-1.23 (2H, m), 1.65-1.78 (1H, m), 1.88 (6H, s), 2.47-2.54 (2H, m), 3.72-3.78 (2H, m), 6.76 (1H, ddd), 6.95-7.05 (2H, m), 7.16-727 (3H, m), 7.66 (1H, ddd), 8.64 (1H, ddd) |

**[Table 63]**

| | | |
|---|---|---|
| 99 | 5 | ESP : 351 |
| 100 | 5 | ESP : 367 |
| 101 | 5 | ESP : 351 ; NMR2 : 1.00-1.08 (2H, m), 1.18-124 (2H, m), 1.59-1.68 (1H, m), 1.88 (6H, s), 2.36-2.44 (2H, m), 3.71-3.80 (2H, m), 6.55 (1H, ddd), 6.65 (1H, ddd), 6.91 (1H, ddd), 7.17-7.27 (3H, m), 7.66 (1H, ddd), 8.64 (1H, ddd) |
| 102 | 5 | ESP : 385 ; NMR2 : 1.01-1.09 (2H, m), 1.17-124 (2H, m), 1.59-1.69 (1H, m), 1.87 (6H, s), 2.44-2.53 (2H, m), 3.76-3.85 (2H, m), 6.64 (1H, ddd), 6.72 (1H, brd), 6.93 (1H, ddd), 7.10 (1H, dd), 722-7.30 (2H, m), 7.60 (1H, dd) |
| 103 | 15 | FP : 336 ; NMR1 : 0.63-0.72 (2H, m), 0.81-1.03 (6H, m), 1.98-2.09 (1H, m), 2.38-2.47 (2H, m), 2.86-2.95 (1H, m), 3.34-3.41 (2H, m), 3.95-4.07 (1H, m), 5.34 (1H, d), 6.66 (1H, d), 6.96 (1H, d) |
| 104 | 15 | FP : 382 ; NMR1 : 0.63-0.70 (2H, m), 0.81-0.89 (2H, m), 0.90-1.03 (4H, m), 1.99-2.08 (1H, m), 238-2.47 (2H, m), 2.85-2.93 (1H, m), 3.34-3.41 (2H, m), 3.95-4.07 (1H, m), 533 (1H, d), 6.63 (1H, d), 7.06 (1H, d) |
| 105 | 15 | FP : 302 ; NMR1 : 0.57-0.65 (2H, m), 0.76-0.83 (2H, m), 0.89-1.03 (4H, m), 1.98-2.08 (1H, m), 2.39-2.48 (2H, m), 2.78-2.87 (1H, m), 3.34-3.46 (2H, m), 3.95-4.08 (1H, m), 5.31 (1H, d), 6.72 (1H, dd), 6.95 (1H, dd), 738 (1H, dd) |
| 106 | 15 | FP : 316 ; NMR1 : 0.69-0.84 (6H, m), 1.06-1.13 (2H, m), 1.40 (3H, s), 2.40-2.48 (2H, m), 2.76-2.85 (1H, m), 3.34-3.45 (2H, m), 3.97-4.09 (1H, m), 5.30 (1H, d), 6.73 (1H, dd), 6.95 (1H, dd), 738 (1H, dd) |
| 107 | 15 | FP : 316 ; NMR1 : 0.44-0.51 (2H, m), 0.68-0.76 (2H, m), 1.82-1.94 (1H, m), 1.96-2.10 (1H, m), 2.25-2.48 (6H, m), 2.64-2.72 (1H, m), 3.34-3.45 (2H, m), 3.62-3.74 (1H, m), 3.96-4.07 (1H, m), 5.30 (1H, d), 6.70 (1H, dd), 6.93 (1H, dd), 737 (1H, dd) |
| 15 | 15 | ESP : 350 ; NMR1 : 0.52-0.56 (2H, m), 0.75-0.81 (2H, m), 1.83-1.92 (1H, m), 1.97-2.09 (1H, m), 2.27-2.45 (6H, m), 2.73-2.79 (1H, m), 3.34-339 (2H, m), 3.65-3.74 (1H, m), 3.97-4.06 (1H, m), 5.36 (1H, d), 6.64 (1H, d), 6.96 (1H, d) |
| 108 | 15 | ESP : 396 ; NMR1 : 0.51-0.55 (2H, m), 0.75-0.80 (2H, m), 1.84-1.92 (1H, m), 1.97-2.09 (1H, m), 227-2.45 (6H, m), 2.73-2.79 (1H, m), 3.34-3.39 (2H, m), 3.65-3.74 (1H, m), 3.97-4.06 (1H, m), 5.35 (1H, d), 6.60 (1H, d), 7.05 (1H, d) |
| 109 | 15 | ESP : 341 ; NMR1 : 0.47-0.51 (2H, m), 0.73-0.78 (2H, m), 1.83-1.93 (1H, m), 1.97-2.09 (1H, m), 2.27-2.42 (4H, m), 2.46-2.50 (2H, m), 2.70-2.76 (1H, m), 3.39-3.44 (2H, m), 3.64-3.73 (1H, m), 4.03-4.13 (1H, m), 5.43 (1H, d), 7.00 (1H, d), 7.84 (1H, d) |
| 110 | 15 | FP : 302 |
| 111 | 15 | ESP : 336 |
| 112 | 15 | ESP : 380 |
| 113 | 15 | FP : 350 |

**[Table 64]**

| | | |
|---|---|---|
| 114 | 15 | FP : 396 |
| 115 | 15 | FP : 350 |
| 116 | 15 | FP : 394 |
| 18 | 18 | FP : 352 |
| 117 | 16 | FP : 304 ; NMR1 : 0.59-0.68 (2H, m), 0.78-0.86 (2H, m), 0.90-1.04 (4H, m), 1.98-2.09 (1H, m), 2.73-2.88 (3H, m), 3.52-3.65 (2H, m), 4.95-5.18 (1H, m), 6.91 (1H, dd), 6.99 (1H, dd), 7.44 (1H, dd) |
| 16 | 16 | FP : 338 ; NMR1 : 0.66-0.74 (2H, m), 0.85-1.05 (6H, m), 2.00-2.09 (1H, m), 2.74-2.93 (3H, m), 3.49-3.61 (2H, m), 4.95-5.18 (1H, m), 6.88 (1H, d), 7.01 (1H, d) |
| 118 | 16 | FP : 382 ; NMR1 : 0.65-0.73 (2H, m), 0.84-1.04 (6H, m), 1.99-2.09 (1H, m), 2.74-2.91 (3H, m), 3.49-3.62 (2H, m), 4.95-5.18 (1H, m), 6.84 (1H, d), 7.11 (1H, d) |
| 119 | 16 | FP : 352 ; NMR1 : 0.76-0.84 (4H, m), 0.85-0.93 (2H, m), 1.09-1.14 (2H, m), 1.41 (3H, s), 2.75-2.94 (3H, m), 3.49-3.62 (2H, m), 4.98-5.20 (1H, m), 6.89 (1H, d), 7.01 (1H, d) |
| 120 | 16 | FP : 398 ; NMR1 : 0.74-0.83 (4H, m), 0.84-0.92 (2H, m), 1.08-1.12 (2H, m), 1.40 (3H, s), 2.74-2.92 (3H, m), 3.50-3.63 (2H, m), 4.97-520 (1H, m), 6.84 (1H, d), 7.11 (1H, d) |
| 121 | 16 | FP : 352 ; NMR1 : 0.52-0.60 (2H, m), 0.77-0.85 (2H, m), 1.82-1.94 (1H, m), 1.96-2.10 (1H, m), 2.26-2.43 (4H, m), 2.71-2.89 (3H, m), 3.49-3.62 (2H, m), 3.64-3.76 (1H, m), 4.96-5.19 (1H, m), 6.85 (1H, d), 7.00 (1H, d) |
| 122 | 16 | ESP : 398 ; NMR1 : 0.51-0.59 (2H, m), 0.76-0.84 (2H, m), 1.82-1.94 (1H, m), 1.96-2.10 (1H, m), 226-2.43 (4H, m), 2.70-2.89 (3H, m), 3.50-3.62 (2H, m), 3.64-3.75 (1H, m), 4.96-5.19 (1H, m), 6.81 (1H, d), 7.10 (1H, d) |
| 123 | 16 | ESP : 354 ; NMR1 : 0.72-0.79 (2H, m), 0.88-0.97 (2H, m), 1.45 (9H, s), 2.75-2.93 (2H, m), 3.02-3.13 (1H, m), 3.53-3.68 (2H, m), 4.93-5.22 (1H, m), 6.81 (1H, d), 7.01 (1H, d) |
| 17 | 17 | FP : 318 ; NMR1 : 0.58-0.67 (1H, m), 0.83-1.07 (7H, m), 2.01-2.11 (1H, m), 2.99 (1H, d), 3.01-3.08 (1H, m), 3.60 (1H, d), 6.49 (1H, d), 6.57 (1H, d), 6.72 (1H, d), 6.95 (1H, d) |
| 124 | 17 | FP : 362 ; NMR1 : 0.56-0.66 (1H, m), 0.82-1.05 (7H, m), 2.01-2.10 (1H, m), 2.99 (1H, d), 2.99-3.07 (1H, m), 3.60 (1H, d), 6.48 (1H, d), 6.54 (1H, d), 6.72 (1H, d), 7.05 (1H, d) |
| 125 | 17 | ESP : 332 ; NMR1 : 0.72-0.83 (1H, m), 0.86-0.92 (2H, m), 0.99-1.10 (3H, m), 1.12-120 (1H, m), 1.30-1.39 (1H, m), 1.46 (3H, s), 3.06 (1H, d), 3.13-324 (1H, m), 3.60 (1H, d), 6.53 (1H, d), 6.67 (1H, d), 6.73 (1H, d), 6.99 (1H, d) |
| 126 | 17 | ESP : 378 ; NMR1 : 0.61-0.71 (1H, m), 0.74-0.79 (2H, m), 0.91-1.06 (4H, m), 1.19-127 (1H, m), 1.42 (3H, s), 2.98 (1H, d), 3.02-3.12 (1H, m), 3.61 (1H, d), 6.49 (1H, d), 6.53 (1H, d), 6.73 (1H, d), 7.05 (1H, d) |

**[Table 65]**

| | | |
|---|---|---|
| 127 | 17 | ESP : 332 ; NMR1 : 0.45-0.58 (1H, m), 0.69-0.79 (1H, m), 0.85-0.99 (2H, m), 1.81-1.96 (1H, m), 1.97-2.12 (1H, m), 2.27-2.44 (4H, m), 2.85-2.95 (1H, m), 3.00 (1H, d), 3.60 (1H, d), 3.64-3.78 (1H, m), 6.49 (1H, d), 6.54 (1H, d), 6.71 (1H, d), 6.94 (1H, d) |
| 128 | 17 | ESP : 378 ; NMR1 : 0.43-0.55 (1H, m), 0.68-0.79 (1H, m), 0.82-0.99 (2H, m), 1.81-1.96 (1H, m), 1.97-2.12 (1H, m), 2.26-2.44 (4H, m), 2.85-2.95 (1H, m), 3.00 (1H, d), 3.61 (1H, d), 3.64-3.77 (1H, m), 6.49 (1H, d), 6.51 (1H, d), 6.71 (1H, d), 7.04 (1H, d) |
| 129 | 17 | ESP : 334 ; NMR1 : 0.72-0.81 (1H, m), 0.84-0.93 (1H, m), 0.99-1.07 (2H, m), 1.45 (9H, s), 3.05 (1H, d), 3.14-323 (1H, m), 3.56 (1H, d), 6.46 (1H, d), 6.50 (1H, d), 6.69 (1H, d), 6.94 (1H, d) |
| 130 | 15 | FP : 302 |
| 131 | 1 | FP : 272 |
| 132 | 2 | ESP : 306 |
| 133 | 5 | ESP : 295 ; NMR1 : 0.47-0.57 (2H, m), 0.76-0.88 (2H, m), 121-132 (4H, m), 1.72-1.86 (4H, m), 223-2.36 (1H, m), 2.92-3.04 (1H, m), 7.32-7.45 (2H, m), 7.85 (1H, ddd), 8.53 (1H, ddd) |
| 134 | 7 | FP : 269 ; NMR1 : 0.64-0.68 (2H, m), 0.89-0.94 (2H, m), 121-1.33 (4H, m), 1.86-1.96 (1H, m), 2.01-2.12 (1H, m), 2.22-2.29 (1H, m), 2.76-2.84 (3H, m), 3.05-3.12 (2H, m), 6.12 (2H, t), 6.86(2H, t) |
| 135 | 5 | ESP : 417 |
| 136 | 2 | ESP : 326 ; NMR2 : 0.87-0.96 (2H, m), 0.97-1.07 (4H, m), 1.14-124 (2H, m), 1.85-1.99 (1H, m), 1.91 (6H, s), 2.77-2.88 (1H, m), 6.61 (1H, s) |
| 137 | 1 | FP : 288 ; NMR1 : 0.28-0.32 (2H, m), 0.75-0.80 (2H, m), 1.85-2.05 (8H, m), 229-2.37 (2H, m), 2.50-2.51 (2H, m), 2.81-2.86 (1H, m) 3.66-3.70 (1H, m) 6.78 (1H, d), 6.94(1H, dd), 7.37(1H, d) |
| 138 | 21 | FP : 395 |
| 28 | 28 | ESP : 287 |
| 139 | 1 | FP : 352 ; NMR1 : 0.72-0.84 (4H, m), 0.86-1.00 (4H, m), 1.83 (6H, s), 1.97-2.05 (1H, m), 2.52-2.59 (1H, m), 7.02 (1H, d), 7.57 (1H, d) |
| 140 | 15 | FP : 341 |
| 141 | 16 | FP : 343 ; NMR1 : 0.47-0.55 (2H, m), 0.74-0.82 (2H, m), 1.83-1.94 (1H, m), 1.96-2.10 (1H, m), 226-2.44 (4H, m), 2.66-2.75 (1H, m), 2.83-2.98 (2H, m), 3.55-3.74 (3H, m), 5.01-5.23 (1H, m), 721 (1H, d), 7.88 (1H, d) |
| 142 | 17 | FP : 323 ; NMR1 : 0.37-0.46 (1H, m), 0.66-0.75 (1H, m), 0.82-0.97 (2H, m), 1.82-1.95 (1H, m), 1.97-2.11 (1H, m), 2.27-2.45 (4H, m), 2.85-2.93 (1H, m), 3.06 (1H, d), 3.68 (1H, d), 3.68-3.76 (1H, m), 6.55 (1H, d), 6.76 (1H, d), 6.88 (1H, d), 7.82 (1H, d) |
| 143 | 4 | ESP : 333 ; NMR2 : 0.85-0.96 (4H, m), 0.99-1.08 (2H, m), 1.14-123 (2H, m), 1.88-2.03 (1H, m), 1.96 (6H, s), 2.49-2.60 (1H, m), 7.40 (1H, s) |

**[Table 66]**

| | | |
|---|---|---|
| 144 | 4 | FP : 313 ; NMR1 : 0.62-0.65 (2H, m), 0.76-0.78 (2H, m), 0.90-0.92 (2H, m), 1.12-1.14 (2H, m), 1.41 (3H, s), 1.90 (6H, s), 3.05-3.09 (1H, m), 7.02 (1H, d), 7.84 (1H, d) |
| 145 | 4 | FP : 313 ; NMR1 : 0.34-0.38 (2H, m), 0.82-0.87 (2H, m), 1.82-1.89 (8H, m), 1.99-2.06 (2H, m), 2.30-2.39 (2H, m), 2.88-2.94 (1H, m), 3.66-3.72 (1H, m), 7.00 (1H, d), 7.84 (1H, d) |
| 146 | 5 | ESP : 367 ; NMR2 : 1.01-1.08 (2H, m), 1.18-1.24 (2H, m), 1.60-1.66 (1H, m), 1.86 (6H, s), 2.51-2.60 (2H, m), 3.71-3.78 (2H, m), 6.89-6.94 (2H, m), 7.12 (1H, dd), 7.20-7.31 (3H, m), 7.60 (1H, dd), 8.54 (1H, dd) |
| 147 | 5 | ESP : 303 ; NMR2 : 0.37-0.43 (2H, m), 0.72-0.80 (2H, m), 0.97-1.40 (2H, m), 1.15-1.21 (2H, m), 1.88 (6H, s), 1.89-1.95 (1H, m), 2.72 (1H, dt), 7.08 (1H, dd), 7.55 (1H, dd), 8.53 (1H, dd) |
| 148 | 2 | ESP : 333 ; NMR1 : 0.62-0.68 (2H, m), 0.91-1.06 (6H, m), 1.87 (6H, s), 2.00-2.09 (1H, m), 3.04-3.12 (1H, m), 7.32 (1H, s) |
| 30 | 30 | FP : 282 |
| 149 | 3 | ESP : 377 ; NMR1 : 0.59-0.69 (2H, m), 0.90-1.06 (6H, m), 1.86 (6H, s), 1.99-2.11 (1H, m), 3.01-3.12 (1H, m), 7.29 (1H, s) |
| 150 | 19 | FP : 299 ; NMR1 : 0.62-0.69 (2H, m), 0.82-1.02 (6H, m), 1.92 (6H, s), 1.98-2.09 (1H, m), 2.80-2.88 (1H, m), 7.37 (1H, d), 7.67 (1H, d) |
| 29 | 29 | FP : 271 ; NMR1 : 0.40-0.45 (2H, m), 0.75-0.80 (2H, m), 0.90-1.01 (4H, m), 1.56 (3H, s), 1.94 (6H, s), 2.01-2.08 (1H, m), 2.67-2.73 (1H, m), 5.77-5.79 (1H, m), 5.92 (1H, t), 6.93-6.94 (1H, m) |
| 151 | 2 | ESP : 333 ; NMR1 : 0.74-0.80 (2H, m), 0.88-1.04 (6H, m), 1.95 (6H, s), 2.00-2.10 (1H, m), 2.81-2.90 (1H, m), 8.38 (1H, s) |
| 152 | 29 | FP : 271 ; NMR1 : 0.25-0.29 (2H, m), 0.82-0.87 (2H, m), 0.91-1.01 (4H, m), 1.92 (6H, s), 1.96 (3H, s), 2.00-2.07 (1H, m), 2.93-2.97 (1H, m), 5.84-5.85 (1H, m), 6.34-6.36 (1H, m), 6.51 (1H, t) |
| 153 | 5 | ESP : 369 ; NMR2 : 1.01-1.09 (2H, m), 1.18-123 (2H, m), 1.66-1.74 (1H, m), 1.88 (6H, s), 2.48-2.54 (2H, m), 3.73-3.79 (2H, m), 6.48-6.54 (1H, m), 6.92-7.04 (2H, m), 7.18-724 (2H, m), 7.65 (1H, dt), 8.62 (1H, ddd) |
| 154 | 5 | ESP : 369 ; NMR2 : 1.01-1.08 (2H, m), 1.18-1.24 (2H, m), 1.75 (1H, tt), 1.89 (6H, s), 2.70-2.78 (2H, m), 3.66-3.72 (2H, m), 6.83 (2H, t), 7.10-721 (3H, m), 7.61 (1H, dt), 8.58 (1H, ddd) |
| 155 | 5 | ESP : 385 ; NMR2 : 1.10 (2H, dt), 1.28-1.34 (2H, m), 1.75 (1H, tt), 1.87 (6H, s), 2.61-2.66 (2H, m), 3.75-3.80 (2H, m), 6.83 (1H, dt), 7.01 (1H, t), 7.07 (1H, dt), 7.15 (1H, d), 7.21-7.25 (1H, m), 7.63 (1H, dd), 8.53 (1H, d) |
| 156 | 5 | ESP : 345 ; NMR2 : 1.01 (2H, dt), 1.21 (2H, dt), 1.56 (1H, tt), 2.08 (2H quint), 2.24-2.29 (2H, m), 3.00 (4H, t), 3.84-3.89 (2H, m), 6.98 (2H, d), 7.17-730 (5H, m), 7.64 (1H, dt), 8.65 (1H, ddd) |
| 40 | 40 | FP : 342 ; NMR2 : 0.63-0.69 (2H, m), 0.85-0.93 (2H, m), 0.99-1.06 (2H, m), 1.16-1.22 (2H, m), 1.89-1.98 (7H, m), 2.75-2.81 (1H, m), 6.95 (1H, d), 7.56-7.58 (1H, m) |

**[Table 67]**

| | | |
|---|---|---|
| 157 | 5 | FP : 416 ; NMR2 : 1.03-1.009 (2H, m), 1.19-1.24 (2H, m), 1.67-1.73 (1H, m), 1.84 (6H, s), 2.63-2.69 (2H, m), 3.88-3.94 (2H, m), 6.64 (1H, d), 6.95 (1H, d), 6.96-7.00 (2H, m), 722-7.34 (3H, m) |
| 158 | 5 | ESP : 281 |
| 159 | 5 | ESP : 337 |
| 160 | 5 | ESP : 401 ; NMR2 : 1.11 (2H, dt), 1.32-1.38 (2H, m), 1.64 (1H, tt), 1.93 (6H, s), 2.57-2.62 (2H, m), 3.79-3.84 (2H, m), 6.89 (1H, d), 7.20-7.30 (3H, m), 7.40 (1H, d), 7.60 (1H, d), 7.83 (1H, t) |
| 161 | 7 | FP : 271 ; NMR1 : 0.04-0.08 (2H, m), 0.72-0.77 (2H, m), 1.82-2.08 (2H, m), 1.96 (6H, s), 227-2.41 (4H, m), 2.79-2.85 (1H, m), 3.65-3.74 (1H, m), 6.02 (2H, t), 6.61 (2H, t) |
| 162 | 7 | FP : 271 ; NMR1 : 0.35-0.39 (2H, m), 0.76-0.78 (2H, m), 0.80-0.85 (2H, m), 1.10-1.13 (2H, m), 1.40 (3H, s), 1.97 (6H, s), 2.90-2.96 (1H, m), 6.02 (2H, t), 6.62 (2H, t) |
| 163 | 7 | FP : 287 ; NMR1 : 1.43-1.91 (12H, m), 1.87 (6H, s), 2.82-2.91 (1H, m), 2.84 (3H, s), 6.09 (2H, t), 6.72 (2H, t) |
| 164 | 11 | FP : 279 ; NMR1 : 0.72 (6H, d), 1.42-1.89 (13H, m), 1.46 (6H, s), 2.74-2.80 (1H, m), 2.87-2.94 (1H, m), 3.79 (3H, s) |
| 165 | 5 | FP : 368 ; NMR2 : 0.17-0.22 (2H, m), 0.51-0.59 (2H, m), 0.77-0.87 (1H, m), 0.96-1.03 (2H,m), 1.14-1.20 (2H, m), 1.71-1.791 (1H, m),1.86 (6H, s), 3.61 (2H, d), 6.55 (1H, d), 6.88 (1H, d) |
| 166 | 5 | FP : 430 ; NMR2 : 0.91-0.97 (2H, m), 1.10-1.16 (2H, m), 1.49-1.57 (1H, m), 1.71-1.81 (8H, m), 2.51 (2H, t), 3.62-3.70 (2H, m), 6.41 (1H, d), 6.85 (1H, d), 7.06-720 (2H, m), 721-734 (3H, m) |
| 167 | 5 | FP : 454 ; NMR2 : 1.04-1.11 (2H, m), 1.18-1.24 (2H, m), 1.74-1.83 (1H, m), 1.90 (6H, s), 2.92-2.99 (2H, m), 3.82-3.90 (2H, m), 6.59 (1H, d), 6.85-6.93 (3H, m), 7.19-7.28 (1H, m) |
| 168 | 5 | ESP : 381 ; NMR2 : 1.09 (2H, dt), 1.32-1.38 (2H, m), 1.68 (1H, tt), 2.12 (2H, quint), 2.60-2.64 (2H, m), 2.97-3.01 (4H, m), 3.90-3.94 (2H, m), 6.87 (2H, t), 7.18-725 (3H, m), 7.66 (1H, dt), 8.62 (1H, d) |
| 169 | 1 | ESP: 342 ; NMR1 : 0.48-0.52 (2H, m), 0.89-1.01 (6H, m), 1.89 (6H,s), 2.02-2.06 (1H, m), 3.04-3.08 (1H, m), 6.94 (1H, m), 7.58 (1H, m) |
| 170 | 5 | FP : 404 |
| 171 | 5 | FP : 356 ; NMR2 : 1.41-1.48 (8H, m), 1.87 (6H,s), 1.96-2.08 (3H, m), 4.53-4.61 (1H, m), 6.62 (1H, d), 6.97 (1H, d) |
| 172 | 1 | FP : 274 ; NMR1 : 0.38-0.42 (2H, m), 0.77-0.82 (2H, m), 0.88-0.98 (4H, m), 1.76 (6H, s), 1.96-2.03 (1H, m), 2.84-2.90 (1H, m), 6.70 (1H, dd), 7.21(1H, dd), 7.46 (1H, dd) |
| 173 | 1 | FP : 288 ; NMR1 : 0.23-0.27 (2H, m), 0.69-0.74 (2H, m), 1.76 (6H, s), 1.82-1.92 (1H, m), 1.95-2.06 (1H, m), 225-2.40 (4H, m) 2.71-2.77 (1H, m), 3.62-3.70, (1H, m), 6.69 (1H, dd), 7.19 (1H, dd), 7.45 (1H, dd) |

**[Table 68]**

| | | |
|---|---|---|
| 174 | 3 | FP : 366 ; NMR1 : 0.39-0.43 (2H, m), 0.82-0.87 (2H, m), 1.83-1.91 (7H, m), 1.96-2.07 (1H, m), 2.30-2.40 (4H, m), 2.87-2.92 (1H, m), 3.65-3.73 (1H, m), 6.65 (1H, d), 7.05 (1H, d) |
| 175 | 8 | ESP : 349 ; NMR1 : 00.13-0.17 (2H, m), 0.78-0.83 (2H, m), 1.83-2.06 (2H, m), 1.96 (6H, s), 227-2.39 (4H, m), 2.85-2.91 (1H, m), 3.66-3.75 (1H, m), 6.10-6.12 (1H, m), 6.59 (1H, t), 6.78-6.80 (1H, m) |
| 176 | 8 | FP : 365 ; NMR1 : 1.44-1.89 (12H, m), 1.87 (6H, s), 2.86-2.96 (1H, m), 2.94 (3H, s), 6.17-6.18 (1H, m), 6.70 (1H, t), 6.93-6.94 (1H, m) |
| 177 | 5 | FP: 356 ; NMR2 : 0.97 (3H, t), 1.37-1.54 (4H, m), 1.84-1.99 (9H, m), 3.86-3.93 (2H, m), 6.70 (1H, d), 6.98 (1H, d) |
| 178 | 4 | FP : 398 |
| 179 | 1 | FP : 288 ; NMR2 : 0.22-0.29 (2H, m), 0.47-0.60 (4H, m), 0.79-0.87 (2H, m), 1.15-1.23 (1H, m), 1.96 (6H, s), 2.70-2.79 (3H, m), 6.73 (1H, dd), 6.87-6.92 (1H, m), 7.13-7.18 (1H, d) |
| 180 | 3 | FP : 368 ; NMR2 : 0.23-0.29 (2H, m), 0.54-0.65 (4H, m), 0.87-0.95 (2H, m), 1,14-124 (1H, m), 1.93 (6H, m), 2.74-2.84 (3H, m), 6.50 (1H, d), 6.86 (1H, d) |
| 181 | 8 | FP : 351 ; NMR1 : 0.39-0.47 (2H, m), 0.73-0.93 (4H, m), 1.10-1.17 (2H, m), 1.41 (3H, s), 1.96 (6H, s), 2.98-3.06 (1H, m), 6.12 (1H, dd), 6.60 (1H, dd), 6.83 (1H, dd) |
| 182 | 5 | ESP : 381 ; NMR2 : 1.00 (2H,dt), 1.13-1.18 (2H, m), 1.63 (1H, tt), 2.08 (2H, quint), 2.24-2.32 (2H, m), 2.99 (4H, t), 3.77-3.85 (2H, m), 6.72-6.90 (3H, m), 7.17-723 (2H, m), 7.64 (1H, dt), 8.64 (1H, dt) |
| 22 | 22 | ESP : 300 ; NMR1 : 0.54-0.58 (2H, m), 0.87-1.00 (6H, m), 1.83 (6H, s), 1.99-2.05 (1H, m), 2.99-3.03 (1H, m), 5.08 (1H, d), 539 (1H, d), 6.72 (1H, d), 6.79 (1H, dd), 6.93 (1H, d) |
| 183 | 3 | ESP : 354 ; NMR1 : 0.50-0.54 (2H, m), 0.84-0.99 (6H, m), 1.73 (6H, s), 1.98-2.05 (1H, m), 2.91-2.96 (1H, m), 6.84 (1H, d), 7.20 (1H, d) |
| 184 | 3 | ESP : 354 ; NMR1 : 0.71-0.73 (4H, m), 0.86-0.99 (4H, m), 1.77 (6H, s), 1.96-2.04 (1H, m), 2.49-2.55 (1H, m), 7.12 (1H, d), 7.60 (1H, d) |
| 185 | 1 | ESP : 316 ; NMR2 : 0.50-0.56 (2H, m), 0.83-0.91 (2H, m), 1.27-1.43 (3H, m), 1.71-2.01 (7H, m), 1.96 (6H, s), 2.68-2.75 (1H, m), 2.81-2.92 (1H, m), 6.73 (1H, dd), 6.90 (1H, dd), 7.16 (1H, dd) |
| 41 | 41 | EI : 233 |
| 186 | 3 | FP : 392 |
| 187 | 1 | ESP : 300 ; NMR1 : 029-037 (2H, m), 0.74-0.83 (2H, m), 1.86 (6H, s), 2.83-2.93 (1H, m), 3.02-3.11 (4H, m), 3.63-3.76 (1H, m), 4.81-4.86 (2H, m), 6.78 (1H, dd), 6.94 (1H, dd), 7.38 (1H, dd) |
| 188 | 3 | ESP : 394 |
| 189 | 1 | ESP : 290 ; NMR1 : 0.44-0.51 (2H, m), 0.86-0.94 (2H, m), 1.45 (9H, s), 1.85 (6H, s), 3.09-3.19 (1H, m), 6.75 (1H, dd), 6.95 (1H, dd), 737 (1H, dd) |
| 190 | 3 | ESP : 368 ; NMR1 : 0.55-0.62 (2H, m), 0.93-1.01 (2H, m), 1.45 (9H, s), 1.83 (6H, s), 3.14-322 (1H, m), 6.63 (1H, d), 7.07 (1H, d) |

**[Table 69]**

| | | |
|---|---|---|
| 191 | 1 | FP : 276 ; NMR2 : 0.71-0.78 (2H, m), 1.00-1.12 (5H, m), 1.87-2.02 (8H, m), 2.94-3.02 (1H, m), 3.10-3.18 (2H, m), 6.80 (1H, dd), 6.97 (1H, dd), 723-7.28 (1H, m) |
| 192 | 3 | ESP : 380 ; NMR1 : 0.40-0.48 (2H, m), 0.82-0.90 (2H, m), 1.84 (6H, s), 2.90-2.98 (1H, m), 3.03-3.13 (4H, m), 3.64-3.77 (1H, m), 4.81-4.86 (2H, m), 6.66 (1H, d), 7.06 (1H, d) |
| 193 | 1 | ESP : 302 ; NMR2 : 0.49-0.60 (2H, m), 0.82-0.92 (2H, m),1.57-1.73 (2H, m), 1.81-2.16 (6H, m), 1.96 (6H, s), 2.69-2.81 (1H, m), 3.20-3.33 (1H, m), 6.74 (1H,dd), 6.91 (1H, dd), 7.17 (1H, dd) |
| 23 | 23 | ESP : 302 ; NMR1 : 0.48-0.52 (2H, m), 0.87-1.00 (6H, m), 1.89 (6H, s), 2.01-2.05 (1H, m), 3.02-3.05 (1H, m), 7.03 (1H, d), 7.89 (1H, d), 9.85 (1H, s) |
| 194 | 7 | FP : 321 ; NMR1 : 0.91-1.02 (4H, m), 1.87-1.97 (1H, m), 1.90 (6H, s), 2.34-2.39 (2H, m), 3.65-3.70 (2H, m), 6.14 (2H, t), 6.88 (2H, t), 7.08-7.10 (2H, m), 7.19-7.29 (3H, m) |
| 24 | 24 | ESP : 382 ; NMR1 : 0.46-0.54 (2H, m), 0.85-0.93 (2H, m), 1.85 (6H, s), 2.96-3.05 (1H, m), 3.32 (1H, s), 3.50 (2H, d), 3.51 (1H, s), 3.79-3.91 (1H, m), 6.67 (1H, s), 7.07 (1H, s) |
| 195 | 3 | ESP : 366 ; NMR2 : 0.78-0.98 (6H, m), 1.24-1.30 (2H, m), 1.48 (3H, s), 1.92 (6H, s), 2.78-2.85 (1H, m), 6.51 (1H, d), 6.87 (1H, d) |
| 196 | 3 | ESP : 380 ; NMR2 : 0.55-0.72 (2H, m), 0.87-1.00 (2H, m), 1.57-2.22 (8H, m), 1.94 (6H, s), 2.71-2.86 (1H, m), 3.16-3.34 (1H, m), 6.51 (1H,d), 6.87 (1H, d) |
| 197 | 3 | FP : 356 ; NMR2 : 0.57-0.64 (2H, m), 0.87-0.94 (2H, m), 1.03 (3H, t), 1.80-1.95 (8H, m), 2.72-2.81 (3H, m), 6.49 (1H, d), 6.85 (1H, d) |
| 198 | 5 | ESP : 363 ; NMR2 : 1.03 (2H, dt), 1.24 (2H, dt), 1.56 (1H, tt), 2.08 (2H, quint), 2.22-2.27 (2H, m), 3.00 (4H, t), 3.84-3.89 (2H, m), 6.92-7.00 (4H, m), 721 (1H, ddd), 724 (1H, d) 7.65 (1H, dt), 8.64 (1H, ddd) |
| 199 | 5 | ESP : 399 ; NMR2 : 1.07 (2H, dt), 1.27-1.32 (2H, m), 1.67 (1H, tt), 2.04-2.16 (2H, m), 2.54-2.58 (2H, m), 2.95-3.06 (4H, m), 3.84-3.88 (2H, m), 6.64 (2H, t), 7.18 (1H, ddd), 7.22 (1H, d), 7.64 (1H, dt), 8.61 (1H, d) |
| 25 | 25 | ESP : 404 ; NMR1 : 0.50-0.57 (2H, m), 0.88-0.96 (2H, m), 1.86 (6H, s), 3.01-3.18 (5H, m), 3.58-3.78 (1H, m), 6.71 (1H, d), 7.10 (1H, d) |
| 200 | 11 | FP : 325 |
| 201 | 11 | FP : 313 |
| 202 | 29 | ESP : 288 ; NMR1 : 0.53-0.57 (2H, m), 0.86-1.00 (6H, m), 1.80 (6H, s), 2.00-2.04 (1H, s), 2.96-3.00 (1H, m), 6.55-0.60 (2H, m) |
| 26 | 26 | FP : 302 ; NMR1 : 0.51-0.55 (2H, m), 0.85-0.99 (6H, m), 1.18 (3H, t), 1.81 (6H, s), 1.99-2.05 (1H, m), 2.72 (2H, q), 2.95-3.00 (1H, m), 6.58 (1H, d), 6.64 (1H, d) |
| 203 | 1 | ESP : 338 ; NMR1 : 120-1.41 (4H, m), 1.77 (6H, s), 2.22-2.35 (1H, m), 2.46 (2H, dd), 4.02 (1H, dd), 6.98-7.14 (3H, m), 7.21-7.34 (3H, m), 7.60-7.72 (2H, m) |

**[Table 70]**

| | | |
|---|---|---|
| 204 | 1 | ESP : 430 ; NMR1 : 0.25-0.31 (2H, m), 0.57-0.64 (2H, m), 1.10-1.13 (1H, m), 1.84 (6H, s), 2.47 (2H, dd), 2.79 (2H, d), 7.02 (1H, d), 7.04-7.08 (2H, m), 7.22 (1H, d), 7.24-7.34 (3H, m) |
| 205 | 1 | FP : 288 ; NMR1 : 0.35-0.40 (2H, m), 0.50-0.56 (2H, m), 0.60-0.66 (2H, m), 0.82-0.88 (2H, m), 1.19-1.29 (1H, m), 1.84 (6H, s), 2.93 (2H, d), 3.09-3.16 (1H, m), 6.88 (1H, dd), 7.34 (1H, dd), 7.56 (1H, dd) |
| 206 | 1 | FP : 380 |
| 207 | 1 | FP : 302 |
| 208 | 1 | FP : 276 ; NMR1 : 0.66 (3H, t), 1.14-1.32 (6H, m), 1.76 (6H, s), 2.21-2.30 (1H, m), 3.75-3.81 (2H, m), 6.99 (1H, dd), 7.58 (1H, dd), 7.61 (1H, dd) |
| 209 | 5 | ESP : 315 ; NMR2 : 0.58-0.64 (2H, m), 0.75-0.82 (2H, m), 0.97-1.04 (2H, m), 1.14-1.20 (2H, m), 1.90 (1H, tt), 1.98-2.23 (2H, m), 2.44 (1H, tt), 2.83-2.93 (2H, m), 2.96-3.07 (2H, m), 7.02 (1H, dd), 7.56 (1H, dd), 8.57 (1H, dd) |
| 210 | 1 | ESP : 288 ; NMR1 : 0.09-0.15 (2H, m), 0.35-0.42 (2H,m), 0.59-0.70 (2H, m), 1.20-1.39 (4H, m), 1.77 (6H, s), 2.25-2.35 (1H, m), 3.82 (2H, d), 6.99 (1H, dd), 7.57 (1H, ABX), 7.61 (1H, ABX) |
| 211 | 1 | ESP : 370 ; NMR1 : 0.34-035 (2H, m), 0.59-0.64 (2H, m), 0.73 (3H, m), 1.17-1.23 (3H, m), 1.83 (6H, s), 2.86-2.89 (2H, m), 3.75-3.79 (2H, m), 6.99-7.00 (1H, m), 7.18-7.19 (1H, m) |
| 31 | 31 | ESP : 324 ; NMR1 : 0.46-0.51 (2H, m), 0.87-1.01 (6H, m), 1.87 (6H, s), 2.00-2.06 (1H, m), 3.00-3.06 (1H, m), 6.83-6.84 (1H, m), 7.23 (1H, t), 730-732 (1H, m) |
| 32 | 32 | ESP:304 |
| 212 | 1 | FP : 392 ; NMR2 : 1.93 (6H, s), 2.46-2.53 (2H, m), 2.56 (3H, s), 4.13 (2H, t), 6.78 (1H, d), 6.88-6.94 (2H, m), 7.03 (1H, d), 7.24-7.35 (3H, m) |
| 213 | 8 | FP : 349 ; NMR1 : 0.52-0.56 (2H,m), 0.85-1.03 (6H, m), 1.77-1.88 (1H, m), 1.97-2.07 (2H, m), 2.72-2.79 (3H, m), 3.04-3.11 (2H, m), 6.14-6.16 (1H, m), 6.73 (1H, t), 6.92-6.93 (1H, m) |
| 214 | 8 | FP : 349 |
| 215 | 1 | FP : 286 ; NMR1 : 0.60-0.64 (2H, m), 0.79-0.84 (2H, m), 0.89-0.98 (4H, m), 1.85-2.01 (2H, m), 2.06-2.17 (1H, m), 2.49-2.55 (2H, m), 2.59-2.65 (1H, m), 2.91-2.99 (2H, m), 6.79 (1H, dd), 738 (1H, dd), 7.49 (1H, dd) |
| 216 | 1 | FP : 312 ; NMR2 : 1.89 (6H, s), 2.30 (2H, t), 2.52 (3H, s), 4.01 (2H, t), 6.82-6.88 (2H, m), 6.93-6.98 (1H, m), 7.25-7.32 (4H, m), 7.45 (1H, dd) |
| 217 | 1 | FP : 288 ; NMR1 : 0.68-0.73 (2H, m), 0.87-0.93 (2H, m), 0.96-1.00 (2H, m), 1.37-1.42 (2H, m), 1.48 (3H, s), 1.82 (6H, s), 3.08-3.15 (1H, m), 6.91 (1H, dd), 7.36 (1H, dd), 7.55 (1H, dd |
| 218 | 1 | ESP : 430 |

**[Table 71]**

| | | |
|---|---|---|
| 43 | 43 | ESP : 358 ; NMR1 : 0.51-0.58 (2H, m), 0.74-0.82 (2H, m), 1.35 (3H, t), 1.79 (6H, s), 2.68-2.77 (1H, m), 4.35 (2H, q), 6.71 (1H, d), 7.06 (1H, d) |
| 219 | 1 | FP : 352 |
| 220 | 1 | FP : 352 ; NMR1 : 1.78 (6H, s), 1.90-1.99 (1H, m), 2.04-2.16 (1H, m), 2.27-2.41 (4H, m), 2.49-2.59 (2H, m), 3.84-3.93 (3H, m), 7.01-7.03 (2H, m), 7.07-7.08 (1H, m), 722-7.32 (3H, m), 7.65-7.67 (2H, m) |
| 44 | 44 | FP : 323 |
| 221 | 5 | FP : 372 ; NMR1 : 0.75 (3H, t), 1.20-1.33 (2H, m), 1.47 (9H, s), 1.85 (6H, s), 3.83-3.90 (2H, m), 6.98 (1H, d), 7.18 (1H, d) |
| 222 | 5 | ESP : 347 |
| 223 | 5 | ESP : 335 |
| 224 | 5 | ESP : 307 |
| 225 | 5 | ESP : 335 ; NMR2 : 1.03 (3H, t), 1.89 (2H, sext), 1.90 (6H, s), 2.26-2.31 (2H, m), 2.61 (2H, t), 3.65-3.71 (2H, m), 6.85 (2H, d), 7.20-7.29 (5H, m), 7.67 (1H, dt), 8.63 (1H, ddd) |
| 226 | 1 | FP : 412 |
| 227 | 5 | ESP : 347 |
| 45 | 45 | ESP : 384 |
| 228 | 1 | FP : 356 ; NMR1 : 0.68-0.74 (2H, m), 0.89-1.00 (4H, m), 1.26-1.30 (2H, m), 1.46 (3H, s), 1.93 (6H, s), 3.19-326 (1H, m), 7.00 (1H, dd), 7.61 (1H, dd) |
| 229 | 7 | ESP : 315 |
| 33 | 33 | FP : 317 |
| 34 | 34 | FP : 331 |
| 230 | 34 | FP : 371 |
| 231 | 1 | FP : 406 |
| 35 | 35 | FP : 363 |
| 232 | 5 | ESP : 371 ; NMR2 : 1.05 (3H, t), 1.91 (6H, s), 1.92 (2H, sext), 2.57-2.62 (2H, m), 2.74 (2H, t), 3.60-3.65 (2H, m), 6.85 (2H, t), 7.16-723 (3H, m), 7.64 (1H, dt), 8.57 (1H, dd) |
| 233 | 1 | ESP : 248 |
| 234 | 1 | ESP : 262 |
| 235 | 1 | ESP : 250 |
| 236 | 1 | ESP : 306 |
| 237 | 3 | ESP : 386 ; NMR1 : 0.38-0.44 (2H, m), 0.86-0.92 (2H, m), 1.33 (3H, t), 1.90 (6H, s), 3.18-3.25 (1H, m), 4.38 (2H, q), 6.72 (1H, d), 7.09 (1H, d) |
| 238 | 1 | FP:356 |

**[Table 72]**

| | | |
|---|---|---|
| 239 | 1 | FP : 396 ; NMR1 : 0.53-0.57 (2H, m), 0.91-0.96 (2H, m), 1.86 (6H, s), 3.00-3.05 (1H, m), 4.02 (2H, dd), 6.68 (1H, d), 7.08 (1H, d) |
| 240 | 1 | FP : 408 ; NMR1 : 0.63-0.67 (2H, m), 0.95-1.00 (2H, m), 1.88 (6H, s), 2.85-2.98 (2H, m), 3,17-3.21 (3H, m), 6.74 (1H, d), 7.12 (1H, d) |
| 241 | 1 | ESP : 394 |
| 242 | 5 | ESP : 321 |
| 243 | 5 | ESP : 349 ; NMR2 : 0.95 (3H, t), 1.43 (2H, sext), 1.82 (2H, quint), 1.90 (6H, s), 2.23-2.29 (2H, m), 2.59 (2H, t), 3.61-3.67 (2H, m), 6.85 (2H, dd), 7.16-7.28 (5H, m), 7.64 (1H, dt), 8.61-8.65 (1H, m) |
| 244 | 1 | ESP : 326 |
| 245 | 1 | ESP : 458 |
| 46 | 46 | ESP 318 |
| 246 | 1 | FP : 410 |
| 247 | 5 | ESP : 359 |
| 248 | 2 | ESP : 322 ; NMR1 : 0.30-0.39 (2H, m), 0.56-0.70 (4H, m), 0.93-1.04 (2H, m), 1.12-124 (1H, m), 1.89 (6H, s), 2.89 (2H, d), 3.13-326 (1H, m), 6.78 (1H, d), 7.03 (1H, d) |
| 249 | 1 | ESP : 420 |
| 250 | 1 | ESP : 404 |
| 251 | 5 | ESP : 353 |
| 252 | 5 | ESP : 369 ; NMR2 : 1.06-1.11 (2H, m), 1.25-1.29 (2H, m), 1.73 (1H, tt), 1.88 (6H, s), 2.43-2.48 (2H, m), 3.74-3.79 (2H, m), 6.42 (1H, ddd), 6.87-6.99 (2H, m), 722-727 (2H, m), 7.69 (1H, dt), 8.64 (1H, ddd) |
| 253 | 5 | ESP : 347 |
| 254 | 46 | ESP : 344 ; NMR1 : 0.50-0.55 (2H, m), 0.75-0.81 (2H, m), 1.80 (6H, s), 2.70-2.77 (1H, m), 3.97 (3H, s), 6.71 (1H, d), 7.06 (1H, d) |
| 255 | 5 | ESP : 363 |
| 256 | 5 | ESP : 403 ; NMR2 : 1.17 (2H, dt), 138-1.44 (2H, m), 1.82 (1H, tt), 1.88 (6H, s), 2.80-2.85 (2H, m), 3.74-3.79 (2H, m), 6.87 (2H, t), 7.15 (1H, d), 722 (1H, tt), 7.63 (1H, dd), 8.51 (1H, d) |
| 257 | 5 | ESP : 347 ; NMR2 : 0.70-0.76 (1H, m), 0.84-0.91 (1H, m), 0.95 (3H, d), 0.96-1.03 (1H, m), 1.04-1.15 (1H, m), 1.80 (3H, s), 1.95 (3H, s), 239 (1H, sext), 3.64 (1H, dd), 3.76 (1H, dd), 6.87 (2H, dd), 7.18-7.28 (5H, m), 7.67 (1H, dt), 8.63 (1H, ddd) |
| 258 | 5 | ESP : 347 |
| 259 | 11 | FP : 317 |
| 260 | 7 | FP : 313 ; NMR1 : 0.07-0.11 (2H, m), 0.79-0.84 (2H, m), 1.45-1.66 (6H, m), 1.70-1.80 (4H, m), 1.91-1.99 (2H, m), 1.96 (6H, s), 2.90-2.96 (1H, m), 3.08-3.15 (1H, m), 6.02 (2H, t), 6.60 (2H, t) |
| 261 | 1 | ESP : 324 |

**[Table 73]**

| | | |
|---|---|---|
| 262 | 16 | FP : 304 ; NMR1 : 0.74-0.80 (2H, m), 0.85-0.92 (2H, m), 1.22-1.36 (4H, m), 223-2.32 (1H, m), 2.81-2.98 (3H, m), 3.44-3.56 (2H, m), 7.01 (1H, dd), 7.44 (1H, dd), 7.60 (1H, dd) |
| 36 | 36 | FP : 415 |
| 263 | 36 | FP : 337 |
| 264 | 1 | ESP: 372 |
| 265 | 1 | ESP : 398 |
| 266 | 7 | FP : 333 ; NMR1 : 0.57-0.62 (1H, m), 0.84-1.06 (4H, m), 1.22-1.27 (1H, m), 1.86 (3H, s), 1.89-1.95 (1H, m), 2.01 (3H, s), 2.07-2.13 (1H, m), 329-334 (1H, m), 6.07 (2H, t), 6.59 (2H, t), 7.04 (2H, d), 7.17-729 (3H, m) |
| 267 | 1 | FP : 459 ; NMR1 : 1.41 (9H, s), 1.50-1.63 (2H, m), 1.81 (6H, s), 1.82-1.86 (2H, m), 2.80-2.99 (3H, m), 3.22 (3H, s), 3.93-4.02 (2H, m), 7.00 (1H, d), 7.60 (1H, d) |
| 268 | 1 | FP : 316 ; NMR1 : 1.17-124 (4H, m), 1.85 (6H, s), 2.13-222 (1H, m), 3.43 (3H, s), 7.14 (1H, d), 7.67 (1H, d) |
| 269 | 1 | FP : 386 ; NMR1 : 0.53-0.59 (2H, m), 0.94-1.01 (2H, m), 1.74-1.86 (2H, m), 1.91-1.98 (2H, m), 1.94 (6H, s), 3.16-325 (1H, m), 3.28-338 (1H, m), 3.46 (2H, dt), 3.95 (2H, dd), 6.99 (1H, d), 7.61 (1H, d) |
| 270 | 35 | FP : 393 |
| 271 | 35 | FP : 351 |
| 272 | 1 | ESP : 340 ; NMR2 : 1.20 (3H, t), 1.39-1.46 (2H, m), 1.85-1.95 (3H, m), 1.89 (6H, s), 4.02 (2H, q), 6.67 (1H, d), 6.98 (1H, d) |
| 273 | 1 | ESP:324 |
| 47 | 47 | FP : 359 ; NMR1 : 1.84 (6H, s), 1.91-2.10 (4H, m), 2.93-3.05 (2H, m), 320-3.40 (3H, m), 3.32 (3H, s), 7.08 (1H, d), 7.64 (1H, d) |
| 274 | 1 | ESP : 342 ; NMR1 : 1.44 (9H, s), 1.80 (6H, s), 3.52 (3H, s), 6.88 (1H, d), 7.17 (1H, d) |
| 275 | 1 | FP : 372 ; NMR1 : 1.05 (6H, s), 1.26 (6H, s), 1.61 (1H, s), 1.87 (6H, s), 327 (3H, s), 7.12 (1H, d), 7.67 (1H, d) |
| 276 | 10 | ESIP : 401 ; NMR1 : 130 (6H, d), 1.86 (6H, s), 2.04-2.19 (2H, m), 2.21-2.35 (2H, m), 3.02-3.14 (2H, m), 3.26-3.39 (1H, m), 3.36 (3H, s), 3.40-3.50 (3H, m), 7.11 (1H, d), 7.65 (1H, d) |
| 277 | 1 | FP : 296 ; NMR2 : 120 (3H, t), 1.38-1.46 (2H, m), 1.83-1.96 (9H, m), 4.04 (2H, q), 6.69 (1H, d), 6.83 (1H, d) |
| 42 | 42 | ESP : 324 |
| 278 | 1 | FP : 282 ; NMR2 : 1.29-1.37 (2H, m), 1.65-1.75 (2H, m), 1.90 (6H, s), 2.03-2.13 (1H, m), 3.59 (3H, s), 6.72 (1H, d), 6.83 (1H, d) |
| 279 | 1 | FP : 332 ; NMR1 : 1.43 (9H, s), 1.86 (6H, s), 3.47 (3H, s), 7.10-7.12 (1H, m), 7.65-7.68 (1H, m) |
| 280 | 1 | FP : 360 ; NMR1 : 0.78-0.84 (2H, m), 0.98-1.05 (2H, m), 1.14-1.21 (4H, m), 1.91 (6H, s), 2.18-2.28 (1H, m), 3.15-3.23 (1H, m), 7.80 (1H, d) |

**[Table 74]**

| | | |
|---|---|---|
| 37 | 37 | FP : 367 ; NMR1 : 0.71-0.77 (2H, m), 0.97-1.07 (2H, m), 1.20-1.26 (4H, m), 1.94 (6H, s), 2.22-2.30 (1H, m), 3.17-327 (1H, m), 7.63 (1H, d) |
| 281 | 2 | ESP : 298 ; NMR1 : 1.44 (9H, s), 1.80 (6H, s), 3.52 (3H, s), 6.91 (1H, d), 7.07 (1H, d) |
| 38 | 38 | FP : 324 ; NMR1 : 0.75-0.91 (4H, m), 0.96-1.13 (4H, m), 1.84 (6H, s), 2.95-3.04 (1H, m), 622 (1H, brs), 6.72 (1H, d), 6.97 (1H, d) |
| 39 | 39 | FP : 326 ; NMR2 : 0.85-0.98 (4H, m), 1.34-1:56 (4H, m), 1.94 (6H, s), 2.85-2.93 (1H, m), 6.54 (1H, d), 6.74 (1H, d) |
| 282 | 1 | FP : 314 ; NMR1: 1.10-1.18 (4H, m), 1.79 (6H, s), 2.10-2.19 (1H, m), 4.24-4.54 (4H, m), 6.91 (1H, d), 7.05 (1H, d) |
| 283 | 1 | EP : 296 ; NMR2: 1.04-1.09 (2H, m), 1.43-1.54 (5H, m), 1.90 (6H, s), 3.52 (3H, m), 6.68 (1H, d), 6.82 (1H, d) |
| 284 | 1 | FP : 292 ; NMR1 : 0.76-0.79 (2H, m), 1.01-1.06 (2H, m), 1.16-130 (4H, m), 1.85 (6H, s), 224-2.31 (1H, m), 3.22-326 (1H, m), 6.60-6.62 (2H., m) |
| 27 | 27 | ESP : 285 |

**[Table 75]**

| | | | | | |
|---|---|---|---|---|---|
| 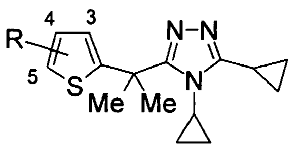 | | | | | |

| No | R | No | R | No | R |
|---|---|---|---|---|---|
| 1 | 4-Me | 12 | 4-F | 23 | 3-F-4-Cl |
| 2 | 4-Me-5-Br | 13 | 4-CN-5-F | 24 | 4-Br-5-Cl |
| 3 | 4-F-5-CN | 14 | 4-Me-5-F | 25 | 4-Me-5-Cl |
| 4 | 4-CF₃-5-Br | 15 | 4-Me-5-CN | 26 | 4,5-diF |
| 5 | 3-Br-5-Cl | 16 | 4-CF₃-5-F | 27 | 4-CF₃-5-Cl |
| 6 | 3,5-diCN | 17 | 3-Cl-5-F | 28 | 4-CF₃-5-CN |
| 7 | 3-F-5-Br | 18 | 3,5-diBr | 29 | 3-CN-5-F |
| 8 | 3-CF₃ | 19 | 3-CN-5-Br | 30 | 3-F-5-CN |
| 9 | 3-CF₃-5-F | 20 | 3,5-diF | 31 | 3-CF₃-5-Br |
| 10 | 3-Cl-5-CF₃ | 21 | 3-CF₃-5-Cl | 32 | 3,5-diCF₃ |
| 11 | 4,5-diBr | 22 | 3-CF₃-5-CN | | |

**[Table 76]**

| | | | | | |
|---|---|---|---|---|---|
| 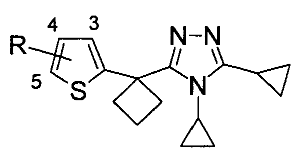 | | | | | |

| No | R | No | R | No | R |
|---|---|---|---|---|---|
| 33 | 5-F | 52 | 5-CF₃ | 70 | 5-CN |
| 34 | 4-Cl | 53 | 4-Cl-5-F | 71 | 4-Cl-5-CN |
| 35 | 4,5-diCl | 54 | 4-Cl-5-Br | 72 | 4-Br |
| 36 | 4-Br-5-Cl | 55 | 4,5-diBr | 73 | 4-CN |
| 37 | 4-CN-5-F | 56 | 4-CN-5-Cl | 74 | 4-CN-5-Br |
| 38 | 4-Me | 57 | 4-Me-5-F | 75 | 4-Me-5-Cl |
| 39 | 4-Me-5-Br | 58 | 4-Me-5-CN | 76 | 4-F |
| 40 | 4,5-diF | 59 | 4-F-5-Cl | 77 | 4-F-5-Br |
| 41 | 4-F-5-CN | 60 | 4-CF₃ | 78 | 4-CF₃-5-Cl |
| 42 | 4-CF₃-5-Br | 61 | 4-CF₃-5-F | 79 | 4-CF₃-5-CN |
| 43 | 3-Cl | 62 | 3-Cl-5-F | 80 | 3-Cl-5-CN |
| 44 | 3,5-diCl | 63 | 3-Cl-5-Br | 81 | 3-Br |
| 45 | 3-Br-5-Cl | 64 | 3,5-diBr | 82 | 3-CN |
| 46 | 3-CN-5-Cl | 65 | 3-CN-5-Br | 83 | 3-CN-5-F |
| 47 | 3,5-diCN | 66 | 3-F | 84 | 3-F-5-Cl |
| 48 | 3-F-5-Br | 67 | 3,5-diF | 85 | 3-F-5-CN |
| 49 | 3-CF₃ | 68 | 3-CF₃-5-Cl | 86 | 3-CF₃-5-Br |
| 50 | 3-CF₃-5-F | 69 | 3-CF₃-5-CN | 87 | 3,5-diCF₃ |
| 51 | 3-Cl-5-CF₃ | | | | |

**[Table 77]**

| | | | | | |
|---|---|---|---|---|---|
| 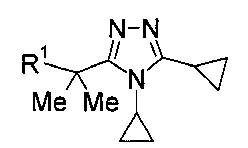 | | | | | |

| No | R¹ | No | R¹ | No | R¹ |
|---|---|---|---|---|---|
| 88 | 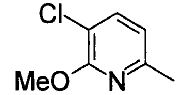 | 97 | 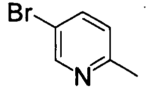 | 106 | 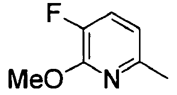 |
| 89 | 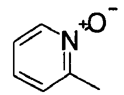 | 98 | 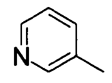 | 107 | 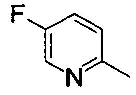 |
| 90 | 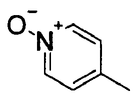 | 99 | 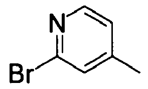 | 108 | 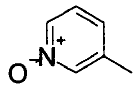 |
| 91 | 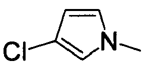 | 100 | 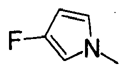 | 109 | 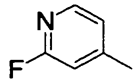 |
| 92 | 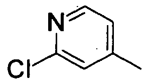 | 101 | 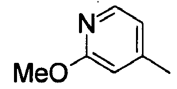 | 110 | 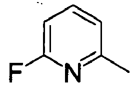 |
| 93 | 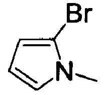 | 102 | 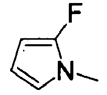 | 111 | 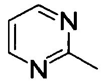 |
| 94 | 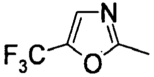 | 103 | 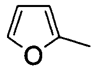 | 112 | 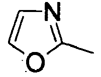 |
| 95 | (iPr)₂CHNH- | 104 | (cPr)₂CHNH- | 113 | (cPr)₂CHN(Me)- |
| 96 | 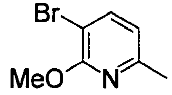 | 105 | (iPr)₂-CHNH- | | |

**[Table 78]**

| | | | | | |
|---|---|---|---|---|---|
| 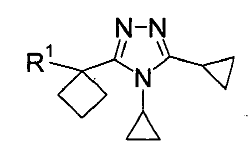 | | | | | |

| No | R¹ | No | R¹ | No | R¹ |
|---|---|---|---|---|---|
| 114 | 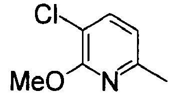 | 125 | 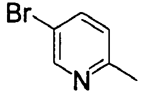 | 136 | 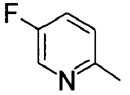 |
| 115 | 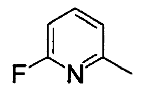 | 126 | 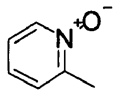 | 137 | 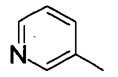 |
| 116 | 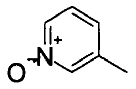 | 127 | 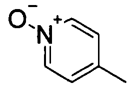 | 138 | 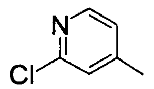 |
| 117 | 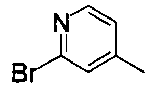 | 128 | 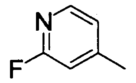 | 139 | 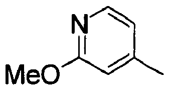 |
| 118 | 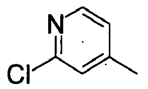 | 129 | 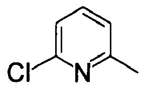 | 140 | 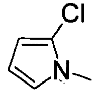 |
| 119 | 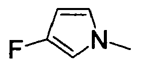 | 130 | 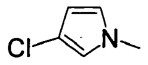 | 141 | 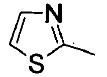 |
| 120 | 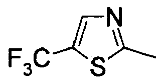 | 131 | 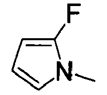 | 142 | 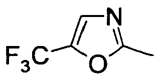 |
| 121 | 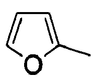 | 132 | 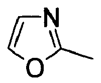 | 143 | 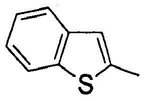 |
| 122 | 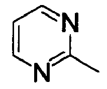 | 133 | 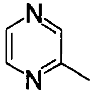 | 144 | (cPr)₂CHNH- |
| 123 | (cPr)₂CHN(Me)- | 134 | (iPr)₂CHNH- | 145 | (iPr)₂CHNH- |
| 124 | 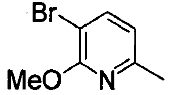 | 135 | 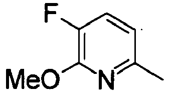 | | |

**[Table 79]**

| | | | | | |
|---|---|---|---|---|---|
| 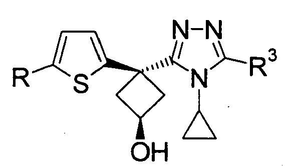 | | | | | |

| No | R | R³ | No R | R | R³ |
|---|---|---|---|---|---|
| 146 | F | 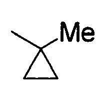 | 150 | F | cBu |
| 147 | CF₃ | | 151 | CF₃ | |
| 148 | Br | | | | |
| 149 | Cl | | | | |

**[Table 80]**

| | | | | | |
|---|---|---|---|---|---|
| 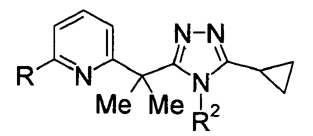 | | | | | |

| No | R² | R | No | R² | R |
|---|---|---|---|---|---|
| 152 | 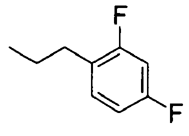 | H | 157 | -(CH₂)₃-Ph | H |
| 153 | | Cl | 158 | | Cl |
| 154 | 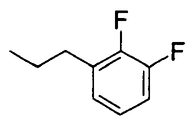 | Cl | 159 | 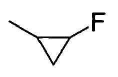 | H |
| | | | 160 | | Cl |
| 155 | 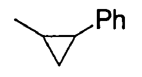 | H | | | |
| 156 | | Cl | | | |

**[Table 81]**

| | | | | | |
|---|---|---|---|---|---|
| 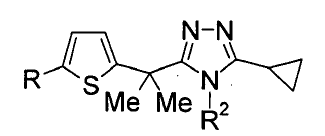 | | | | | |

| No | R² | R | No | R² | R |
|---|---|---|---|---|---|
| 161 | -(CH₂)₂-Ph | H | 171 | 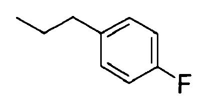 | H |
| | | | 172 | | Cl |
| 162 | | Cl | | | |
| | | | 173 | | Br |
| 163 | 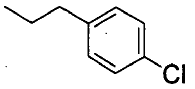 | H | 174 | 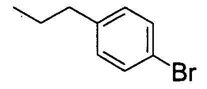 | H |
| 164 | | Cl | 175 | | Cl |
| 165 | | Br | 176 | | Br |
| 166 | 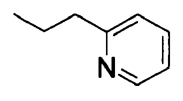 | H | 177 | 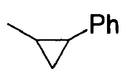 | H |
| 167 | | Cl | 178 | | Cl |
| 168 | | Br | 179 | | Br |
| 169 | -(CH₂)₃-Ph | H | 180 | | H |
| | | | 181 | 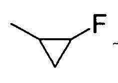 | Cl |
| 170 | | Cl | | | |
| | | | 182 | | Br |

**[Table 82]**

| | | | | | |
|---|---|---|---|---|---|
| 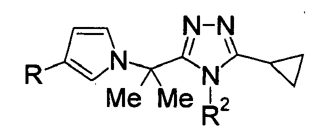 | | | | | |

| No | R² | R | No | R² | R |
|---|---|---|---|---|---|
| 183 | -(CH₂)₂-Ph | Br | 190 | 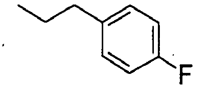 | H |
| | | | 191 | | Br |
| 184 | 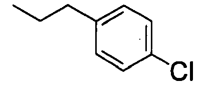 | H | 192 | 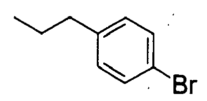 | H Br |
| 185 | | Br | 193 | | |
| 186 | 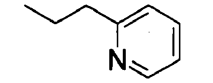 | H | 194 | 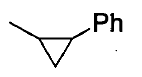 | Br |
| 187 | | Br | | | |
| 188 | -(CH₂)₃-Ph | H | 195 | 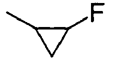 | H Br |
| 189 | | Br | 196 | | |

### INDUSTRIAL APPLICABILITY

Since the compounds of the present invention have excellent 11β-HSD1-inhibitory activity, they are useful as preventive and therapeutic agents for the diseases in which 11β-HSD1 is concerned, such as hyperglycemia, insulin resistance, obesity, hyperlipidemia, hypertension, osteoporosis, glaucoma, lowering of cognition function and the like, particularly hyperglycemia, insulin resistance and the like.

## Claims

1. A triazole derivative represented by the formula (I) or a pharmaceutically acceptable salt thereof: 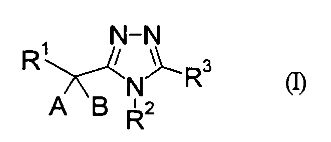 [symbols in the formula represent the following meanings;
R¹: a heterocyclic group or -N(R⁰)-R⁴,
wherein the heterocyclic group of R¹ may be substituted,
R⁰: -H or lower alkyl,
R⁴: C₁₋₇ alkyl, halogeno-lower alkyl, lower alkyl substituted with cycloalkyl, cycloalkyl, aryl, lower alkylene-aryl, lower alkylene-aromatic heterocyclic group, -S(O)₂-lower alkyl, -S(O)₂-aryl, or -S(O)₂-aromatic heterocyclic group,
wherein the cycloalkyl, aryl and aromatic heterocyclic group of R⁴ may be substituted respectively,
A and B: the same or different from each other and each is lower alkyl, or A and B in combination, and together with the carbon atom to which these are bonded, may form a cycloalkyl ring which may be substituted,
R²: lower alkyl, halogeno-lower alkyl, cycloalkyl, aryl, lower alkylene-CO₂R⁰, lower alkylene-cycloalkyl, lower alkylene-aryl or lower alkylene-aromatic heterocyclic group,
wherein the cycloalkyl, aryl and aromatic heterocyclic group of R² may be substituted respectively,
R³: -H, halogen, lower alkyl, halogeno-lower alkyl, -OR⁰, -CO₂R⁰, cycloalkyl, lower alkylene-cycloalkyl or a saturated heterocyclic group,
wherein the cycloalkyl and a saturated heterocyclic group of R³ may be substituted respectively,
with the proviso that
N-[2-(4-chlorophenyl)ethyl]-N-methyl-1-(5-methyl-4-phenyl-4H-1,2,4-triazol-3-yl)cyclohex-2-ene-1-amine, and
(5-chloro-2-{3-[1-(dimethylamino)cyclopropyl]-5-methyl-4H-1,2,4-triazol-4-yl}phenyl)(2-chlorophenyl)methanone are excluded].

2. The compound described in claim 1, wherein R³ is lower alkyl, or cycloalkyl which may be substituted.

3. The compound described in claim 2, wherein A and B are both methyl; or the ring formed by A and B in combination together with the carbon atom to which these are bonded is cyclobutyl ring or cyclobutenyl ring which may respectively be substituted.

4. The compound described in claim 3, wherein R¹ is an aromatic heterocyclic ring which may be substituted.

5. The compound described in claim 4, wherein R² is lower alkyl, cycloalkyl or lower alkylene-(aryl which may be substituted).

6. The compound described in claim 5, wherein R³ is cyclopropyl or cyclobutyl which may be respectively substituted with lower alkyl.

7. The compound described in claim 6, wherein A and B are both methyl.

8. The compound described in claim 7, wherein R¹ is pyridyl or thienyl which may respectively be substituted with a group selected from the group consisting of halogen, lower alkyl and halogeno-lower alkyl.

9. The compound described in claim 8, wherein R² is cyclopropyl or -(CH₂)₂-(phenyl which may be substituted with halogen).

10. The compound described in claim 1 which is selected from the group consisting of
3-[1-(5-bromo-2-thienyl)-1-methylethyl]-4,5-dicyclopropyl-4H-1,2,4-triazole,
2-(1-{5-cyclopropyl-4-[2-(2,6-difluorophenyl)ethyl]-4H-1,2,4-triazol-3-yl}-1-methylethyl)pyridine,
3,4-dicyclopropyl-5-{1-methyl-1-[5-(trifluoromethyl)-2-thienyl]ethyl} -4H-1,2,4-triazole, and
3,4-dicyclopropyl-5-{1-[3-fluoro-5-(trifluoromethyl)-2-thienyl]-1-methylethyl}-4H-1,2,4-triazole,
or a pharmaceutically acceptable salt thereof.

11. A pharmaceutical composition, which comprises the compound described in claim 1 or a pharmaceutically acceptable salt thereof and a pharmaceutically acceptable carrier.

12. The pharmaceutical composition described in claim 11, which is an 11β-hydroxysteroid dehydrogenase type 1 inhibitor.

13. The pharmaceutical composition described in claim 11, which is an insulin resistance-improving agent.

14. The pharmaceutical composition described in claim 11, which is an agent for preventing or treating diabetes.

15. Use of the compound described in claim 1 or a pharmaceutically acceptable salt thereof, for the manufacture of an 11β-hydroxysteroid dehydrogenase type 1 inhibitor, an insulin resistance improving agent or an agent for preventing or treating diabetes.

16. A method for preventing or treating diabetes, which comprises administering an effective amount of the compound described in claim 1 or a salt thereof to a patient.
